# EUROPEAN PATENT SPECIFICATION

(11) **EP 3 157 942 B1**
(45) Date of publication and mention of the grant of the patent: **17.06.2020**
(21) Application number: 15733963.1
(22) Date of filing: 18.06.2015
(51) Int. Cl.: C07K 1/107

(54) **SITE SPECIFIC PROTEIN MODIFICATIONS**
ORTSSPEZIFISCHE PROTEINMODIFIKATIONEN
MODIFICATIONS DE PROTÉINES SPÉCIFIQUES À UN SITE

(30) Priority: 23.06.2014 US 201462015868 P; 20.11.2014 US 201462082337 P
(43) Date of publication of application: 26.04.2017
(73) Proprietor: Novartis AG, 4056 Basel (CH)
(72) Inventor: USERA, Aimee Richardson, Cambridge, Massachusetts 02139 (US); YUAN, Jun, Cambridge, Massachusetts 02139 (US); LOU, Changgang, Cambridge, Massachusetts 02139 (US)
(74) Representative: Campbell, Lachlan Clive
(86) International application number: PCT/US2015/036330
(87) International publication number: WO 2015/200080

(56) References cited:
- WO-A1-2013/148117
- Anonymous: "Preferentially biotinylate N-terminal alpha-amino groups in peptides.", Thermo Scientific , 2009, XP002742982, Retrieved from the Internet: URL:https://tools.lifetechnologies.com/con tent/sfs/brochures/TR0046-Biotinylate-N-te rminus.pdf [retrieved on 2015-08-03]
- Z.Q. JIA ET AL: "Cardiovascular effects of a PEGylated apelin", PEPTIDES, vol. 38, no. 1, 1 November 2012 (2012-11-01), pages 181-188, XP055131938, ISSN: 0196-9781, DOI: 10.1016/j.peptides.2012.09.003
- LEE H ET AL: "N-terminal site-specific mono-PEGylation of epidermal growth factor", PHARMACEUTICAL RESEARCH, vol. 20, no. 5, 1 May 2003 (2003-05-01), pages 818-825, XP009114507, SPRINGER NEW YORK LLC, US ISSN: 0724-8741, DOI: 10.1023/A:1023402123119
- CHAN A O-Y ET AL: "Modification of N-terminal [alpha]-amino groups of peptides and proteins using ketenes", JOURNAL OF THE AMERICAN CHEMICAL SOCIETY, vol. 134, no. 5, 8 February 2012 (2012-02-08), pages 2589-2598, XP002742983, AMERICAN CHEMICAL SOCIETY USA DOI: 10.1021/JA208009R

## Description

### FIELD OF THE INVENTION

The present invention relates generally to a novel method of introducing modifying groups to a protein or polypeptide. In particular, the present invention relates to selective derivation of the amino functionality at the N-terminus of the protein or the polypeptide using the presence of a neighboring histidine amino acid to increase the reactivity of the N-terminal amino functionality.

### BACKGROUND OF THE INVENTION

It is well-known that the properties and characteristics of proteins or peptides may be modified by conjugating groups to the protein or peptide. Generally, such conjugation generally requires some functional group in the protein to react with another functional group in a conjugating group. Amino groups, such as the N-terminal amino group or the ε-amino group in lysine residues have been used in combination with suitable acylating reagents for this purpose. It is often desired or necessary to control the conjugation reaction, such as where the conjugating compounds are attached to the protein and to control how many conjugating groups are attached. This is often referred to as specificity or selectivity.

Site-specific modification of proteins or peptides is a longstanding challenge in the pharmaceutical and biotechnology arts. The classic methods oftentimes lead to non-specific labeling (e.g. NHS labeling) or require engineering (e.g. maleimide Cys labeling or unnatural amino acids). In addition, the repertoire of selective chemical reactions, however, is very limited. One alternative is, by recombinant methods, to introduce special unnatural amino acids having a unique reactivity and then exploit this reactivity in the further derivatization. Another alternative is the use of enzymes which recognize structural and functional features of the protein to be modified.

Despite recombinant methods, the selective derivatization of proteins/peptides remains a very difficult task. Thus, there is a need in the art for a general method of selectively derivatizing amino acid residues such as at the N-terminus of a protein or peptide.

### SUMMARY OF THE INVENTION

The present invention relates to a method as defined in the claims for modifying a target protein or a target peptide at the amino functionality of the N-terminal amino acid comprising the steps of:
a. modifying the target protein or target peptide so that the resulting protein or peptide contains a histidine amino acid at the position adjacent to the N-terminus amino acid; and
b. contacting the protein or peptide containing a histidine amino acid at the position adjacent to the N-terminus amino acid with an acylating compound at a pH lower than 6, to form a modified protein or polypeptide.

Methods are described for modifying the target protein or target peptide so that the resulting protein or peptide contains a histidine amino acid at the position adjacent to the N-terminus amino acid. Such methods will be described in more details herein.

The invention as defined in the claims, pertains to the selective method for modifying a target protein or a target peptide as described supra using an acylating agent of Formula: 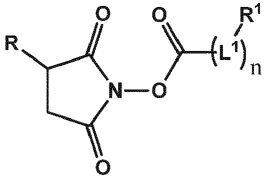 wherein:
L¹ is a linker;
R is H or -S(O)₂Na;
R¹ is a moiety comprising a reactive group that facilitates covalent attachment to a biointeractive agent or to an analytical agent or R¹ is a biointeractive agent or an analytical agent,
n is 0 or 1.

The disclosure encompassing the invention pertains to the selective method for modifying a protein or a peptide at the N-terminus amino group as described herein, wherein the biointeractive or analytical agent is selected from biotin, fluorophore, toxin, chelator, a half-life extending moiety (such as for example albumin binding moiety, Fc variant or native Fc), an imaging reagent and a peptide.

The invention pertains to the selective method for modifying a protein or peptide at the N-terminus amino group, as defined in the claims, wherein the R¹ is a moiety comprising a reactive group that facilitates covalent attachment to a biointeractive or analytical agent. In one particular aspect, the method of the invention further comprises a step of reacting a biointeractive agent or an analytical agent with the reactive group present in the acylating agent.

In one embodiment, the target protein or target peptide is selected from a Growth Differentiation Factor 15 (GDF15) protein, homologs, variants, fragments and other modified form thereof, native Fc, Fc variant or APJ agonist peptide.

These and other aspects of the invention will be elucidated in the following detailed description of the invention.

### BRIEF DESCRIPTION OF THE DRAWING

### DETAILED DESCRIPTION

### Definition:

For purposes of interpreting this specification, the following definitions will apply unless specified otherwise and whenever appropriate, terms used in the singular will also include the plural and vice versa.

The present invention addresses the aforementioned needs by providing a method of introducing modifying compounds to a target protein or peptide in a selective manner.

It must be noted that as used herein and in the appended claims, the singular forms "a", "an" and "the" include plural referents unless the context clearly dictates otherwise. Thus, for example, reference to "the conjugate" includes reference to one or more conjugates; reference to "the polypeptide" includes reference to one or more polypeptides; and so forth.

The term alkyl refers to a fully saturated branched or unbranched (or straight chain or linear) hydrocarbon moiety, comprising 1 to 30 carbon atoms. Preferably the alkyl comprises 5 to 20 carbon atoms, and more preferably 10 to 15 carbon atoms. C₁₀₋₁₅alkyl refers to an alkyl chain comprising 10 to 15 carbons.

The term "alkenyl" refers to a branched or unbranched hydrocarbon having at least one carbon-carbon double bond. The term "C₂₋₃₀-alkynyl" refers to a hydrocarbon having two to seven carbon atoms and comprising at least one carbon-carbon triple

The term "alkynyl" refers to a branched or unbranched hydrocarbon having at least one carbon-carbon triple bond. The term "C₂₋₃₀-alkynyl" refers to a hydrocarbon having two to seven carbon atoms and comprising at least one carbon-carbon triple bond.

The term aryl refers to monocyclic or bicyclic aromatic hydrocarbon groups having 6-10 carbon atoms in the ring portion. Representative examples of aryl are phenyl or naphthyl.

The term heteroaryl includes monocyclic or bicyclic heteroaryl, containing from 5-10 ring members selected from carbon atoms and 1 to 5 heteroatoms, and each heteroatoms is independently selected from O, N or S wherein S and N may be oxidized to various oxidation states. For bicyclic heteroaryl system, the system is fully aromatic (i.e. all rings are aromatic).

The term cycloalkyl refers to saturated or unsaturated but non-aromatic monocyclic, bicyclic or tricyclic hydrocarbon groups of 3-12 carbon atoms, preferably 3-8, or 3-7 carbon atoms. For bicyclic, and tricyclic cycloalkyl system, all rings are non-aromatic. For example, cycloalkyl encompasses cycloalkenyl and cycloalkynyl. The term "cycloalkenyl" refers to a bicyclic or tricyclic hydrocarbon group of 3-12 carbon atoms, having at least one carbon-carbon double bond. The term "cycloalkynyl" refers to a bicyclic or tricyclic hydrocarbon group of 3-12 carbon atoms, having at least one carbon-carbon triple bond.

The term heterocyclyl refers to a saturated or unsaturated non-aromatic (partially unsaturated but non-aromatic) monocyclic, bicyclic or tricyclic ring system which contains at least one heteroatom selected from O, S and N, where the N and S can also optionally be oxidized to various oxidation states. In one embodiment, heterocyclyl moiety represents a saturated monocyclic ring containing from 5-7 ring atoms and optionally containing a further heteroatom, selected from O, S or N. The heterocyclic ring may be substituted with alkyl, halo, oxo, alkoxy, haloalkyl, haloalkoxy. In other embodiment, heterocyclyl is di- or tricyclic. For polycyclic system, some ring may be aromatic and fused to saturated or partially saturated ring or rings. The overall fused system is not fully aromatic. For example, a heterocyclic ring system can be an aromatic heteroaryl ring fused with saturated or partially saturated cycloalkyl ring system.

The term "conjugate" is intended to refer to the entity formed as a result of a covalent attachment of target protein/peptide to a biointeractive/analytical agent, optionally via a linker. The Term "conjugation" is intended to refer the step of covalently attaching the target protein/peptide to a biointeractive/analytical agent, optionally via a linker.

As used herein, the term "polypeptide" refers to a polymer of amino acid residues linked by peptide bonds, whether produced naturally or synthetically. Polypeptides of less than about 10 amino acid residues are commonly referred to as "peptides." The term "peptide" is intended to indicate a sequence of two or more amino acids linked by peptide bonds, wherein said amino acids may be natural or unnatural. The term encompasses the terms polypeptides and proteins, which may consist of two or more peptides held together by covalent interactions, such as for instance cysteine bridges, or non-covalent interactions. The art-recognized three letter or one letter abbreviations are used to represent amino acid residues that constitute the peptides and polypeptides of the invention. Except when preceded with "D", the amino acid is an L-amino acid. When the one letter abbreviation is a capital letter, it refers to the D-amino acid. When the one letter abbreviation is a lower case letter, it refers to the L-amino acid. Groups or strings or amino acid abbreviations are used to represent peptides. Peptides are indicated with the N-terminus on the left and the sequence is written from the N-terminus to the C-terminus.

Peptides of the invention contain non-natural amino acids (i.e., compounds that do not occur in nature) and other amino acid analogs as are known in the art may alternatively be employed.

Certain non-natural amino acids can be introduced by the technology described in Deiters et al., J Am Chem Soc 125:11782-11783, 2003; Wang and Schultz, Science 301:964-967, 2003; Wang et al., Science 292:498-500, 2001; Zhang et al., Science 303:371-373, 2004 or in US Patent No. 7,083,970. Briefly, some of these expression systems involve site-directed mutagenesis to introduce a nonsense codon, such as an amber TAG, into the open reading frame encoding a polypeptide of the invention. Such expression vectors are then introduced into a host that can utilize a tRNA specific for the introduced nonsense codon and charged with the non-natural amino acid of choice. Particular non-natural amino acids that are beneficial for purpose of conjugating moieties to the polypeptides of the invention include those with acetylene and azido side chains.

A "protein" is a macromolecule comprising one or more polypeptide chains. A protein may also comprise non-peptidic components, such as carbohydrate groups. Carbohydrates and other nonpeptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless. A protein or polypeptide encoded by a non-host DNA molecule is a "heterologous" protein or polypeptide.

An "isolated polypeptide or isolated protein" is a polypeptide or protein (for example GDF15) that is essentially free from cellular components, such as carbohydrate, lipid, or other proteinaceous impurities associated with the polypeptide in nature. Typically, a preparation of isolated polypeptide or protein contains the polypeptide or protein in a highly purified form, i.e., at least about 80% pure, at least about 90% pure, at least about 95% pure, greater than 95% pure, such as 96%, 97%, or 98% or more pure, or greater than 99% pure. One way to show that a particular protein preparation contains an isolated polypeptide or protein is by the appearance of a single band following sodium dodecyl sulfate (SDS)-polyacrylamide gel electrophoresis of the protein preparation and Coomassie Brilliant Blue staining of the gel. However, the term "isolated" does not exclude the presence of the same polypeptide or protein in alternative physical forms, such as dimers or alternatively glycosylated or derivatized forms. Preferably, the isolated polypeptide is substantially free from any other contaminating polypeptides or other contaminants that are found in its natural environment that would interfere with its therapeutic, diagnostic, prophylactic or research use.

One of ordinary skill in the art will appreciate that various amino acid substitutions, e.g, conservative amino acid substitutions, may be made in the sequence of any of the polypeptide or protein described herein, without necessarily decreasing its activity. As used herein, "amino acid commonly used as a substitute thereof" includes conservative substitutions (i.e., substitutions with amino acids of comparable chemical characteristics). For the purposes of conservative substitution, the non-polar (hydrophobic) amino acids include alanine, leucine, isoleucine, valine, glycine, proline, phenylalanine, tryptophan and methionine. The polar (hydrophilic), neutral amino acids include serine, threonine, cysteine, tyrosine, asparagine, and glutamine. The positively charged (basic) amino acids include arginine, lysine and histidine. The negatively charged (acidic) amino acids include aspartic acid and glutamic acid. Examples of amino acid substitutions include substituting an L-amino acid for its corresponding D-amino acid, substituting cysteine for homocysteine or other non-natural amino acids having a thiol-containing side chain, substituting a lysine for homolysine, diaminobutyric acid, diaminopropionic acid, ornithine or other non-natural amino acids having an amino containing side chain, or substituting an alanine for norvaline or the like.

The term "amino acid," as used herein, refers to naturally occurring amino acids, unnatural amino acids, amino acid analogues and amino acid mimetics that function in a manner similar to the naturally occurring amino acids, all in their D and L stereoisomers if their structure allows such stereoisomeric forms. Amino acids are referred to herein by either their name, their commonly known three letter symbols or by the one-letter symbols recommended by the IUPAC-IUB Biochemical Nomenclature Commission.

The term "naturally occurring" refers to materials which are found in nature and are not manipulated by man. Similarly, "non-naturally occurring," "un-natural," and the like, as used herein, refers to a material that is not found in nature or that has been structurally modified or synthesized by man. When used in connection with amino acids, the term "naturally occurring" refers to the 20 conventional amino acids (i.e., alanine (A or Ala), cysteine (C or Cys), aspartic acid (D or Asp), glutamic acid (E or Glu), phenylalanine (F or Phe), glycine (G or Gly), histidine (H or His), isoleucine (I or IIe), lysine (K or Lys), leucine (L or Leu), methionine (M or Met), asparagine (N or Asn), proline (P or Pro), glutamine (Q or Gin), arginine (R or Arg), serine (S or Ser), threonine (T or Thr), valine (V or Val), tryptophan (W or Trp), and tyrosine (Y or Tyr)).

The terms "non-natural amino acid" and "unnatural amino acid," as used herein, are interchangeably intended to represent amino acid structures that cannot be generated biosynthetically in any organism using unmodified or modified genes from any organism, whether the same or different. The terms refer to an amino acid residue that is not present in the naturally occurring (wild-type) protein sequence or the sequences of the present invention. These include, but are not limited to, modified amino acids and/or amino acid analogues that are not one of the 20 naturally occurring amino acids, selenocysteine, pyrrolysine (Pyl), or pyrroline-carboxy-lysine (Pcl, e.g., as described in PCT patent publication WO2010/48582). Such non-natural amino acid residues can be introduced by substitution of naturally occurring amino acids, and/or by insertion of non-natural amino acids into the naturally occurring (wild-type) protein sequence or the sequences of the invention. The non-natural amino acid residue also can be incorporated such that a desired functionality is imparted to the molecule, for example, the ability to link a functional moiety (e.g., PEG). When used in connection with amino acids, the symbol "U" shall mean "non-natural amino acid" and "unnatural amino acid," as used herein.

The term "analogue" as used herein referring to a polypeptide or protein means a modified peptide or protein wherein one or more amino acid residues of the peptide/protein have been substituted by other amino acid residues and/or wherein one or more amino acid residues have been deleted from the peptide/protein and/or wherein one or more amino acid residues have been added the peptide/protein. Such addition or deletion of amino acid residues can take place at the N-terminal of the peptide and/or at the C-terminal of the peptide.

The term "APJ" (also referred to as "apelin receptor," "angiotensin-like-1 receptor," "angiotensin II-like-1 receptor," and the like) indicates a 380 residue, 7 transmembrane domain, *Gi* coupled receptor whose gene is localized on the long arm of chromosome 11 in humans (NCBI Reference Sequence: NP_005152.1, and encoded by NCBI Reference Sequence: NM_005161). APJ was first cloned in 1993 from genomic human DNA using degenerate oligonucleotide primers (O'Dowd et al. Gene, 136:355-60, 1993) and shares significant homology with angiotensin II receptor type 1. Despite this homology however, angiotensin II does not bind APJ. Although orphan for many years, the endogenous ligand has been isolated and named apelin (Tatemoto et al., Biochem Biophys Res Commun 251, 471-6 (1998)).

The term "APJ agonist" includes apelin polypeptides: Apelin indicates a 77 residue preprotein (NCBI Reference Sequence: NP_0059109.3, and encoded by NCBI Reference Sequence: NM_017413.3), which gets processed into biologically active forms of apelin peptides, such as apelin-36, apelin-17, apelin-16, apelin-13, apelin-12. The full length mature peptide, referred to as "apelin-36," comprises 36 amino acids, but the most potent isoform is the pyroglutamated form of a 13mer of apelin (apelin-13), referred to as "Pyr-1-apelin-13 or Pyr¹-apelin-13" Different apelin forms are described, for instance, in United States Patent 6,492,324B1. Apelin peptide agonists are also described in patent application numbers WO 2013/111110, WO 2014/081702, WO 2015/013168, WO 2015/013165, WO 2015/013167 and WO 2015/013169.

The terms "GDF15 polypeptide" and "GDF15 protein" are used interchangeably and mean a naturally-occurring wild-type polypeptide expressed in a mammal, such as a human or a mouse. For purposes of this disclosure, the term "GDF15 protein" can be used interchangeably to refer to any full-length GDF15 polypeptide, which consists of 308 amino acid residues; (NCI Ref. Seq. NP_004855.2) containing a 20 amino acid signal peptide, a 167 amino acid prodomain, and a mature domain of 112 amino acids which is excised from the prodomain by furin-like proteases. A 308-amino acid GDF15 polypeptide is referred to as "full-length" GDF15 polypeptide; a 112 amino acids GDF15 polypeptide (e.g. amino acids 197-308) is a "mature" GDF15 polypeptide. The mature GDF15 peptide contains the seven conserved cysteine residues required for the formation of the cysteine knot motif (having three intrachain disulfide bonds) and the single interchain disulfide bond that are typical for TGF∼ superfamily members. The mature GDF15 peptide contains two additional cysteine residues that form a fourth intrachain disulfide bond. Therefore, biologically active GDF15 is a homodimer of the mature peptide covalently linked by one interchain disulfide bond. A GDF15 protein or polypeptide therefore also includes multimer, more particularly dimer of the protein.

The term "GDF15 mutant polypeptide" encompasses a GDF15 polypeptide in which a naturally occurring GDF15 polypeptide sequence has been modified. Such modifications include, but are not limited to, one or more amino acid substitutions, including substitutions with non-naturally occurring amino acids non-naturally-occurring amino acid analogs and amino acid mimetics.

In one aspect, the term "GDF15 mutant protein" refers to a GDF15 protein sequence (e.g., SEQ ID NO 1-8) in which at least one residue normally found at a given position of a native GDF15 polypeptide is deleted or is replaced by a residue not normally found at that position in the native GDF15 sequence. In some cases it will be desirable to replace a single residue normally found at a given position of a native GDF15 polypeptide with more than one residue that is not normally found at the position; in still other cases it may be desirable to maintain the native GDF15 polypeptide sequence and insert one or more residues at a given position in the protein; in still other cases it may be desirable to delete a given residue entirely; all of these constructs are encompassed by the term "GDF 15 mutant protein. The present invention also encompasses nucleic acid molecules encoding such GDF15 mutant polypeptide sequences.

In various embodiments, a GDF15 mutant protein comprises an amino acid sequence that is at least about 85 percent identical to a naturally-occurring GDF15 protein. In other embodiments, a GDF15 polypeptide comprises an amino acid sequence that is at least about 90 percent, or about 95, 96, 97, 98, or 99 percent identical to a naturally-occurring GDF15 polypeptide amino acid sequence. Such GDF15 mutant polypeptides preferably, but need not, possess at least one activity of a wild-type GDF15 mutant polypeptide, such as the ability to lower blood glucose, insulin, triglyceride, or cholesterol levels; the ability to reduce body weight; or the ability to improve glucose tolerance, energy expenditure, or insulin sensitivity.

Although the GDF15 polypeptides and GDF15 mutant polypeptides, and the constructs comprising such polypeptides are primarily disclosed in terms of human GDF15, the invention is not so limited and extends to GDF15 polypeptides and GDF15 mutant polypeptides and the constructs comprising such polypeptides where the GDF15 polypeptides and GDF15 mutant polypeptides are derived from other species (e.g., cynomolgous monkeys, mice and rats). In some instances, a GDF15 polypeptide or a GDF15 mutant polypeptide can be used to treat or ameliorate a metabolic disorder in a subject in a mature form of a GDF15 mutant polypeptide that is derived from the same species as the subject.

A GDF15 mutant polypeptide is preferably biologically active. In various respective embodiments, a GDF15 polypeptide or a GDF15 mutant polypeptide has a biological activity that is equivalent to, greater to or less than that of the naturally occurring form of the mature GDF15 protein. Examples of biological activities include the ability to lower blood glucose, insulin, triglyceride, or cholesterol levels; the ability to reduce body weight; or the ability to improve glucose tolerance, lipid tolerance, or insulin sensitivity; the ability to lower urine glucose and protein excretion.

As used herein in the context of the structure of a polypeptide or protein, the term "N-terminus" (or "amino terminus") and "C-terminus" (or "carboxyl terminus") refer to the extreme amino and carboxyl ends of the polypeptide, respectively. The term "N-terminal amino acid" refers to the last amino acid present at the N-terminus. The term "amino acid adjacent to the N-terminus amino acid" refer to the amino acid present at the position adjacent to the last amino acid of the N-terminus. (i.e. the second amino acid present in the protein or peptide counting from the N-terminus).

The term half-life extending moiety refers to for example a polymer, such as polyethylene glycol (PEG), fatty acids, a cholesterol group, a carbohydrate or oligosaccharide; or any natural or synthetic protein, polypeptide or peptide that binds to a salvage receptor. The half-life extending moiety can bind to plasma protein (e.g. albumin and immunoglobulin) with long serum half-lives. For example, the half-life extending moiety is an IgG constant domain or fragment thereof (e.g., the Fc region), Human Serum Albumin (HSA), or albumin-binding polypeptides. The half-life extending moiety may be an albumin binding residue or other plasma protein binding residue. An "Albumin binding residue" as used herein means a residue which binds non-covalently to human serum albumin. In one disclosure the albumin binding residue is a lipophilic residue. In another disclosure, the albumin binding residue is negatively charged at physiological pH. A binding residue typically comprises a carboxylic acid which can be negatively charged. In an embodiment, the half-life extending moiety is a fatty acid, which can be defined as a C6-70alkyl, a C6-70alkenyl or a C6-70alkynyl chain, each of which is substituted with at least one carboxylic acid (for example 1, 2, 3 or 4 CO2H) and optionally further substituted with hydroxyl group. Examples of fatty acid are defined by Formulae A1, A2 and A3. In other disclosure, the half-life extending moiety is a small molecule which binds non-covalently to a plasma protein (e.g. Albumin). Such small molecules are for examples, Warfarin, aspirin, benzodiazepine derivatives (e.g. diazepam), indoles, cardenolides (e.g. digitoxin) and biliary acids.

Half-life extending moieties include Albumin, which refers to the most abundant protein in the blood plasma having a molecular weight of approximately between 65 and 67 kilodaltons in its monomeric form, depending on species of origin. The term "albumin" is used interchangeably with "serum albumin" and is not meant to define the source of albumin which forms a conjugate with the modified peptides of the invention. Thus, the term "albumin" as used herein may refer either to albumin purified from a natural source such as blood or serous fluis, or it may refer to chemicaly synthetisized or recombinantly produced albumin.

Half-life extending moieties include "native Fc" which refers to molecule or sequence comprising the sequence of a non-antigen-binding fragment resulting from digestion of whole antibody or produced by other means, whether in monomeric or multimeric form, and can contain the hinge region. The original immunoglobulin source of the native Fc is preferably of human origin and can be any of the immunoglobulins, although IgG1 and IgG2 are preferred. Native Fc molecules are made up of monomeric polypeptides that can be linked into dimeric or multimeric forms by covalent (i.e., disulfide bonds) and non-covalent association. The number of intermolecular disulfide bonds between monomeric subunits of native Fc molecules ranges from 1 to 4 depending on class (e.g., IgG, IgA, and IgE) or subclass (e.g., IgG1, IgG2, IgG3, IgA1, and IgGA2). One example of a native Fc is a disulfide-bonded dimer resulting from papain digestion of an IgG (see Ellison et al., 1982, Nucleic Acids Res. 10: 4071-9). The term "native Fc" as used herein is generic to the monomeric, dimeric, and multimeric forms.

Half-life extending moieties include "Fc variant" which refers to a molecule or sequence that is modified from a native Fc but still comprises a binding site for the salvage receptor, FcRn (neonatal Fc receptor). International Publication Nos. WO 97/34631 and WO 96/32478 describe exemplary Fc variants, as well as interaction with the salvage receptor. Thus, the term "Fc variant" can comprise a molecule or sequence that is humanized from a non-human native Fc. Furthermore, a native Fc comprises regions that can be removed because they provide structural features or biological activity that are not required for the bioconjugate of the invention. Thus, the term "Fc variant" comprises a molecule or sequence that lacks one or more native Fc sites or residues, or in which one or more Fc sites or residues has be modified, that affect or are involved in: (1) disulfide bond formation, (2) incompatibility with a selected host cell, (3) N-terminal heterogeneity upon expression in a selected host cell, (4) glycosylation, (5) interaction with complement, (6) binding to an Fc receptor other than a salvage receptor, or (7) antibody-dependent cellular cytotoxicity (ADCC). Fc variants are described in further detail hereinafter.

Half-time extending moieties refer to "Fc domain" which encompasses native Fc and Fc variants and sequences as defined above. As with Fc variants and native Fc molecules, the term "Fc domain" includes molecules in monomeric or multimeric form, whether digested from whole antibody or produced by other means. An Fc domain can be conjugated to a polypeptide of Formula I' or anyone of Formulae I-IX via, for example, a covalent bond between the Fc domain and the peptide sequence. Such Fc proteins can form multimers via the association of the Fc domains and both these Fc proteins and their multimers are an aspect of the present invention.

Half-life extending moieties include "modified Fc fragment", which shall mean an Fc fragment of an antibody comprising a modified sequence. The Fc fragment is a portion of an antibody comprising the CH₂, CH₃ and part of the hinge region. The modified Fc fragment can be derived from, for example, IgGI, IgG2, IgG3, or IgG4. FcLALA is a modified Fc fragment with a LALA mutation (L234A, L235A), which triggers ADCC with lowered efficiency, and binds and activates human complement weakly. Hessell et al. 2007 Nature 449:101-104. Additional modifications to the Fc fragment are described in, for example, U.S. Patent No. 7,217,798.

The linker separates the target protein/peptide and the biointeractive/analytical agent or any moiety comprising a reactive group which facilitates covalent attachment to the biointeractive/analytical agent. In this disclosure, its chemical structure is not critical, since it serves primarily as a spacer.

The linker is a chemical moiety that contains two reactive groups/functional groups, one of which can react with the target peptide/protein and the other with the biointeractive/bionanalyitical agent. The two reactive/functional groups of the linker are linked via a linking moiety or spacer, structure of which is not critical as long as it does not interfere with the coupling of the linker to the target protein/peptide and to the biointeractive/analytical agent.

The linker can be made up of amino acids linked together by peptide bonds. The linker may be made up of from 1 to 20 amino acids linked by peptide bonds, wherein the amino acids are selected from the 20 naturally occurring amino acids. The 1 to 20 amino acids may be selected from the amino acids glycine, serine, alanine, methionine, asparagine, glutamine, cysteine and lysine. A linker may be made up of a majority of amino acids that are sterically unhindered, such as glycine and alanine. Linkers may be polyglycines, polyalanines, combinations of glycine and alanine (such as poly(Gly-Ala)), or combinations of glycine and serine (such as poly(Gly-Ser)).

The linker may comprise 1 to 20 amino acids which are selected from unnatural amino acids. While a linker of 1-10 amino acid residues is preferred for conjugation with the biointeractive or analytical agent, the present disclosure contemplates linkers of any length or composition. An example of non-natural amino acid linker is 8-Amino-3,6-dioxaoctanoic acid having the following formula: 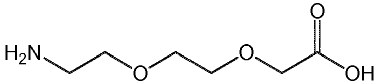 or its repeating units.

The linkers described herein are exemplary, and linkers that are much longer and which include other residues are contemplated. Non-peptide linkers are also contemplated.

Alternatively, the linker may comprise one or more alkyl groups, alkenyl groups, cycloalkyl groups, aryl groups, heteroaryl groups, heterocyclic groups, polyethylene glycol and/or one or more natural or unatural amino acids, or combination thereof, wherein each of the alkyl, alkenyl, cycloalkyl, aryl, heteroaryl, heterocyclyl, polyethylene glycol and/or the natural or unatural amino acids are optionally combined and linked together, or linked to the biointeractive/analytical agent and/or to the target protein/peptide, via a chemical group selected from -C(O)O-, -OC(O)-, -NHC(O)-, -C(O)NH-, -O-, -NH-, -S-, -C(O)-, -OC(O)NH-, -NHC(O)-O-, =NH-O-, =NH-NH- or =NH-N(alkyl)-.

Linkers containing alkyl spacer are for example -NH-(CH₂)_{z}-C(O)- or -S-(CH₂)_{z}-C(O)- or -O-(CH₂)_{z}-C(O)-, -NH-(CH₂)_{z}-NH-, -O-C(O)-(CH2)_{z}-C(O)-O-, -C(O)-(CH₂)_{z}-O-, -NHC(O)-(CH₂)-C(O)-NH- and the like wherein z is 2-20 can be used. These alkyl linkers can further be substituted by any non-sterically hindering group, including, but not limited to, a lower alkyl (e.g., C₁-C₆), lower acyl, halogen (e.g., CI, Br), CN, NH₂, or phenyl.

The linker can also be of polymeric nature. The linker may include polymer chains or units that are biostable or biodegradable. Polymers with repeat linkage may have varying degrees of stability under physiological conditions depending on bond lability. Polymers may contain bonds such as polycarbonates (-O-C(O)-O-), polyesters (-C(O)-O-), polyurethanes (-NH-C(O)-O-), polyamide (-C(O)-NH-). These bonds are provided by way of examples, and are not intended to limit the type of bonds employable in the polymer chains or linkers. Suitable polymers include, for example, polyethylene glycol (PEG), polyvinyl pyrrolidone, polyvinyl alcohol, polyamino acids, divinylether maleic anhydride, N-(2-hydroxypropyl)-methacrylicamide, dextran, dextran derivatives, polypropylene glycol, polyoxyethylated polyol, heparin, heparin fragments, polysaccharides, cellulose and cellulose derivatives, starch and starch derivatives, polyalkylene glycol and derivatives thereof, copolymers of polyalkylene glycols and derivatives thereof, polyvinyl ethyl ether, and the like and mixtures thereof. A polymer linker is for example polyethylene glycol (PEG). The PEG linker can be linear or branched. A molecular weight of the PEG linker is not restricted to any particular size, but can have a molecular weight between 100 to 5000 Dalton for example 500 to 1500 Dalton.

The linker contains appropriate functional-reactive groups at both terminals that form a bridge between the amino group of the target peptide/protein and a functional/reactive group on the biointeractive/bioanalytical agent

The linker may comprise several linking moieties (or spacer) of different nature (for example a combination of amino acids, heterocyclyl moiety, PEG and/or alkyl moieties). In this instance, each linking moiety contains appropriate functional-reactive groups at both terminals in order to link these moieties together and in order to form a bridge at each terminal with the respective partner.

The modified target protein/peptide or construct thereof (i.e. peptide/protein attached to a partial linker) include reactive groups which can react with available reactive functionalities on the analytical/biointeractive agent or construct thereof (i.e. previously attached a partial linker) to form a covalent bond. Reactive groups are chemical groups capable of forming a covalent bond. Reactive groups are located at one site of conjugation and can generally be carboxy, phosphoryl, acyl group, ester or mixed anhydride, maleimide, N-hydroxysuccinimide, tetrazine, alkyne, imidate, pyridine-2-yl-disulfanyl, thereby capable of forming a covalent bond with functionalities like amino group, hydroxyl group, alkene group, carboxy group, hydrazine group, hydroxylamine group, an azide group or a thiol group at the other site of conjugation.

Reactive groups of particular interest for conjugating a target peptide/target protein to a biointeractive/analytical agent and/or a linker moiety and/or to conjugate various linking moieties of different nature together are N-hydroxysuccinimide, alkyne (more particularly cyclooctyne).

Functional group (Fg) include: 1. thiol groups for reacting with reactive groups such as maleimides, tosyl sulfone or pyridine-2-yldisulfanyl; 2. amino groups (for example amino functionality of an amino acid) for reacting with reactive group such as carboxylic acid or activated carboxylic acid (e.g. amide bond formation via N-hydroxysuccinamide chemistry), phosphoryl groups, acyl group or mixed anhydride; 3. Azide to undergo a Huisgen cycloaddition with an alkyne and more particularly a cyclooctyne reactive group (more commonly known as click chemistry); 4. carbonyl group to react with a reactive group selected from hydroxylamine or hydrazine to form oxime or hydrazine respectively; 5. Alkene to react with a tetrazine reactive group in an aza [4+2] addition. While several examples of linkers and functionalities/reactive group are described herein, the disclosure contemplates linkers or any length and composition.

The term "toxin" refers to small molecules or peptides that are capable of interacting with a macromolecules such as enzyme, cellular receptors and cells and cause harm or diseases. Such toxins can be used in cancer therapy to destroy cancer cells. Because of their toxicity, such toxins cannot be used as a drug themselves. They are typically linked to a protein that directs them to the cancer cells. Examples of toxins are MMAF (monomethyl auristatin F), MMAE (monomethyl auristatin E), mertansine (DM-1), maytansine, cryptophycin, tubulysin and dolastatin 10.

The term "chelator" or "chelating agent" refers to an organic molecule capable of forming a complex with metal ions (e.g. lanthanide). Chelators are commonly used to label protein or peptide. The end-products of metal ion conjugates are used for radioimmunodetection, radioimmunotherapy, magnetic resonance imaging, photodynamic therapy or other similar modalities. Non-limiting examples of chelator or chelating agents are DTPA (Diethylenetriaminepentaacetic Anhydride) and derivative thereof, NOTA (1,4,7-triazacyclononane-N,N',N"-triacetic acid) and derivative thereof such As NODA-GA, DOTA (1,4,7,10-tetraazacyclododecane-N,N',N",N"'-tetraacetic acid) (binding radioactive metal ion) and derivative thereof, TETA (1,4,8,11-tetraazacyclotetradecane-N,N',N",N"'-tetraacetic acid) and derivative thereof, DTTA N-(p-isothiocyanatobenzyl)-diethylenetriamine-N,N',N",N'''-tetraacetic acid. These and other chelators are readily available from commercial sources.

The term "imaging reagent" are chemicals designed to allow clinicians to determine whether a mass is benign or malignant and locate metastatic cancer sites in the body. Imaging agents target a particular tumor, after which the imaging instruments can be adjusted to neutralize the target. Examples of imaging agents are fluorophores, vivotags, fluorescent nanoparticles, luciferin and xenolight.

The term "Fluorophore" refer to a chemical compound that can re-emit light upon light excitation. Fluorophores typically contain several combined aromatic groups, or plane or cyclic molecules with several π bonds. Fluorophores are generally covalently bonded to a macromolecule, serving as a marker (or dye, or tag, or reporter) for affine or bioactive reagents (antibodies, peptides, nucleic acids). Fluorophores are notably used to stain tissues, cells, or materials in a variety of analytical methods, i.e., fluorescent imaging and spectroscopy. Examples include fluorescein, rhodamine, Cy dyes such as Cy5 and Cy7, Alexa dies such as Alexa 750, Alexa 647, and Alexa 488, coumarins, and the like.

A "biointeractive agent" as used herein refers to an organic moiety or compound that invokes a biological response when introduced into a living tissue or cell. Example of biointeractive agents include antigens, toxins, therapeutic proteins or peptides and the like. Biointeractive agents may be small molecules and macromolecules.

An "analytical agent" as used herein refers to an organic moiety or compound that can be detected by instrumental methods for qualitatively or quantitatively characterizing the material to which the analytical agent is bound or otherwise associated. Examples of such analytical agents include labels for example fluorophores or radio labels.

### Embodiments

Various embodiments of the invention are described herein. It will be recognized that features specified in each embodiment may be combined with other specified features to provide further embodiments.

In embodiment 1, the invention pertains to a method for modifying a target protein or a target peptide at the amino functionality of the N-terminal amino acid as defined in claim 1.

In an embodiment, the invention pertains to a method according to embodiment 1, wherein the amino acid sequence XH- is MH- and AH-.

In an embodiment, the invention pertains to a method according to embodiment 1, wherein the amino acid sequence XHX'- is selected from AHA- and MHA.

The invention pertains to a method according to embodiment 1 wherein R1 is biotin. Non-limiting examples of such acylating agent for biotinylation are:
SO₃NHS-biotin of Formula: 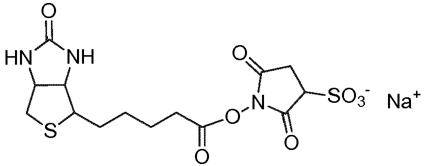
SO₃NHS-LC-biotin of Formula: 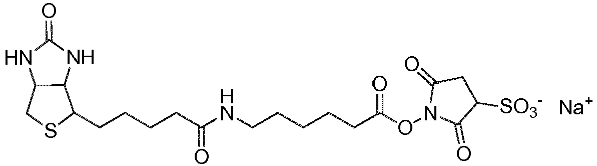
NHS-PEG24-biotin of Formula: 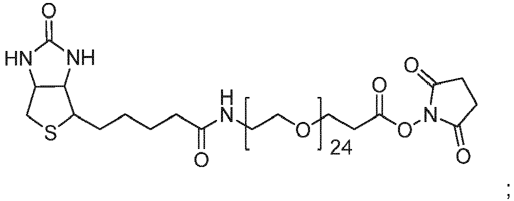
and other known biotin constructs which are readily available for example from commercial sources.

The invention also pertains to a method according to embodiment 1 wherein R1 is a fatty acid (FA) moiety. Examples of fatty acid are C₆₋₇₀alkyl, C₆₋₇₀alkenyl, C₆₋₇₀alkynyl chain substituted with at least one carboxylic acid (for example 1, 2, 3 or 4 CO₂H) and optionally further substituted with hydroxyl group. Fatty acid (FA) is preferably conjugated to the target protein or target peptide via a linker L¹. In an embodiment, the invention pertains to a method according to embodiment 1 wherein the R1 is selected from the following acids: 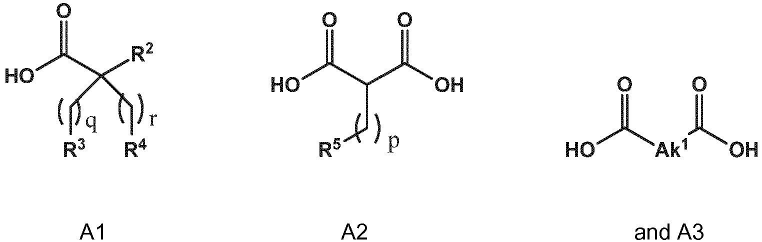
R² is CO₂H, H;
R³, R⁴ and R⁵ are independently of each other H, OH, CO₂H, -CH=CH₂ or -C=CH;
Ak¹ is a branched C₆-C₃₀alkylene;
q, r and p are independently of each other an integer between 6 and 30; and wherein the point of attachment is any of the CO2H functionality; or an amide, an ester or a pharmaceutically acceptable salt thereof.

In an embodiment, R1 is selected from the following fatty acids: 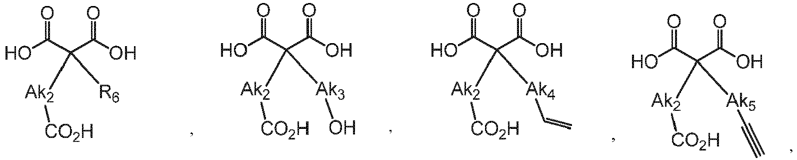 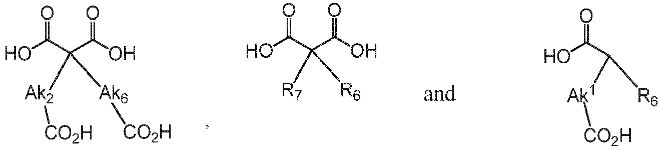 wherein Ak², Ak³, Ak⁴, Ak⁵ and Ak⁶ are independently a (C₈₋₂₀)alkylene, R⁶ and R⁷ are independently (C₈₋₂₀)alkyl.

In an embodiment, R1 is selected from the following fatty acids:

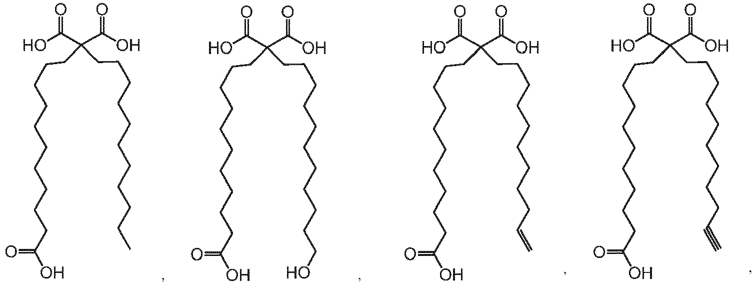

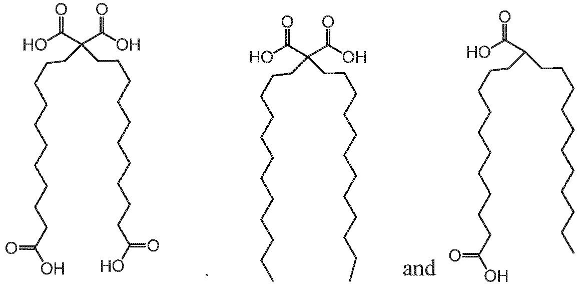

These fatty acid moieties have been described in co-filed US application numbers 62/015,862 (attorney docket number PAT056274-US-PSP), 62/082,327 (Attorney docket number PAT056274-US-PSP02) and 62/107,016 (Attorney docket number PAT056274-US-PSP03).

In an embodiment, the invention pertains to a method wherein the acylating agent is: 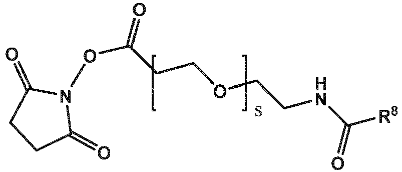
R8 is a linear or branched C₆₋₇₀alkyl, a linear or branched C₆₋₇₀alkenyl or a linear or branched C₆₋₇₀alkynyl, each of which is optionally substituted with optionally substituted with 1 to 3 substituents selected from carboxy and hydroxyl;
s is 1 to 34. In a particular aspect of this embodiment, R8 is 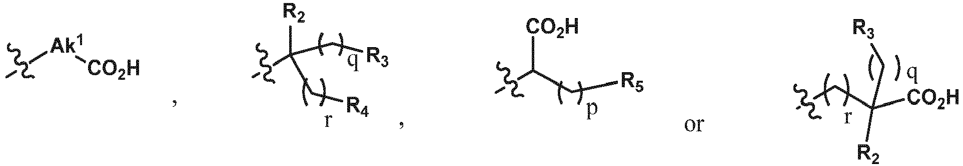
wherein Ak¹, R², R³, R⁴, R⁵, q, r and p are defined supra. Examples of such fatty acid-PEG-NHS moieties have been described in a co-filed application No 62/015,862 (attorney docket number PAT056274-US-PSP) and US applications No 62/082,327 (Attorney docket number PAT056274-US-PSP02) and 62/107,016 (Attorney docket number PAT056274-US-PSP03).

In another embodiment, the invention pertains to a method according to embodiment 1 wherein R1 is a reactive group which is a cycloalkyne moiety and the acylating agent is of Formula:

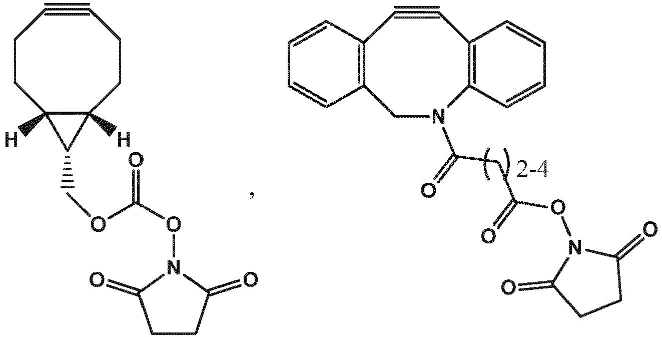

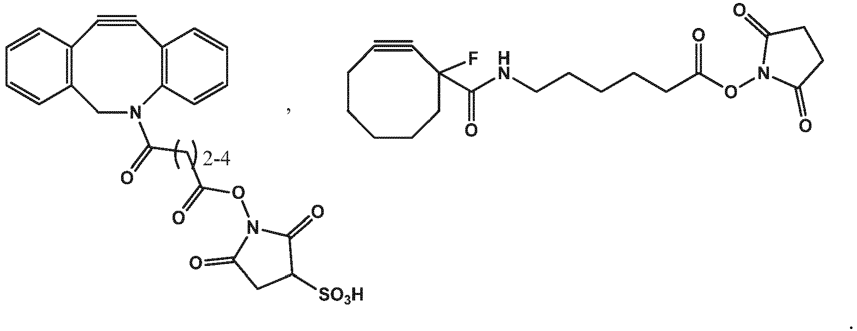

In another embodiment, the reactive group is tetrazine and the acylating agent is of Formula:

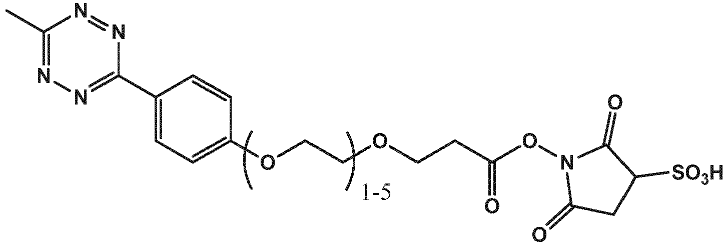

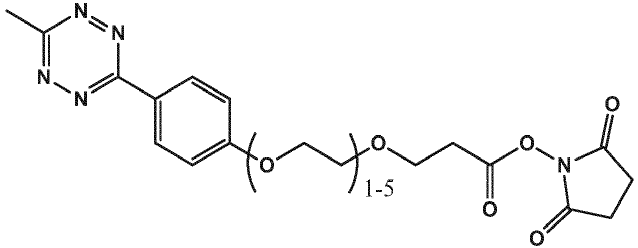

In an embodiment, the invention relates to a method wherein the acylating agent is:

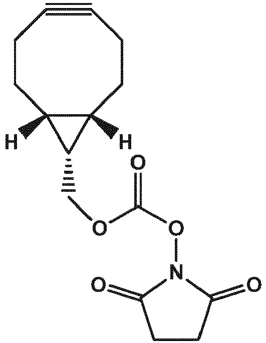

In a further embodiment, the invention pertains to a method further comprising a step of reacting a biointeractive agent or an analytical agent with the reactive group present in the acylating agent, wherein the biointeractive or bionalytical agent is reacted via a functional group wherein the functional group is azide and the biointeractive agent or an analytical agent comprising the azido functional group is: 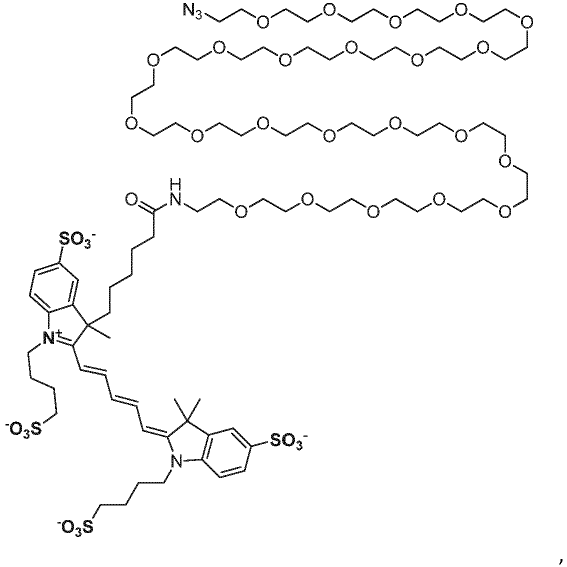 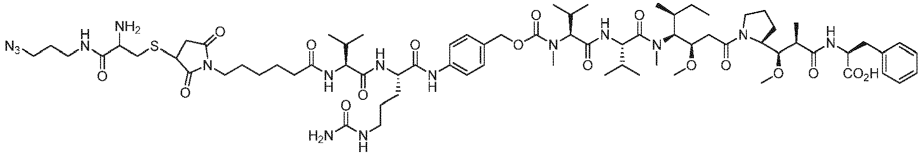 , or 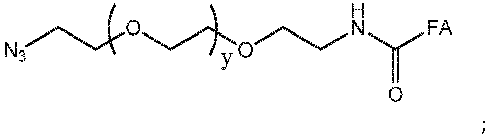 wherein y is 0 to 34 and FA is a fatty acid residue attached via one of its carboxylic acid functionality to the PEG linker and FA has the following Formulae:

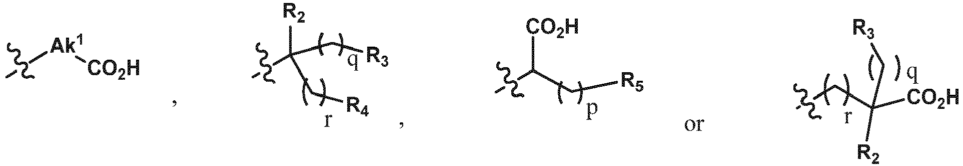

Another example of biointeractive agent comprising an azido functional group is a therapeutic peptide wherein an azido lysine has been introduced (preferably at the N-terminus) optionally via a linker (e.g. polyglycine). An example of such biointeractive agent is.

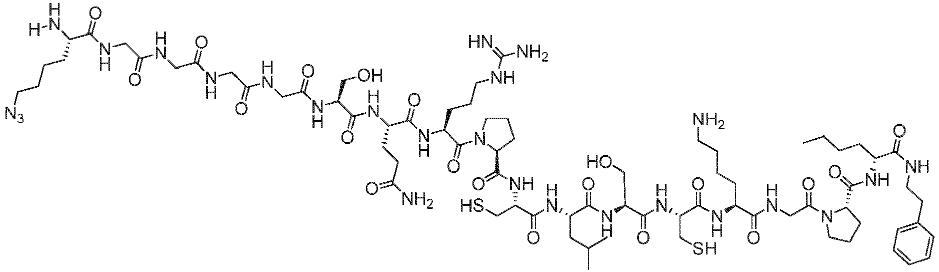

In an embodiment, the invention relates to a method according to any one of the preceding embodiments wherein the target protein or target peptide is APJ agonist peptide, a native Fc or a GDF15 protein or mutant thereof.

In an embodiment, the invention relates to a method according to any one of the preceding embodiments wherein the pH is less than 5.

In an embodiment, the invention relates to a method according to any one of the preceding embodiments wherein the pH is about 4.5. In an embodiment the pH is from 4.5 to 4.8. In an embodiment, the pH is about 4.6. In another embodiment, the pH is about 4.75. The pH can be adjusted using buffer solution of sodium acetate, sodium phosphate or potassium phosphate or combination thereof. Non-limiting examples of buffers are NaOAc, sodium or potassion phosphate dibasic (Na₂HPO₄ or K₂HPO₄).

In an embodiment, the invention relates to a method according to any one of the preceding embodiments wherein the pH is 4.

A conjugate is prepared from the methods disclosed herein. In another disclosure, a vaccine is disclosed that is prepared with a conjugate or a modified protein/peptide disclosed herein. In another disclosure, a therapeutic protein/peptide is disclosed having a modified protein/peptide disclosed herein. In another disclosure, an imaging agent is disclosed having a modified protein/peptide disclosed herein. In another disclosure, a labeling tool is disclosed having modified protein/peptide disclosed herein.

The methods, the conjugates obtained from the methods, the linkers and the biointeractive/analytical agent are also defined in the Examples section below.

### Step a) of the method: Synthesis of target Peptide/Protein so that the resulting protein or peptide contains a histidine amino acid at the position adjacent to the N-terminus amino acid.

A target protein/peptide can be one that already possesses a histidine amino acid at the position adjacent to the N-terminus amino acid. If a target protein does not possess a histidine amino acid at the position adjacent to the N-terminus amino acid, it is possible to modify the protein/peptide according to step a) of the invention. Insertions of amino acid residues in proteins can be brought about by standard techniques known to persons skilled in the art, such as post-translational chemical modification or transgenetic techniques.

The instant method of this disclosure involves the modification of the target peptide or protein so that to activate the amino functionality of the amino acid at the N-terminus.
Step a may be represented by the following schemes 1 to 5:
Scheme 1 describes a step wherein the target protein or peptide is modified by replacing the amino acid adjacent to the N-terminal amino acid of said target protein or target peptide with a histidine amino acid: 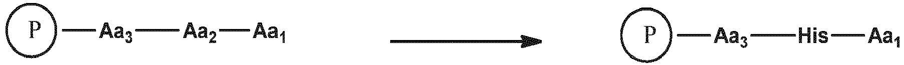 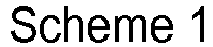 wherein P is a target protein/peptide with its last 3 amino acids at the N-terminus (Aa3, Aa2, Aa1).

Scheme 2 describes a step wherein the target protein or peptide is modified by replacing the two amino acids at the N-terminus (i.e. Aa₁ and Aa₂) of said target protein or target peptide with an amino acid sequence XH- wherein H is histidine and X is an amino acid selected from M, A, Q, N and G:

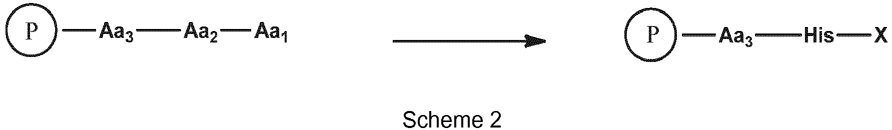

Scheme 3 describes a step wherein the target protein or peptide is modified by replacing the three amino acids at the N-terminus (i.e. Aa₁, Aa₂ and Aa₃) of said target protein or target peptide with an amino acid sequence XHX'- wherein H is Histidine and X and X' are independently M, A, Q, N and G;

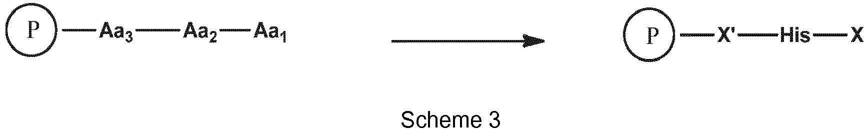

Schemes 4 and 5 describe a step wherein the target peptide or protein is modified by appending a two amino acid sequence of Formula XH or a three amino acid sequence of Formula XHX'- at the N-terminus of said target protein or target peptide, wherein H is histidine and X and X' are independently selected from M, A, Q, N and G:

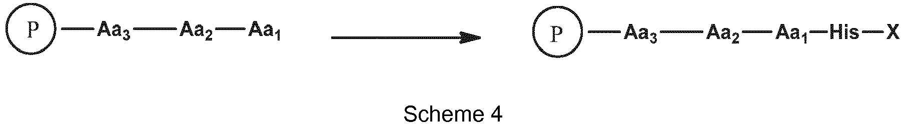

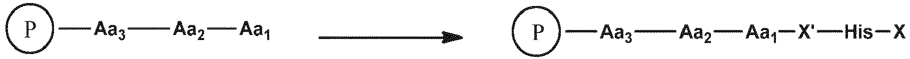

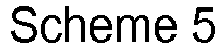

The modified peptides or polypeptides/protein as described supra in Schemes 1 to 5 may be produced by either synthetic chemical processes or by recombinant methods or combination of both methods. The peptides or polypeptides/protein constructs may be prepared as full-length or may be synthesized as non-full length fragments and joined. The peptides and polypeptides used in the present invention method can be produced by the per se known procedures for peptide synthesis. The methods for peptide synthesis may be any of a solid-phase synthesis and a liquid-phase synthesis. Thus, the peptide and polypeptide of interest can be produced by condensing a partial peptide or amino acid capable of constituting the protein with the residual part thereof and, when the product has a protective group, the protective group is detached whereupon a desired peptide can be manufactured. The known methods for condensation and deprotection include the procedures described in the following literature (1) - (5).
(1) M. Bodanszky and M. A. Ondetti, Peptide Synthesis, Interscience Publishers, New York, 1966,
(2) Schroeder and Luebke, The Peptide, Academic Press, New York, 1965,
(3) Nobuo Izumiya et al.. Fundamentals and Experiments in Peptide Synthesis, Maruzen, 1975,
(4) Haruaki Yajima and Shumpei Sakakibara, Biochemical Experiment Series 1, Protein Chemistry IV, 205, 1977, and
(5) Haruaki Yajima (ed.), Development of Drugs-Continued, 14, Peptide Synthesis, Hirokawa Shoten.

After the reaction, the peptide or polypeptide can be purified and isolated by a combination of conventional purification techniques such as solvent extraction, column chromatography, liquid chromatography, size exclusion chromatography (SEC) and recrystallization. Where the peptide isolated as above is a free compound, it can be converted to a suitable salt by the known method. Conversely where the isolated product is a salt, it can be converted to the free peptide by the known method.

The amide of polypeptide can be obtained by using a resin for peptide synthesis which is suited for amidation. The resin includes chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, 4-(2',4'-dimethoxyphenyl-Fmoc-aminomethyl)phenoxy resin, 2-chlorotrityl chloride resin, and so on. Using such a resin, amino acids whose α-amino groups and functional groups of side-chain have been suitably protected are condensed on the resin according to the sequence of the objective peptide by various condensation techniques which are known per se. At the end of the series of reactions, the peptide or the protected peptide is removed from the resin and the protective groups are removed and if necessary, disulfide bonds are formed to obtain the objective polypeptide.

For the condensation of the above-mentioned protected amino acids, a variety of activating reagents for peptide synthesis can be used such as HATU, HCTU or e.g. a carbodiimide. The carbodiimide includes DCC, N,N' -diisopropylcarbodiimide, and N-ethyl-N'-(3-dimethylaminopropyl)carbodiimide. For activation with such a reagent, a racemization inhibitor additive, e.g. HOBt or Oxyma Pure can be used. The protected amino acid can be directly added to the resin along with the activation reagents and racemization inhibitor or be preactivated as symmetric acid anhydride, HOBt ester, or HOOBt ester then added to the resin. The solvent for the activation of protected amino acids or condensation with the resin can be properly selected from among those solvents which are known to be useful for peptide condensation reactions. For example, N,N-dimethylformamide, N-methylpyrrolidone, chloroform, trifluoroethanol, dimethyl sulfoxide, DMF, pyridine, dioxane, methylene chloride, tetrahydrofuran, acetonitrile, ethyl acetate, or suitable mixtures of them can be mentioned. The reaction temperature can be selected from the range hitherto-known to be useful for peptide bond formation and is usually selected from the range of about -20°C - 50°C. The activated amino acid derivative is generally used in a proportion of 1.5-4 fold excess. If the condensation is found to be insufficient by a test utilizing the ninhydrin reaction, the condensation reaction can be repeated to achieve a sufficient condensation without removing the protective group. If repeated condensation still fails to provide a sufficient degree of condensation, the unreacted amino group can be acetylated with acetic anhydride or acetylimidazole.

The protecting group of amino group for the starting material amino acid includes Z, Boc, tertiary-amyloxycarbonyl, isobornyloxycarbonyl, 4-methoxybenzyloxycarbonyl, Cl-Z, Br-Z , adamantyloxycarbonyl, trifluoroacetyl, phthalyl, formyl, 2-nitrophenylsulfenyl, diphenylphosphinothioyl, or Fmoc. The carboxy-protecting group that can be used includes but is not limited to the above-mentioned C₁₋₆ alkyl, C₃-₈ cycloalkyl and C₆₋₁₀aryl-C₁₋₂alkyl as well as 2-adamantyl, 4-nitrobenzyl, 4-methoxybenzyl, 4-chlorobenzyl, phenacyl, benzyloxycarbonylhydrazido, tertiary-butoxycarbonylhydrazido, and tritylhydrazido.

The hydroxy group of serine and threonine can be protected by esterification or etherification. The group suited for said esterification includes carbon-derived groups such as lower alkanoyl groups, e.g. acetyl etc., aroyl groups, e.g. benzoyl etc. , benzyloxycarbonyl, and ethoxycarbonyl. The group suited for said etherification includes benzyl, tetrahydropyranyl, and tertiary-butyl. The protective group for the phenolic hydroxyl group of tyrosine includes Bzl, Cl₂-Bzl, 2-nitrobenzyl, Br-Z, and tertiary-butyl.

The protecting group of imidazole for histidine includes Tos, 4-methoxy-2,3,6-tri ethylbenzenesulfonyl, DNP, benzyloxymethyl, Bum, Boc, Trt, and Fmoc.

The activated carboxyl group of the starting amino acid includes the corresponding acid anhydride, azide and active esters, e.g. esters with alcohols such as pentachlorophenol, 2,4,5-trichlorophenol, 2,4-dinitrophenol, cyanomethyl alcohol, p-nitrophenol, HONB, N-hydroxysuccinimide, N-hydroxyphthalimide, HOBt, etc. The activated amino group of the starting amino acid includes the corresponding phosphoramide.

The method for elimination of protective groups includes catalytic reduction using hydrogen gas in the presence of a catalyst such as palladium black or palladium-on-carbon, acid treatment with anhydrous hydrogen fluoride, methanesulfonic acid, trifluoromethanesulfonic acid, trifluoroacetic acid, or a mixture of such acids, base treatment with diisopropylethylamine, triethylamine, piperidine, piperazine, reduction with sodium metal in liquid ammonia. The elimination reaction by the above-mentioned acid treatment is generally carried out at a temperature of -20°C - 40°C and can be conducted advantageously with addition of a cation acceptor such as anisole, phenol, thioanisole, m-cresol, p-cresol, dimethyl sulfide, 1,4-butanedithiol, 1,2-ethanedithiol. The 2,4-dinitrophenyl group used for protecting the imidazole group of histidine can be eliminated by treatment with thiophenol, while the formyl group used for protecting the indole group of tryptophan can be eliminated by alkali treatment with dilute sodium hydroxide solution or dilute aqueous ammonia as well as the above-mentioned acid treatment in the presence of 1,2-ethanedithiol, 1,4-butanedithiol.

The method for protecting functional groups which should not take part in the reaction of the starting material, the protective groups that can be used, the method of removing the protective groups, and the method of activating the functional groups that are to take part in the reaction can all be selected judicially from among the known groups and methods.

Another method for obtaining the amide form of the polypeptide comprises amidating the -carboxyl group of the C-terminal amino acid at first, then extending the peptide chain to the N-side until the desired chain length, and then selectively deprotecting the α-amino group of the C-terminal peptide and the α-carboxy group of the amino acid or peptide that is to form the remainder of the objective polypeptide and condensing the two fragments whose α-amino group and side-chain functional groups have been protected with suitable protective groups mentioned above in a mixed solvent such as that mentioned hereinbefore. The parameters of this condensation reaction can be the same as described hereinbefore. From the protected peptide obtained by condensation, all the protective groups are removed by the above-described method to thereby provide the desired crude peptide. This crude peptide can be purified by known purification procedures and the main fraction be lyophilized to provide the objective amidated polypeptide. To obtain an ester of the polypeptide, the α-carboxyl group of the C-terminal amino acid is condensed with a desired alcohol to give an amino acid ester and then, the procedure described above for production of the amide is followed.

Alternatively, recombinant expression methods are particularly useful for the production of the modified peptide or protein as described in Schemes 1 to 5. Recombinant protein expression using a host cell (a cell artificially engineered to comprise nucleic acids encoding the sequence of the peptide and which will transcribe and translate, and optionally, secrete the peptide into the cell growth medium) is used routinely in the art. For recombinant production process, a nucleic acid coding for amino acid sequence of the peptide would typically be synthesized by conventionally methods and integrated into an expression vector. Such methods is particularly preferred for manufacture of the polypeptide compositions comprising the peptides fused to additional peptide sequences or other proteins or protein fragments or domains. The host cell can optionally be at least one selected from from E.Coli, COS-1, COS-7, HEK293, BHT21, CHO, BSC-1, Hep G2, 653, SP2/0, 293, heLa, myeloma, lymphoma, yeast, insect or plant cells, or any derivative, immortalized or transformed cell thereof.

The disclosure also encompasses polynucleotides encoding the above-described variants that may be in the form of RNA or in the form of DNA, which DNA includes cDNA, genomic DNA, and synthetic DNA. The DNA may be double-stranded or single-stranded. The coding sequences that encode the compositions of the present invention may vary as a result of the redundancy or degeneracy of the genetic code.

The polynucleotides that encode for the compositions of the present invention may include the following: only the coding sequence for the variant, the coding sequence for the variant and additional coding sequence such as a functional polypeptide, or a leader or secretory sequence or a pro-protein sequence; the coding sequence for the variant and non-coding sequence, such as introns or non-coding sequence 5' and/or 3' of the coding sequence for the variant. Thus the term "polynucleotide encoding a variant" encompasses a polynucleotide that may include not only coding sequence for the variant but also a polynucleotide, which includes additional coding and/or non-coding sequence.

The disclosure further relates to variants of the described polynucleotides that encode for fragments, analogs and derivatives of the polypeptide that contain the indicated substitutions. The variant of the polynucleotide may be a naturally occurring allelic variant of the human GDF15 sequence, a non-naturally occurring variant, or a truncated variant as described above. Thus, the present disclosure also includes polynucleotides encoding the variants described above, as well as variants of such polynucleotides, which variants encode for a fragment, derivative or analog of the disclosed variant. Such nucleotide variants include deletion variants, substitution variants, truncated variants, and addition or insertion variants as long as at least one of the indicated amino acid substitutions of the first embodiment is present.

The polynucleotides can be expressed in hosts after the sequences have been operably linked to (i.e., positioned to ensure the functioning of) an expression control sequence. These expression vectors are typically replicable in the host organisms either as episomes or as an integral part of the host chromosomal DNA. Commonly, expression vectors will contain selection markers, e.g., tetracycline, neomycin, and dihydrofolate reductase, to permit detection of those cells transformed with the desired DNA sequences. The GDF15 variant can be expressed in mammalian cells, insect, yeast, bacterial or other cells under the control of appropriate promoters. Cell free translation systems can also be employed to produce such proteins using RNAs derived from DNA constructs of the present invention.

*Escherichia Coli (E. coli)* is a prokaryotic host useful particularly for cloning the polynucleotides of the present disclosure. Other microbial hosts suitable for use include *Bacillus subtilus, Salmonella typhimurium,* and various species of *Serratia, Pseudomonas, Streptococcus,* and *Staphylococcus,* although others may also be employed as a matter of choice. In these prokaryotic hosts, one can also make expression vectors, which will typically contain expression control sequences compatible with the host cell (e.g., an origin of replication). In addition, any of a number of well-known promoters may be present, such as the lactose promoter system, a tryptophan (Trp) promoter system, a beta-lactamase promoter system, or a promoter system from phages lambda or T7. The promoters will typically control expression, optionally with an operator sequence, and have ribosome binding site sequences and the like, for initiating and completing transcription and translation.

One skilled in the art of expression of proteins will recognize that methionine or methionine-arginine sequence can be introduced at the N-terminus of the mature sequence for expression in *E. coli* and are contemplated within the context of this invention. Thus, unless otherwise noted, compositions of the present invention expressed in *E. coli* have a methionine sequence introduced at the N-terminus.

Other microbes, such as yeast or fungi, may also be used for expression. *Pichia pastoris, Saccharomyces cerevisiae, Schizosaccharomyces pombe,* and *Pichia angusta* are examples of preferred yeast hosts, with suitable vectors having expression control sequences, such as promoters, including 3-phosphoglycerate kinase or other glycolytic enzymes, and an origin of replication, termination sequences and the like as desired. Aspergillus *niger, Trichoderma reesei;* and *Schizophyllum commune,* are examples of fungi hosts, although others may also be employed as a matter of choice.

Mammalian tissue cell culture may also be used to express and produce the polypeptides. A number of suitable host cell lines capable of secreting intact variants have been developed in the art, and include the CHO cell lines, various COS cell lines, NSO cells, Syrian Hamster Ovary cell lines, HeLa cells, or human embryonic kidney cell lines (i.e. HEK293, HEK293EBNA).

Expression vectors for mammalian cells can include expression control sequences, such as an origin of replication, a promoter, an enhancer, and necessary processing information sites, such as ribosome binding sites, RNA splice sites, polyadenylation sites, and transcriptional terminator sequences. Preferred expression control sequences are promoters derived from SV40, adenovirus, bovine papilloma virus, cytomegalovirus, Raus sarcoma virus, and the like. Preferred polyadenylation sites include sequences derived from SV40 and bovine growth hormone.

The vectors containing the polynucleotide sequences of interest (e.g., that encode the compositions of the present invention and expression control sequences) can be transferred into the host cell by well-known methods, which vary depending on the type of cellular host. For example, calcium chloride transfection is commonly utilized for prokaryotic cells, whereas calcium phosphate treatment or electroporation may be used for other cellular hosts.

Various methods of protein purification may be employed and such methods are known in the art and described, for example, in Deutscher, Methods in Enzymology 182: 83-9 (1990) and Scopes, Protein Purification: Principles and Practice, Springer-Verlag, NY (1982). The purification step(s) selected will depend, for example, on the nature of the production process used for the compositions.

The polypeptides may be prepared in substantially pure or isolated form (e.g. , free from other polypeptides). The polypeptides can be present in a composition that is enriched for the polypeptide relative to other components that may be present (e.g. , other polypeptides or other host cell components). For example, purified polypeptide may be provided such that the polypeptide is present in a composition that is substantially free of other expressed proteins, e.g. , less than 90%, less than 60%, less than 50%, less than 40%, less than 30%, less than 20%, less than 10%, less than 5%, or less than 1%, of the composition is made up of other expressed proteins.

### Step b) of the method: Site specific conjugation to the modified protein/peptide (from step a) - Selective acetylation of the amino group at the N-terminus of the modified peptide/protein

The acylation of the N-terminus amino group of a target protein or peptide may be represented by the following Scheme 6: 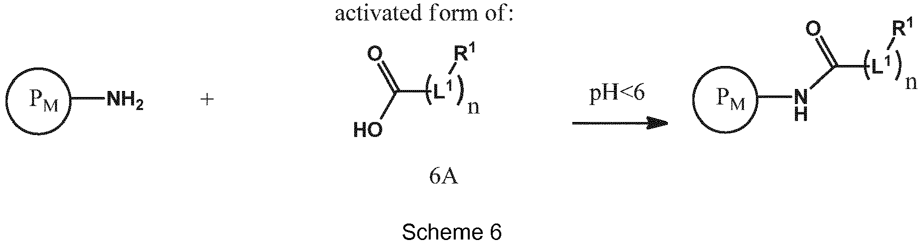 wherein P_{M} is a modified peptide or protein according to step a), R¹, L¹ and n are as defined supra and the activated form of the acid **6A** is the NHS-ester. The acylation reaction is carried out in aqueous media at a pH of less than 6. In another embodiment, the acylation reaction is carried out at a pH of less than 5. In yet another embodiment, the acylation reaction is carried out at a pH of about 4. In yet another embodiment, the acylation reaction is carried out at a pH of about 4.5. In yet another embodiment, the acylation reaction is carried out at a pH between 4.5 to 4.8. In yet another embodiment the pH is about 4.6. In yet another embodiment, the pH is about 4.75. The pH can be adjusted using buffer solution of sodium acetate, sodium phosphate or potassium phosphate or combination thereof. Non-limiting examples of buffers are NaOAc, sodium or potassion phosphate dibasic (Na₂HPO₄ or K₂HPO₄).

R¹ may be a biointeractive agent or an analytical agent.

Alternatively, R¹ may be a moiety comprising a reactive group that facilitates covalent attachment to a biointeractive agent or to an analytical agent.

When R1 is a moiety comprising a reactive group, step b) of the method of the invention may be represented by the following scheme 7: 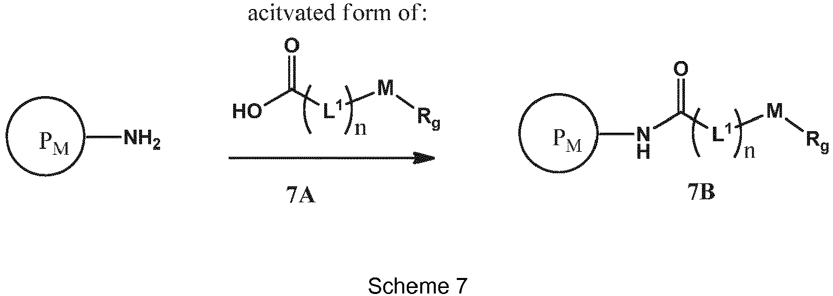 wherein P_{M}, L¹, and n are defined supra; M is a chemical moiety and R_{g} is a reactive group. Examples of R_{g} are acyl, ester or mixed anhydride, alkyne, tetrazine, maleimide, imidate, carboxy.

The method of the invention may further comprise a step of reacting a biointeractive agent or an analytical agent with the reactive group R_{g} of conjugate **7B,** wherein the biointeractive or bionalytical agent is reacted via a corresponding functional group and optionally via a linker L2.

This step may be represented by the following Scheme 8: 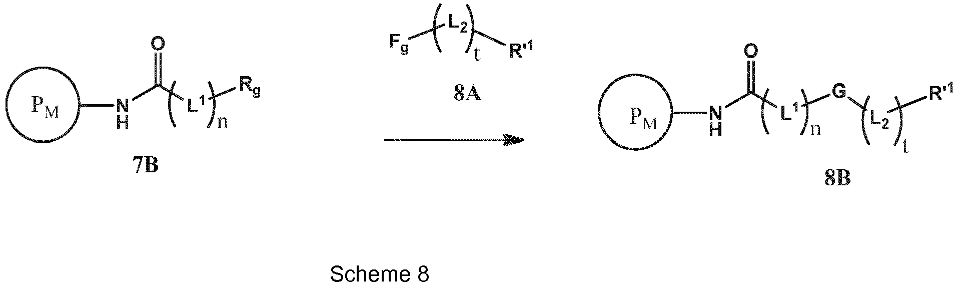
wherein P_{M} is a target protein or target peptide which has been modified according to step a) so that the resulting protein or peptide contains a histidine amino acid at the position adjacent to the N-terminus amino acid;
L¹ and L² are linkers;
R_{g} is a reactive group;
F_{g} is a functional group;
G is the resulting chemical group formed as a result of reacting R_{g} with F_{g};
R'1 is a biointeractive or analytical agent;
n and t are independently 0 or 1.

Examples of functional group (Fg) are amino group, azide group, alkene group, thiol group or hydrazine, hydroxylamine groups.

Functional group (Fg) include: 1. thiol groups for reacting with reactive groups such as maleimides, tosyl sulfone or pyridine-2-yldisulfanyl; 2. amino groups (for example amino functionality of an amino acid) for reacting with reactive group such as carboxylic acid or activated carboxylic acid (e.g. amide bond formation via N-hydroxysuccinamide chemistry), phosphoryl groups, acyl group or mixed anhydride; 3. Azide to undergo a Huisgen cycloaddition with an alkyne and more particularly a cyclooctyne reactive group (more commonly known as click chemistry); 4. carbonyl group to react with a reactive group selected from hydroxylamine or hydrazine to form oxime or hydrazine respectively; 5. Alkene to react with a tetrazine reactive group in an aza [4+2] addition. While several examples of linkers and functionalities/reactive group are described herein, the invention contemplates linkers or any length and composition.

In one embodiment, the invention pertains to a method wherein R¹ is a moiety containing an alkyne reactive group (i.e. R_{g} is alkyne or cycloalkyne or bicycloalkyne) that facilitates covalent attachment to a biointeractive agent containing an azide functionality (i.e.: in compound **8A,** t is 0 and F_{g} is azido). Alternatively the biointeractive or analytical agent may be attached to a linker L² which contains an azide functionality at a terminal position (i.e.: in compound **8A** t is 1 and F_{g} is azido). Moieties containing an alkyne reactive group are readily available from commercial sources. Of particular interest are cycloalkyne moieties. Examples of the use of cyclic alkyne in click chemistry for protein labeling has been described in US 2009/0068738. Specific examples have been described supra.

This chemistry involves an azide-alkyne Huisgen cycloaddition, which is more commonly known as click chemistry. In this particular embodiment, the resulting chemical group **G** which is formed as a result of the azide-alkyne cycloaddition is a triazole as illustrated in scheme 9: 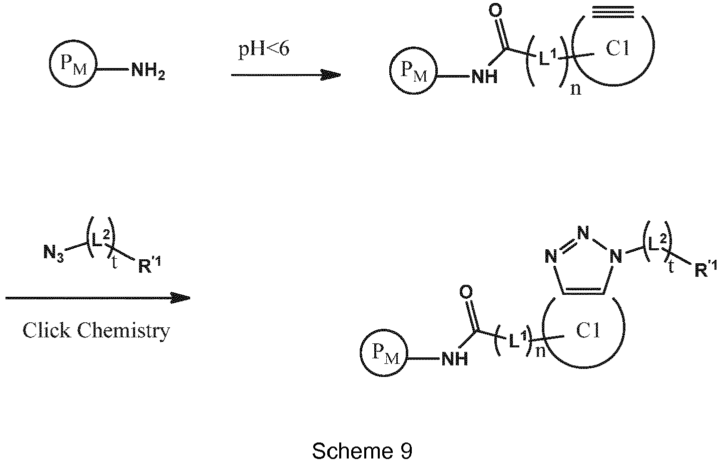 wherein all variables have been described supra and C1 is a mono, di or tricyclic carbocyclic or heterocyclic ring system optionally substituted with fluorine. In one embodiment, L¹ is a C1-C20 alkylene linker wherein the alkylene chain is optionally substituted with oxo (=O), and wherein one or more carbon is replaced with O or NH.

In another embodiment, the invention pertains to a method wherein R1 is a moiety containing a tetrazine reactive group to facilitate covalent attachment to a biointeractive/analytical agent containing an alkene functionality (preferably a strained alkene) (i.e.: in compound **8A,** s is 0 and F_{g} is alkene or cycloalkene). Alternatively the biointeractive or analytical agent may be attached to a linker L² which contains an alkene functionality at a terminal position (i.e.: in compound **8A** s is 1 and F_{g} is alkene or cycloalkene). Tetrazine moieties are readily available from commercial sources and non-limiting examples have been described supra.This chemistry is known as an aza [4+2] cycloadditon. In this particular embodiment, the resulting chemical group **G** which is formed as a result of the tetrazine-alkene cycloaddition is a multicyclic group of Formula: 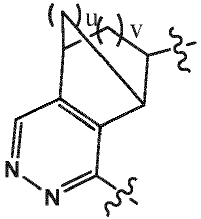 wherein u is an integer of 0 to 2 and v is an integer of 0 to 3.

Scheme 10 illustrates the further derivatisation using tetrazine-alkene cycloaddition

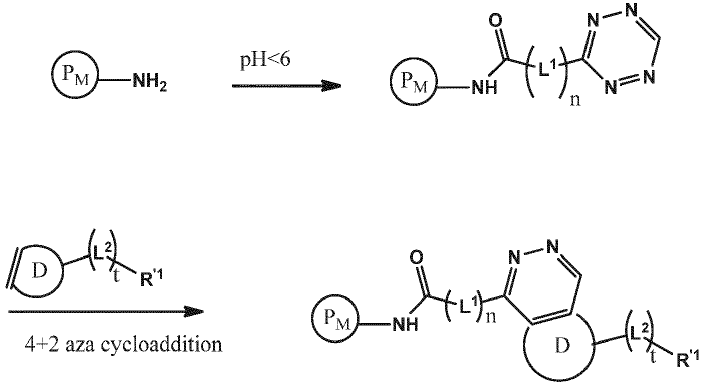

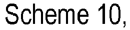

Wherein the variables are as defined supra and D is a strained alkene, i.e. a mono or bicycloakenyl ring system.

In another disclosure, the disclosure pertains to a method wherein R1 is a moiety containing a maleimide reactive group to facilitate covalent attachment to a biointeractive/analytical agent containing a thiol functionality (i.e.: in compound **8A,** s is 0 and F_{g} is -SH). Alternatively the biointeractive or analytical agent may be attached to a linker L² which contains an alkene functionality at a terminal position (i.e.: in compound **8A** s is 1 and F_{g} is - SH). Maleimide containing moieties are readily available from commercial sources. In this particular embodiment, the resulting chemical group **G** which is formed as a result of the thiol-maleimide reaction is of Formula:

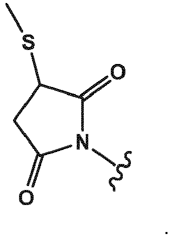

Scheme 11 illustrates the maleimide-thiol further derivatisation:

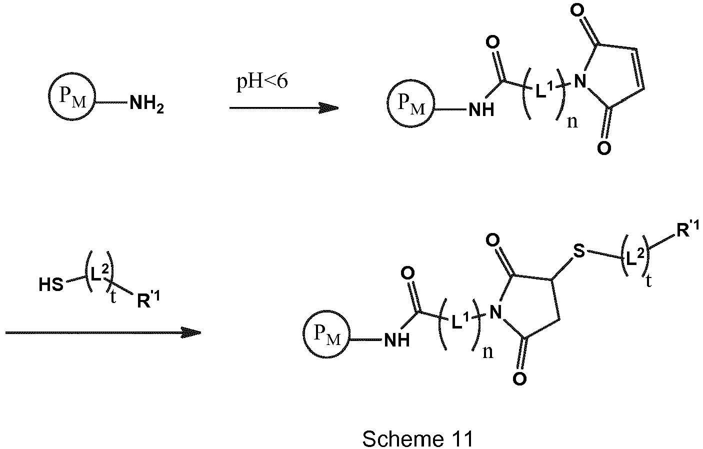

The chemistry and reagents described supra are used to generate the construct F_{g}-(L²)ₜ-R'¹ and/or activated form of HO-C(O)-(L¹)ₙ-R¹ and/or activated form of HO-C(O)-(L¹)ₙ-R_{g}. Each linking unit may possess the appropriate reactive group/functional group at each terminal. The alkyne(cycloalkyne), maleimide or tetrazine reactive groups are reacted with a functional group (azide, thiol and alkene respectively) which is present on the reacting partner.

### Medical and diagnostic applications of the method of the invention

Thus, it may be desirable to modify proteins to alter the physico-chemical properties of the protein/peptide, such as e.g. to increase (or to decrease) solubility to modify the bioavailability of therapeutic proteins. It may be desirable to modify the clearance rate in the body by conjugating compounds to the protein/peptide which binds to plasma proteins, such as e.g. albumin, or which increase the size of the protein to prevent or delay discharge through the kidneys. Conjugation may also alter and in particular decrease the susceptibility of a protein/peptide to hydrolysis, such as e.g. in vivo proteolysis.

In another disclosure, it may be desirable to conjugate a label to facilitate analysis of the protein/peptide. Examples of such labels include radioactive isotopes, fluorescent markers such as the fluorophores already described and enzyme substrates.

In still another disclosure, a compound is conjugated to a protein to facilitate isolation of the protein. For example, a compound with a specific affinity to a particular column material may be conjugated to the protein. It may also be desirable to modify the immunogenicity of a protein, e.g. by conjugating a protein so as to hide, mask or eclipse one or more immunogenic epitopes at the protein.

In one disclosure, the disclosure provides a method of improving pharmacological properties of target proteins. The improvement is with respect to the corresponding unmodified protein. Examples of such pharmacological properties include functional in vivo half-life, immunogenicity, renal filtration, protease protection and albumin binding or other plasma protein binding of any specific protein.

### Therapeutic Uses of the Modified Proteins

To the extent that the unmodified protein/peptide is a therapeutic protein, the invention also relates to the modified proteins for use in therapy, and in particular to pharmaceutical compositions comprising the modified proteins. Thus, as used herein, the terms "treatment" and "treating" mean the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications, The patient to be treated is preferably a mammal, in particular a human being, but it may also include animals, such as dogs, cats, cows, sheep and pigs. Nonetheless, it should be recognized that therapeutic regimens and prophylactic (preventative) regimens represents separate aspects for the uses disclosed herein and contemplated by treating physician or veterinarian.

A "therapeutically effective amount" of a modified protein as used herein means an amount sufficient to cure, alleviate or partially arrest the clinical manifestations of a given disease and its complications. An amount adequate to accomplish this is defined as "therapeutically effective amount". Effective amounts for each purpose will depend on e.g. the severity of the disease or injury as well as the weight, sex, age and general state of the subject. It will be understood that determining an appropriate dosage may be achieved using routine experimentation, by constructing a matrix of values and testing different points in the matrix, which is all within the ordinary skills of a trained physician or veterinarian.

The methods and compositions disclosed herein provide modified proteins for use in therapy. As such, a typical parenteral dose is in the range of 10-9 mg/kg to about 100 mg/kg body weight per administration. Typical administration doses are from about 0.0000001 to about 10 mg/kg body weight per administration. The exact dose will depend on e.g. indication, medicament, frequency and mode of administration, the sex, age and general condition of the subject to be treated, the nature and the severity of the disease or condition to be treated, the desired effect of the treatment and other factors evident to the person skilled in the art. Typical dosing frequencies are twice daily, once daily, bi-daily, twice weekly, once weekly or with even longer dosing intervals. Due to the prolonged half-lives of the active compounds compared to the corresponding un-conjugated protein, dosing regimen with long dosing intervals, such as twice weekly, once weekly or with even longer dosing intervals is a particular embodiment, . Many diseases are treated using more than one medicament in the treatment, either concomitantly administered or sequentially administered. It is, therefore, contemplated that the modified proteins in therapeutic methods for the treatment of one of the diseases can be used in combination with one or more other therapeutically active compound normally used in the treatment of a disease. It is also contemplated that the use of the modified protein in combination with other therapeutically active compounds normally used in the treatment of a disease in the manufacture of a medicament for that disease.

### Pharmaceutical composition

The conjugate prepared using the method of the instant invention may be administered in any of a variety of ways, including subcutaneously, intramuscularly, intravenously, intraperitoneally, inhalationally, intranasally, orally etc. Particularly preferred employ continuous intravenous administration of the conjugates, or an amide, ester, or salt thereof. The conjugates may be administered as a bolus or as a continuous infusion over a period of time. An implantable pump may be used. Intermittent or continuous conjugates administration may be continued for one to several days (e.g., 2-3 or more days), or for longer periods of time, e.g., weeks, months, or years. Intermittent or continuous conjugates administration may be provided for at least about 3 days, preferably at least about 6 days. Intermittent or continuous conjugate administration may be provided for at least about one week. Intermittent or continuous conjugate administration may be provided for at least about two weeks. It may be desirable to maintain an average plasma conjugate concentration above a particular threshold value either during administration or between administration of multiple doses. A desirable concentration may be determined, for example, based on the subject's physiological condition, disease severity, etc. Such desirable value(s) can be identified by performing standard clinical trials. Alternatively, the peptides and conjugates thereof could be delivered orally via FcRn mechanism. (Nat Rev Immunol. 7(9), 715-25, 2007; Nat Commun. 3;3:610, 2012, Am J Physiol Gastrointest Liver Physiol 304: G262-G270, 2013).

A pharmaceutical composition can be provided comprising a conjugate prepared using the method of the present invention or an amide, an ester or a salt thereof and one or more pharmaceutically acceptable carriers. The pharmaceutical composition can be formulated for particular routes of administration such as oral administration, subcutaneous administration, parenteral administration, and rectal administration, etc. In addition, the pharmaceutical compositions can be made up in a solid form (including without limitation capsules, tablets, pills, granules, lyophilizates, powders or suppositories), or in a liquid form (including without limitation solutions, suspensions or emulsions). The pharmaceutical compositions can be subjected to conventional pharmaceutical operations such as aseptic manufacturing, sterilization and/or can contain conventional inert diluents, cake forming agents, tonicity agents, lubricating agents, or buffering agents, as well as adjuvants, such as preservatives, stabilizers, wetting agents, emulsifers and buffers, etc.

Pharmaceutical compositions suitable for injectable use typically include sterile aqueous solutions (where water soluble) or dispersions and sterile powders for the extemporaneous preparation of sterile injectable solutions or dispersion.

For intravenous administration, suitable carriers include physiological saline, bacteriostatic water, Cremophor ELTM (BASF, Parsippany, N.J.) or phosphate buffered saline (PBS). In all cases, the composition should be sterile and should be fluid to the extent that easy syringability exists. Preferred pharmaceutical formulations are stable under the conditions of manufacture and storage and must be preserved against the contaminating action of microorganisms such as bacteria and fungi. In general, the relevant carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, amino acids, sorbitol, sodium chloride in the composition. Prolonged absorption of the injectable compositions can be brought about by including in the composition an agent which delays absorption, for example, aluminum monostearate and gelatin. A multifunctional excipient such as recombinant albumin may be incorporated into the formulation process to facilitate the stabilization of the conjugate product from degradation and assist in the administration and release of the active component. (BioPharm International, 2012, Vol 23, Issue 3, pp 40-44).

Certain injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, or contain about 1-50%, of the active ingredient.

Sterile injectable solutions can be prepared by incorporating the active compound in the required amount in an appropriate solvent with one or a combination of ingredients enumerated above, as required, followed by filration sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze- drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. Formulations for oral delivery may advantageously incorporate agents to improve stability within the gastrointestinal tract and/or to enhance absorption.

For administration by inhalation, the therapeutic agents are preferably delivered in the form of an aerosol spray from pressured container or dispenser which contains a suitable propellant, e.g., a gas such as carbon dioxide, or a nebulizer. It is noted that the lungs provide a large surface area for systemic delivery of therapeutic agents.

The agents may be encapsulated, e.g., in polymeric microparticles such as those described in U.S. publication 20040096403, or in association with any of a wide variety of other drug delivery vehicles that are known in the art. The agents may be delivered in association with a charged lipid as described, for example, in U.S. publication 20040062718. It is noted that the latter system has been used for administration of a therapeutic polypeptide, insulin, demonstrating the utility of this system for administration of peptide agents.

Systemic administration can also be by transmucosal or transdermal means.

Suitable compositions for transdermal application include an effective amount of a conjugate with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Suitable compositions for topical application, e.g., to the skin and eyes, include aqueous solutions, suspensions, ointments, creams, gels or sprayable formulations, e.g., for delivery by aerosol or the like. Such topical delivery systems will in particular be appropriate for dermal application. They are thus particularly suited for use in topical, including cosmetic, formulations well-known in the art. Such may contain solubilizers, stabilizers, tonicity enhancing agents, buffers and preservatives.

The pharmaceutical composition may be for subcutaneous administration. Suitable formulation components and methods for subcutaneous administration of polypeptide therapeutics (e.g., antibodies, fusion proteins and the like) are known in the art. See, e.g., Published United States Patent Application No 2011/0044977 and US Patent No. 8,465,739 and US Patent No. 8,476,239. Typically, the pharmaceutical compositions for subcutaneous administration contain suitable stabilizers (e.g, amino acids, such as methionine, and or saccharides such as sucrose), buffering agents and tonicifying agents.

As used herein a topical application may also pertain to an inhalation or to an intranasal application. They may be conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomizer or nebuliser, with or without the use of a suitable propellant.

Pharmaceutical compositions and dosage forms can be provided that comprise one or more agents that reduce the rate by which the compound prepared by the method of the present invention as an active ingredient will decompose. Such agents, which are referred to herein as "stabilizers," include, but are not limited to, antioxidants such as ascorbic acid, pH buffers, or salt buffers, recombinant Albumin.

As used herein, the term "pharmaceutically acceptable salts" refers to salts that retain the biological effectiveness and properties of the conjugates and, which typically are not biologically or otherwise undesirable. In many cases, the conjugates are capable of forming acid and/or base salts by virtue of the presence of amino and/or carboxyl groups or groups similar thereto.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfornate, chloride/hydrochloride, chlortheophyllonate, citrate, ethandisulfonate, fumarate, gluceptate, gluconate, glucuronate, hippurate, , hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, multifunctional excipient such as recombinant albumin and the like and combinations thereof, as would be known to those skilled in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

### Example of the invention

### Method Experimental

### Abbreviations

| | |
|---|---|
| ACN | acetronitrile |
| BOC | *tert*-Butyloxycarbonyl |
| DIEA | diisopropylethylamine |
| DIPEA | *N,N*'-Diisopropylethylamine |
| DMF | *N,N'*-Dimethylformamide |
| DMSO | Dimethylsulfoxide |
| DTT | Dithiothreitol |
| Fmoc | fluorenylmethyloxycarbonyl chloride |
| HATU | 1-[Bis(dimethylamino)methylene]-1H-1,2,3-triazolo[4,5-b]pyridinium-3-oxid hexafluorophosphate |
| HCTU | 2-(6-Chloro-1H-benzotriazole-1-yl)-1,1,3,3-tetramethylaminium hexafluorophosphate |
| HOBt | Hydroxybenzotriazole |
| HPLC | High performance liquid chromatography |
| HPF6 | hexafluorophosphoric acid |
| LC | liquid chromatography |
| MALDI | Matrix-assisted laser desorption ionization NHS N-hydrosuccinimide |
| MS | Mass spectrometry |
| NMP | N-methylpyrrolidone |
| NMR | nuclear magnetic resonance |
| PBS | Phosphate buffered saline |
| PEG | polyethylene glycol |
| PS | peptide synthesis |
| PG | protective group |
| RP-HPLC | reverse phase HPLC |
| SM | starting material |
| SCF | super critical fluid |
| SPPS | Solid phase peptide synthesis |
| TIC | total in current |
| TIPS | triisopropylsilane |
| TFA | trifluoracetic acid |
| Trt | trityl |
| UPLC | Ultra performance liquid chromatography |

### LCMS Methods

| | | |
|---|---|---|
| **Method A** | | |
| | Column | Acquity BEH 1.7µm 2.1x50mm |
| | Column Temperature | 50 C |
| | Eluents | A: Water (0.1% formic acid); B: ACN (0.1% formic acid) |
| | Flow Rate | 1 mL/min |
| | Gradient | 0 min 40% B; 40% to 98% B in 1.40 min; 2.05 min 98% B; 2.1 min 40%B |
| | Mass Spectrometer | Single Quadrupole ESI scan range 120-1600 |
| | UPLC | Waters Acquity |
| **Method B** | | |
| | Column | Proswift Monolith 4.6x50mm |
| | Column Temperature | 50 C |
| | Eluents | A: Water (0.1% formic acid); B: ACN (0.1% formic acid) |
| | Flow Rate | 1 mL/min |
| | Gradient | 0.7 min 2% B; 2% to 60% B in 12.8 min; 14 min 60% B; 14.2 min 2% B |
| | Mass Spectrometer | Qtof ESI scan range 600-3500; deconvoluted by Max Ent 1 in Mass Lynx software package |
| | UPLC | Waters Acquity |
| **Method C** | | |
| | Column | Hilic 2.1 x 100mm |
| | Column Temperature | 55 C |
| | Eluents | A: CO₂ B: MeOH |
| | Flow Rate | 2mL/min |
| | Gradient | 0.15 min 2% B; 2% to 50% B in 1.5 min; 2.1 min 50% B; 2.25 min 2% B; 2.5 min 2% B |
| | Mass Spectrometer | Single Quadrupole ESI |
| | SCF | Waters Acquity |
| **Method D** | | |
| | Column | Proswift Monolith 4.6 x 50mm |
| | Column Temperature | 50 C |
| | Eluents | A: Water (0.1% formic acid) B: ACN (0.1% formic acid) |
| | Flow Rate | 1mL/min |
| | Gradient | 0 min 3% B; 3% to 90% B in 7 min; 7.1 min 15% B; 7.70 min 95% B; 7.8 min 3% B |
| | Mass Spectrometer | Qtof ESI scan range 100-1900; deconvoluted by Max Ent 1 in Mass Lynx software package |
| | UPLC | Waters Acquity |
| **Method E** | | |
| | Column | XBridge C18 Column, 3.5 µm, 2.1 x 50 mm |
| | Column Temperature | 50 C |
| | Eluents | A: Water (0.1% formic acid) B: ACN |
| | Flow Rate | 1mL/min 0.0 min to 1.21 min; 1 mL/min to 2 mL/min in 0.49 min; 1.5 mL/min to 1.0 mL/min in 0.29 min |
| | Gradient | 0 min 1% B; 1% to 30% B in 1.2 min; 1.21 min 95% B; 1.71 min 1% B |
| | Mass Spectrometer | Single Quadrupole ESI scan range 150-1600 |
| | HPLC | Agilent 1100 |

### UPLC HRMS Method F:

**Column:** Acquity BEH300 C4 1.7 µm, 2.1x50mm
**Eluent A:** Water (0.1% TFA)
**Eluent B:** ACN (0.1% TFA)
**Flow:** 0.5 mL/min
**Temperature:** 40°C
**Gradient:** 20% hold 0.5 min, ramp to 80% ACN in 10 min

### General peptide synthesis (step a of the method of the invention: modifying peptide so that the resulting peptide contains a histidine at the position adjacent to the N-terminus amino acid)

The peptides were synthesized by standard solid phase Fmoc chemistry. The peptides were assembled on the Liberty microwave peptide synthesizer (CEM Corporation, North Carolina, USA). Peptides with an unsubstituted carboxamide on the C-terminus were synthesized from Fmoc protected Rink-Amide-AM-resin (Novabiochem, USA).

Synthesis Cycle: The resin was washed with DMF and DCM. Fmoc was removed by treatment with 20% piperidine/DMF (typicaly 7 ml per 0.1 mmol twice). The resin was washed with DMF and. Coupling was done by addition of the Fmoc-amino acid (5 eq.; 0.2 M solution in DMF), HCTU (5 eq.; 0.5 M solution in DMF), and DIPEA (10 eq.; 2 M solution in NMP) followed by mixing of the suspension with nitrogen at 75 °C for typically 5 to 50 min with microwave power 0 to 20 watts depending on the specific requirements. After washing with DMF the coupling step might be repeated once depending on the specific requirements. The resin was washed with DMF.

The peptides were purified by preparative reversed-phase HPLC. The following column was used: Waters XBridge 30x50mm 5um column. Mobile phases consisted of eluent A (5 mM NH4OH in H2O) and eluent B (ACN). Gradients were designed based on the specific requirements of the separation problem. Pure products were lyophilized from ACN/H2O.

In the following the synthesis of a representative example is described.

### Peptide 1: AHNGDLKF-NH2

### Synthesis of AHNGDLKF-NH2

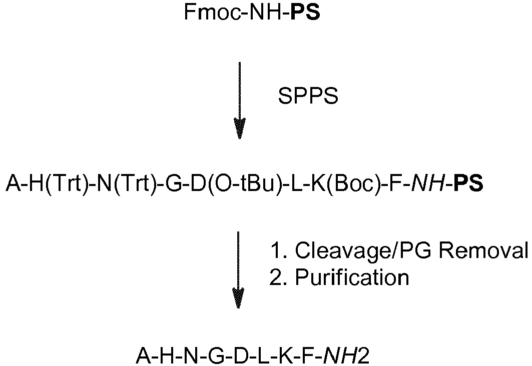

### Preparation of A-H(Trt)-N(Trt)-G-D(O-tBu)-L-K(Boc)-F-NH-PS

Fmoc protected Rink-Amide-AM-resin (316 mg, 0.25 mmol) was subjected to solid phase peptide synthesis (SPPS) on the Liberty microwave peptide synthesizer.

Coupling was performed as follows:

| Coupling | AA | Number of couplings x Reaction time | Temperature °C | Microwave power |
|---|---|---|---|---|
| 1 | F | 1 x 7.5 min | 75 | 20 |
| 2 | K(Boc) | 1 x 7.5 min | 75 | 20 |
| 3 | L | 1 x 7.5 min | 75 | 25 |
| 4 | D(O-tBu) | 1 x 7.5 min | 75 | 20 |
| 5 | G | 1 x 7.5 min | 75 | 20 |
| 6 | N(Trt) | 1 x 7.5 min | 75 | 20 |
| 7 | H(Trt) | 1x2 min | 50 | 0 |
| | | 1x4 min | 50 | 25 |
| 8 | A | 1 x 7.5 min | 75 | 20 |

### Preparation of Peptide 1

### (Cleavage from the resin with concomitant protecting group removal then purification)

A mixture of TFA/H2O/TIPS (95:2.5:2.5) (3 mL, containing 0.2 g DTT) was added to Intermediate (0.25 mmol) and the suspension was shaken at rt for 3 h. The cleavage solution was filtered off and the resin was washed with 95% aq. TFA (1 mL). The combined cleavage and washing solutions were poured onto a mixture of cold diethyl ether (30 mL), giving a precipitate. The suspension was centrifuged and the supernatant poured off. Diethyl ether (30 mL) were added to the residue, the suspension was vortexed for 3 min and centrifuged, and the supernatant was poured off, The wash process was repeated twice. The solid was dried under vacuum The crude was purified by preparative HPLC (gradient: 5-20% B over 3.2 min) and lyophilized from ACN/H2O to afford Example 1 (49 mg, 0.054 mmol). LCMS (Waters Acquity UPLC XBridge C18 3.5µm 3.0x30mm, 40°C, Eluent A: Water + 5 mM NH4OH, Eluent B: ACN, gradient 5% to 95% B/A over 2 mins): Rentention time: 0.70 mins; measured: [M+H]+=900.8; calculated: [M+H]+=901.0).

Peptides 2 and 3 were synthesized in analogy:
**Peptide 2.** ALNGDLKF-NH2
   LCMS Rentention time: 0.81 mins; measured: [M+H]+=876.8; calculated: [M+H]+=877.0).
**Peptide 3 (reference example).** MHNGDHLF-NH2
   LCMS Rentention time: 0.68 mins; measured: [M+H]+=969.8; calculated: [M+H]+=970.1).

### General NHS reactivity assay: (Step b of the invention)

Sulfo-NHS-LC biotin (5 eq) is added to peptide (0.2mg/ml) in pH 4 30 mM NaOAc buffer and reaction stands at room temp. After one hour, reaction is quenched with 200eq hydrazine hydrate and conversion is monitored by LCMS and mapped by MALDI or NMR. The ratio of compounds were estimated based on UV absorption at 214 nm.
SM=starting material
+1=reactivity on N-terminus
+1'=reactivity at lysine
+2=reactivity at both N-terminus and lysine

| Sequence | Results by LC-MS (SM: +1 :+1':+2)* |
|---|---|
| AHNGDLKL | 0:9:1:0 |
| ALNGDLKF | 7:1:0:0 |
| MHNGDHLF | 0:10:n/a:0 |

### Example 1: Step b of the invention using AHNGDLKF-NH2 and Sulfo-NHS-LC biotin as the acylating agent

LCMS Product Rentention time: 1.81 mins; measured: [M+H]+=1239.63; calculated: [M+H]+=1239.6200).

Mapping data (using MALDI LIFT method): Biotin +Alanine: measured [M+H]+=∼411; calculated: [M+H]+=411.21). Biotin +AH: measured [M+H]+=548.27; calculated: [M+H]+=548.27). No Alanine alone mass detected.

### Example 2: Step b of the invention using ALNGDLKF-NH2 and Sulfo-NHS-LC biotin as the acylating agent

LCMS Product Rentention time: 1.29 mins; measured: [M+H]+=876.50; calculated: [M+H]+=877.0). Very little addition (mostly starting material)

### Example 3: Step b using MHNGDHLF -NH2 and Sulfo-NHS-LC biotin as an acylating agent.

LCMS Porduct Rentention time: 2.05 mins; measured: [M+H]+=1308.5972; calculated: [M+H]+=1308.59).

Mapping data (using MALDI LIFT method): Mapping data (using MALDI LIFT method): Biotin +Methionine: measured [M-H]=470.64; calculated: [M-H]=470.66). No Methionine alone mass detected.

### Example 4: Site specific conjugation of Sulfo-NHS-LC biotin to an APJ agonist peptide (Intermediate c):

### Step 1: Synthesis of A-H-Q-R-P-C-L-S-C-K-G-P-(D-Nle)-Phenethyl amine Intermediate a

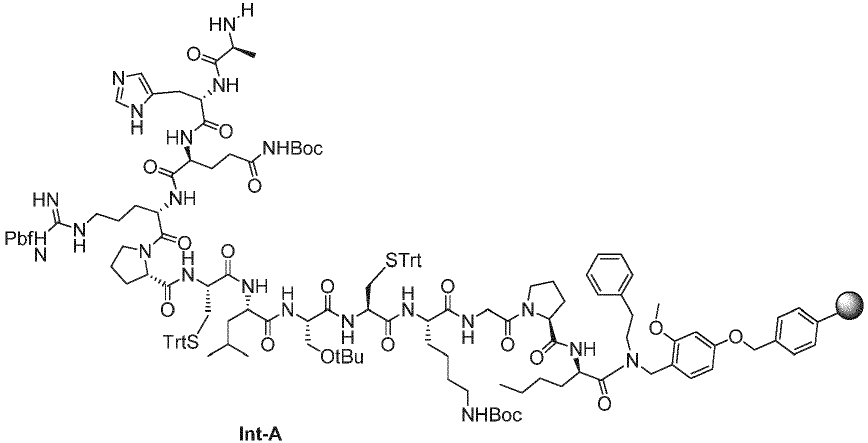

Phenethylamine-AMEBA resin (Sigma Aldrich, 0.1 mmol, 1.0 mmol/g) was subjected to solid phase peptide synthesis on an automatic peptide synthesizer (CEM Liberty Blue Microwave) with standard double Arg for the Arg residues and D-Nle coupled double time. Amino acids were prepared as 0.2 M solutions in DMF. A standard coupling cycle was defined as follows:
- Amino acid coupling: AA (5 eq.), HATU (5 eq.), DIEA (25 eq.)
- Washing: DMF (3 X 7 mL)
- Fmoc Deprotection: 20% Piperidine/0.1 M HOBt (2 x 7 mL)
- Washing: DMF (4 x 7 mL then 1 x 5 mL)

| Coupling | AA | Number of couplings x Reaction time (Temp) | Coupling Method |
|---|---|---|---|
| 1 | Fmoc-D-Nle-OH | 1 x 10 min (70°C) | DIEA/HATU |
| 2 | Fmoc-L-Pro-OH | 1 x 5 min (70°C) | DIEA/HATU |
| 3 | Fmoc-L-Gly-OH | 1 x 5 min (70°C) | DIEA/HATU |
| 4 | Fmoc-L-Lys-OH | 1 x 5 min (70°C) | DIEA/HATU |
| 5 | Fmoc-L-Cys-OH | 1 x 5 min (70°C) | DIEA/HATU |
| 6 | Fmoc-L-Ser-OH | 1 x 5 min (70°C) | DIEA/HATU |
| 7 | Fmoc-L-Leu-OH | 1 x 5 min (70°C) | DIEA/HATU |
| 8 | Fmoc-L-Cys-OH | 1 x 5 min (70°C) | DIEA/HATU |
| 9 | Fmoc-L-Pro-OH | 1 x 5 min (70°C) | DIEA/HATU |
| 10 | Fmoc-L-Arg-OH | 2 x 25 min (25°C) | DIEA/HATU |
| 11 | Fmoc-L-Gln-OH | 1 x 5 min (70°C) | DIEA/HATU |
| 12 | Fmoc-L-His-OH | 1 x 5 min (70°C) | DIEA/HATU |
| 13 | Fmoc-L-Ala-OH | 1 x 5 min (70°C) | DIEA/HATU |

After the assembly of the peptide, the resin was washed with DMF (2 x 50 mL) and DCM (2 x 50 mL) then dried under vacuum to give **Intermediate a** (276 mg, 0.1 mmol).

### Step 2: Preparation of Intermediate b (Cleavage of peptide from resin)

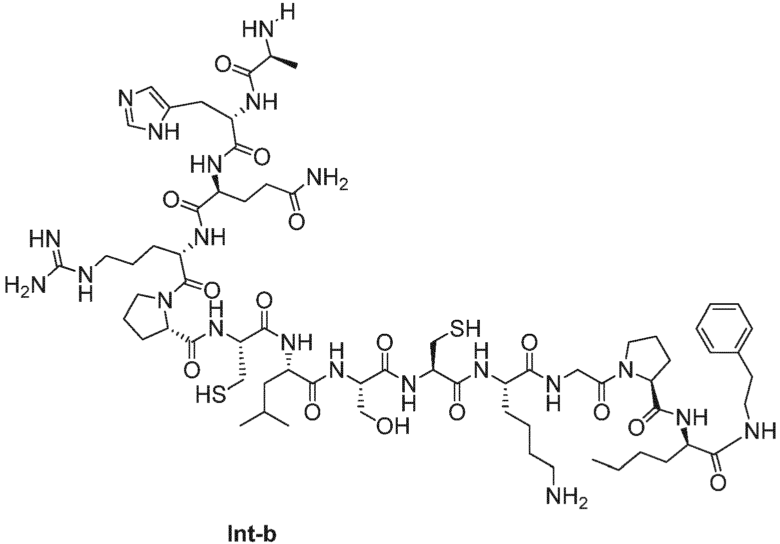

**Intermediate a** (276 mg, 0.1 mmol) was combined with 4 mL TFA solution (37 mL TFA, 1 mL H₂O, 1 mL TIPS, 3.06 g DTT) and shaken at r.t. for 3 hours. The solution was removed from the resin and precipitated into 40 mL cold Et₂O. The solution was vortexed and let stand over ice for 10 minutes before centrifuging at 4000 rpm for 5 minutes. The solvent was removed and the white solid was washed twice more with cold Et₂O (40 mL each time), centrifuged (5 minutes each time) and decanted. The solid was dried under vacuum overnight yielding **Intermediate b-batch 1** (17.4 mg, 0.012 mmol). LCMS (SQ2 ProductAnalysis-Acidic-Peptide-Polar, Acquity UPLC BEH C18 column, 130 Å, 1.7 µm, 2.1 mm x 50 mm, 50°C): Rt = 1.83 minutes, MS [M+H] 1513.5.

### Step 3: Preparation of Intermediate c (Cyclization of cysteine residues)

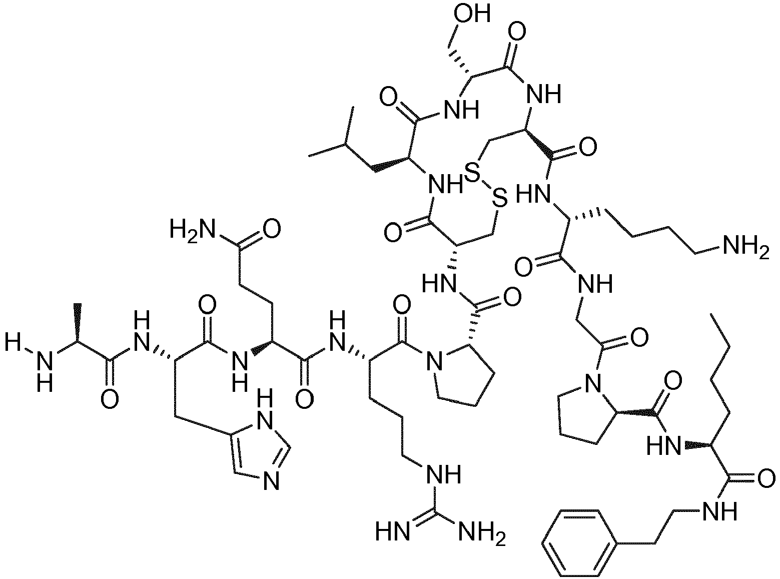

**Intermediate b-batch1 and** 2 (29.6 mg, 0.020 mmol) was dissolved in water (3 mL) and 10 drops of DMSO to give a slightly cloudy solution. Iodine (50 mM in HOAc, 0.783 mL, 0.039 mmol) was added slowly dropwise and the reaction was mixed at r.t. overnight. LCMS analysis of the crude reaction showed complete conversion of starting material. 0.5 M ascorbic acid was added dropwise until color dissipated. The material was purified via MS-triggered HPLC. Lyophilization of the pooled fractions gave 7 mg of the desired product as a white powder (4.63 µmol, 24%). LCMS (SQ2 ProductAnalysis-Acidic-Peptide, Acquity UPLC BEH C18 column, 130 Å, 1.7 µm, 2.1 mm x 50 mm, 50°C): Rₜ = 0.90 minutes, MS [M+H] 1511.8.

### Step 4: Preparation of Intermediate d (NHS/Peptide Reactivity)

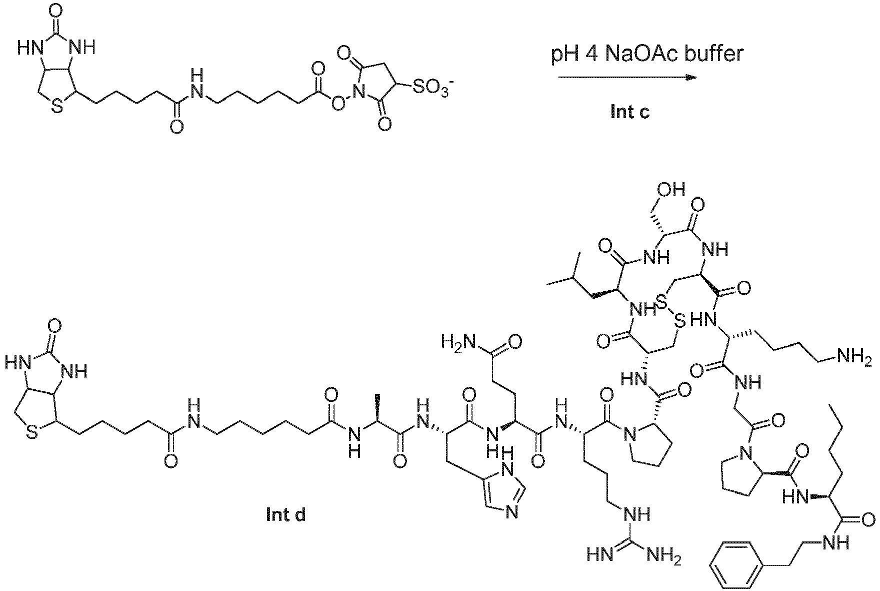

Sulfo-NHS-LC-Biotin (Sigma Aldrich, 0.737 mg, 1.324 µmol, 5 eq.) was dissolved in 30 mM pH 4 NaOAc buffer (266 µL) and added to **Intermediate c** (0.4 mg, 0.265 mmol). The reaction was mixed at r.t. for 16 hours. 33 µL of the reaction mixture was removed and quenched with hydrazine monohydrate (0.6 µL, 200 eq.). Complete conversion of starting material to +1 product was observed by LCMS. Labeling at N-terminal Ala residue was confirmed by digesting the reaction mixture with Trypsin Gold (Promega) according to manufacturer protocol and LCMS analysis. LCMS (SQ2 ProductAnalysis-Acidic-Peptide, Acquity UPLC BEH C18 column, 130 Å, 1.7 µm, 2.1 mm x 50 mm, 50°C): Rₜ = 2.29 minutes, MS [M+2/2] 925.8.

### Step 5: Digestion of Intermediate d with Trypsin Gold (N-terminal labeling)

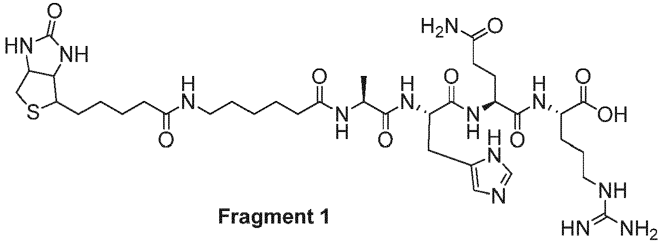

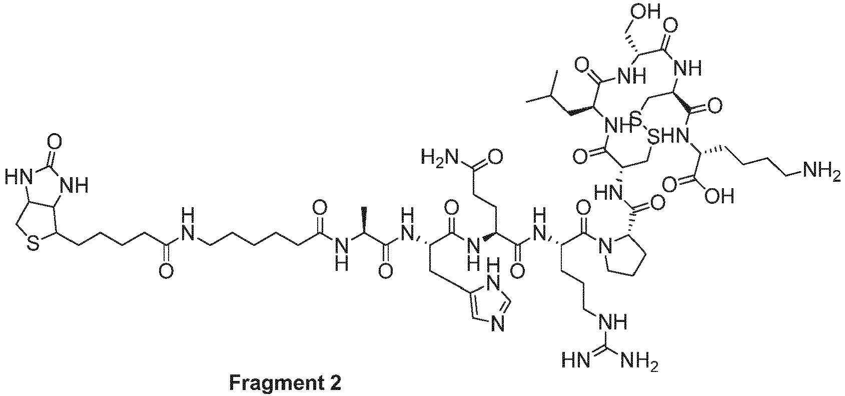

A solution of Trypsin Gold (Promega Part#TB309) was prepared in 50 mM acetic acid (1 µg/µL). The crude mixture of **Intermediate d** (100 µL, 0.081 µmol) was diluted with 50 mM Tris-HCI, 1 mM CaCl₂ pH 7.4 (200 µL) to give a 0.5 mg/mL solution. Trypsin Gold (3 µL) was added and the reaction mixed at 37°C for 16 hours. LCMS analysis indicated that cleavage had occurred at the C-terminal R and K positions of **Intermediate d** with one addition of biotin on each fragment. LCMS (SQ4 ProductAnalysis-20min-Acidic-XXPolar, Acquity CSH 1.7 µm 2.1x100 mm, 50°C): Fragment 1: AHQR +1 biotin Rₜ = 5.92 minutes, MS [M+2/2] calculated: 425.715, observed: 426.1; Fragment 2: AHQRPCLSCK +1 biotin Rₜ = 11.40 minutes, MS [M+2/2] calculated:740.345, observed: 740.9, MS [M+3/3] calculated: 493.896, observed: 494.4.

### Example of Therapeutic Protein (GDF15): Step a) of the method of the invention: modifying peptide so that the resulting peptide contains a histidine at the position adjacent to the N-terminus amino acid

### Expression of human GDF-15 proteins in E.coli cells

*E.coli* strains BL21 (DE3) Gold (Stratagene) and Rosetta (DE3) pLysS cells (Novagen) were transformed with constructs of Sequence ID No 1 to 7 and construct MAHA-(200-308)-hGDF15 respectively, cloned into pET26b vectors. Transformed cells were grown under antibiotic selection first in 3 ml and then in 50 ml Luria Broth (Bacto-Tryptone 10g/L, yeast extract 5g/L, NaCl 5/L, glucose 6g/L) until an OD600 of 1.5 was reached. The pre-cultures were used to inoculate two 1-L fermenters filled with Terrific Broth medium (NH4SO4 1.2 g/L, H2PO4 0.041 g/L, K2HPO4 0.052 g/L, Bacto-Tryptone 12 g/L, Yeast Extract 24 g/L). The cultures were induced by automatic addition of 1 mM isopropyl-beta-D-thiogalactopyranoside (IPTG) when pH increased above 7.1. Other fermentation parameters were: temp = 37°C; pH 7.0 +/- 0.2 adjusted by addition of 2N NaOH/H2SO4; pO2>30% with cascades of stirrer speed, air flow and oxygen addition. Five hours post induction the cultures were cooled to 10°C and cells were harvested by centrifugation.

### Purification and refolding of GDF15 variants

### a) Inclusion bodies

Recombinant E coli pellets expressing the protein of interest were resuspended (5% w/v) in 50 mM NaH₂PO₄ / 150 mM NaCl / 5 mM benzamidine.HCI / 5 mM DTT, pH 8.0 at 4°C, homogenized and lysed by 2 passages through a French press (800 and 80 bar). Inclusion bodies (IBs) were isolated by centrifugation at 12'000 rpm for 60 min at 4°C.

### b) Purification of crude unfolded protein

IBs were solubilized (5% w/v) in 6 M guanidine / 100 mM NaH₂PO₄ / 10 mM Tris / 20 mM beta-mercaptoethanol, pH 8.0 and stirred for 2 hours at room temperature. Debris was removed by centrifugation at 12'000 rpm. The solubilized IBs were further purified on Ni-NTA-Superflow (the construct without His tag binds as well to this resin due to the high histidine content). After base-line washing with 6 M guanidine / 100 mM NaH₂PO₄ / 10mM Tris / 5 mM beta-mercaptoethanol, pH 8.0, bound material was eluted with the same buffer adjusted to pH 4.5. The eluate was adjusted to pH 8.0, 100 mM DTT was added and the solution stirred over night at 4°C. The pH was then adjusted to 2 by addition of trifluoroacetic acid (TFA, 10% stock in water) and the solution further diluted 1:1 with 0.1% TFA in water. The crude protein solution was further purified by RP-HPLC (Poros) using a gradient of 0-50% acetonitrile in 50 min. The GDF-15 containing fractions were pooled and lyophilized.

### c) Protein folding

Method 1: Lyophilized material was dissolved at 2 mg/ml in 100 mM acetic acid, diluted 15-20 folds in folding buffer (100 mM CHES / 1 M NaCl / 30 mM CHAPS / 5 mM GSH / 0.5 mM GSSG / 20% DMSO, pH 9.5, 4°C) and the solution gently stirred during 3 days at 4°C. After 3 days 3 volumes of 100 mM acetic acid was added and the solution concentrated by ultrafiltration (5 kDa cut-off) to about 100-200 ml, diluted 10 fold with 100 mM acetic acid and re-concentrated. The refolded material was further purified by preparative RP-HPLC on a Vydac C4 column run at 50°C (buffer A: 0.1% TFA in water; buffer B: 0.05% TFA in acetonitrile). After loading the column was washed with 15% buffer B and eluted with a gradient of 15% B to 65% B in 50 min. Collected fractions containing the protein of interest were diluted with an equal volume of buffer A and lyophilized. Refolding yields were about 25% for both proteins.

Method 2: Protocol followed as in method 1 with folding buffer: 100 mM CHES, pH 9.4, 0.9 M arginine, 0.5 M NaCl, 1 mM EDTA, 2.5 mM GSH, 1 mM GSSG (final concentration).

The following GDF15 mutants were prepared according to above procedure:

### M-(His)₆-hGDF15 (reference example)

MHHHH HHAR NGDHC PLGPG RCCRL HTVRA SLEDL GWADW VLSPR EVQVT MCIGA
CPSQF RAANM HAQIK TSLHR LKPDT VPAPC CVPAS YNPMV LIQKT DTGVS LQTYD
DLLAK DCHCI (SEQ ID No 1).

LCMS: Calculated mass: 26462 Observed Mass: 26464

### MH-(199-308)-hGDF15 (reference example)

MHNGDHCPLGPGRCCRLHTVRASLEDLGWADWVLSPREVQVTMCIGACPSQFRAANMHAQI
KTSLHRLKPDTVPAPCCVPASYNPMVLIQKTDTGVSLQTYDDLLAKDCHCI (SEQ ID No 2)

LCMS: Calculated mass: 24636 Observed Mass: 24638

### M-(His)₆-M-hGDF15 (reference example)

MHHHHHHMARNGDHCPLGPGRCCRLHTVRASLEDLGWADWVLSPREVQVTMCIGACPSQF
RAANMHAQIKTSLHRLKPDTVPAPCCVPASYNPMVLIQKTDTGVSLQTYDDLLAKDCHCI (SEQ
ID No 3).

LCMS: Calculated mass: 26724 Observed Mass: 26725

### M-(His)₆-cynoGDF15 (reference example)

MHHHHHHARNGDRCPLGPGRCCRLHTVHASLEDLGWADWVLSPREVQVTMCIGACPSQFRE
ANMHAQIKMNLHRLKPDTVPAPCCVPASYNPMVLIQKTDTGVSLQTYDDLLAKDCHCV (SEQ
ID No 4).

LCMS: Calculated mass (dimer): 26664 Observed Mass: 26665

### His-dGDF15 (reference example):

MHHHHHHAHARDGCPLGEGRCCRLQSLRASLQDLGWANWVVAPRELDVRMCVGACPSQFR
SANTHAQMQARLHGLNPDAAPAPCCVPASYEPVVLMHQDSDGRVSLTPFDDLVAKDCHCV
(SEQ ID NO 5).

LCMS: Calculated mass (dimer): 26368 Observed Mass: 26363

### AH-(199-308)-hGDF15

AHNGDHCPLGPGRCCRLHTVRASLEDLGWADWVLSPREVQVTMCIGACPSQFRAANMHAQI
KTSLHRLKPDTVPAPCCVPASYNPMVLIQKTDTGVSLQTYDDLLAKDCHCI (SEQ ID No 5)

### Step 1: preparation of construct M-His₆-TEV(ENLYFQ/A)-H-hsGDF15 aa199-308

The construct M-His6-TEV(ENLYFQ/A)-H-hsGDF15 aa199-308 was prepared according to the above procedure (steps a, b and c).

### Step 2: TEV cleavage of the protein from step 1

The lyophilized protein was solubilized in water to a final concentration of 1.75 mg/ml. The protein was unfolded again by diluting 1:1 in 6M Guan/50mM Tris, pH 8,0 + 50mM DTT, and stirred at RT for 1h. The material was re-purified by preparative RP-HPLC on a Vydac C4 column and lyophilized. 26mg of lyophilisate were solubilized in 26ml 50mM Tris/3M UREA, pH 7,5 + 3000 Units AcTEV Protease (Invitrogen, 12575-023) and incubated for 4 days. The pH was then adjusted to pH 2.0 by addition of trifluoroacetic acid (TFA, 10% stock in water) and the solution further diluted to 150 ml with 0.1 % TFA in water. After filtration through a 0.22um membrane the material was again purified by preparative RP-HPLC on a Vydac C4 column to isolate successfully cleaved protein. Fractions were collected manually; target protein-containing fractions were isolated and lyophilized. The cleaved GDF15 protein was then refolded and refolded protein purified as described above.
LCMS: Calculated mass (dimer): 24516 Observed Mass:24518

The following GDF15 mutants is prepared according to the above procedure:

### MHA-(200-308)-hGDF15 (reference example)

MHAGDHCPLGPGRCCRLHTVRASLEDLGWADWVLSPREVQVTMCIGACPSQFRAANMHAQI
KTSLHRLKPDTVPAPCCVPASYNPMVLIQKTDTGVSLQTYDDLLAKDCHCI (SEQ ID No. 7)

LCMS: Calculated mass (dimer): 24752

The following GDF15 mutants was prepared according to the above procedure:

### AHA-(200-308)-hGDF15

AHAGDHCPLGPGRCCRLHTVRASLEDLGWADWVLSPREVQVTMCIGACPSQFRAANMHAQI
KTSLHRLKPDTVPAPCCVPASYNPMVLIQKTDTGVSLQTYDDLLAKDCHCI (SEQ ID No. 8)

LCMS: Calculated mass(dimer): 24430; Observed mass (dimer): 24432

### Site specific conjugation of MH-(199-308)GDF15 (reference example) with a fatty acid acylating agent

### Step 1: preparation of the fatty acid-linker acylating agent:

### Step 1a: 1-Benzyl 3-tert-butyl 2-undecylmalonate

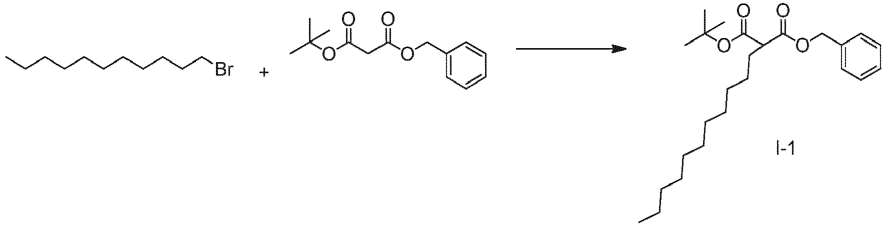

To a suspension of NaH (160mg, 4.0mmol) in DMF (8mL) at 0°C under N₂, was added benzyl tert-butyl malonate (1.0g, 4.0mmol) in DMF (2mL). The mixture was stirred for 50min after which 1-bromoundecane in DMF (2mL) was added. After an additional hour of stirring the reaction was allowed to warm to room temperature. The reaction was maintained overnight. Et₂O (100mL) and water (20mL) were added to partition the reaction. The aqueous phase was extracted with Et₂O (100mL), and the combined organics dried over Na₂SO₄. The solvent was evaporated and the residue purified by flash column (C18 12g, 40-100% ACN / water +0.1% TFA) to yield the title compound as a colorless oil (1.14g, 2.82mmol, 71%): LCMS Method A Rt = 1.58min, M+Na 427.4; ¹H NMR (400 MHz, CHLOROFORM-d) δ ppm 0.84 - 0.96 (m, 3 H) 1.28 (br. s, 12 H) 1.31 (m, J=3.90 Hz, 6 H) 1.41 (s, 9 H) 1.88 (q, J=7.38 Hz, 2 H) 3.29 (t, J=7.58 Hz, 1 H) 5.19 (q, J=12.27 Hz, 2 H) 7.30 - 7.42 (m, 5 H).

### Step 1b: 1,11-Dibenzyl 11-tert-butyl docosane-1,11,11-tricarboxylate

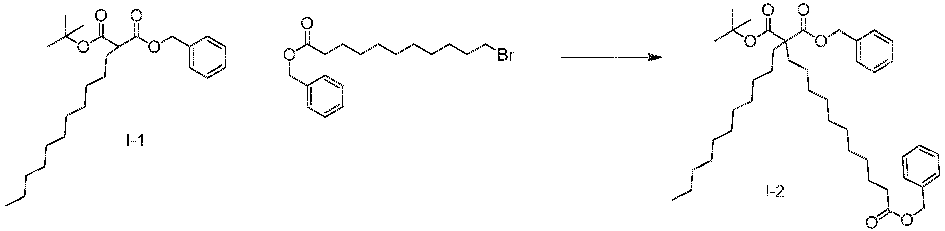

A solution of **I-1** (650mg, 1.61mmol) in DMF (1mL) was slowly added to a suspension of NaH (70.7mg, 1.77mmoL) in DMF (6mL) at 0°C under N₂. The mixture was stirred for 40min before the addition of benzyl 11-bromoundecanoate (571mg, 1.61mmol) in DMF (1mL). Upon addition the reaction was allowed to warm to room temperature and stirred overnight. The reaction was diluted with Et₂O (100mL) and extracted with water (20mL). The aqueous phase was extracted with Et₂O (100mL), and the combined organics dried over Na₂SO₄. The solvent was evaporated. The residue was purified by flash column (silica 80g, 0-10% EtOAc / HEP) to yield **I-2** as a colorless oil (823mg, 1.21mmol, 75%): ¹H NMR (400 MHz, CHLOROFORM-d) δ ppm 0.84 - 0.94 (m, 3 H) 1.12 (m, J=6.60 Hz, 4 H) 1.19 - 1.33 (m, 28 H) 1.35 (s, 9 H) 1.66 (quin, J=7.40 Hz, 2 H) 1.85 (t, J=8.44 Hz, 4 H) 2.37 (t, J=7.52 Hz, 2 H) 5.14 (s, 2 H) 5.16 (s, 2 H) 7.30 - 7.42 (m, 10 H).

### Step 1c: 13-(Benzyloxy)-2-((benzyloxy)carbonyl)-13-oxo-2-undecyltridecanoic acid

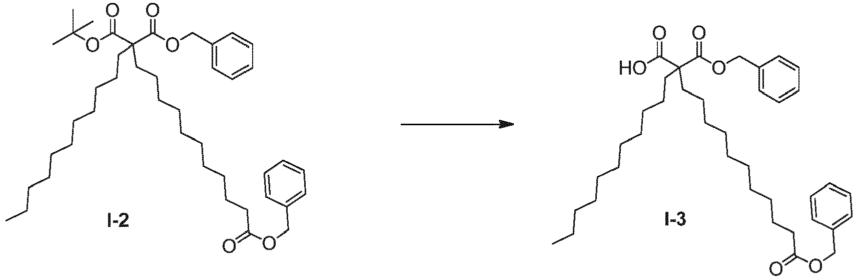

To a solution of intermediate **I-2** (200mg, 0.295mmol) in DCM (3mL) was added TFA (0.6mL), and the reaction stirred at room temperature for 3hrs. The solvent was evaporated and the residue purified by flash column (silica 12g, 0-15% EtOAc / HEP) to yield the title compound (177mg, 0.284mmol, 96%): ¹H NMR (400 MHz, CHLOROFORM-d) δ ppm 0.87 - 0.94 (m, 3 H) 0.94 - 1.05 (m, 2 H) 1.19 (br. s., 14 H) 1.23 - 1.37 (m, 16 H) 1.65 (quin, *J*=7.40 Hz, 2 H) 1.78 - 1.91 (m, 2 H) 1.93 - 2.05 (m, 2 H) 2.37 (t, J=7.52 Hz, 2 H) 5.14 (s, 2 H) 5.27 (s, 2 H) 7.31 - 7.44 (m, 10 H).

### Step 1d: 1,11-Dibenzyl 11-(2,5-dioxocyclopentyl) docosane-1,11,11-tricarboxylate

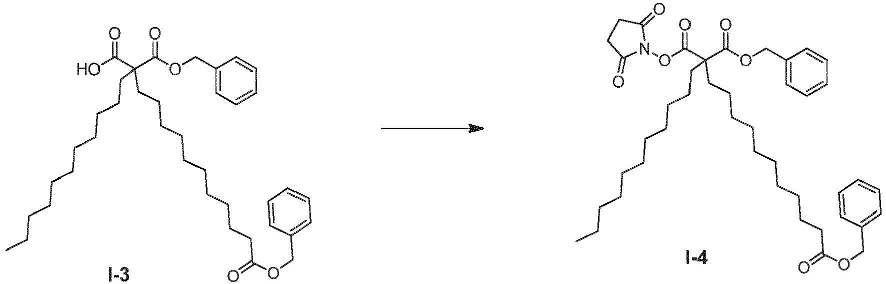

A solution of DCC (58.6mg, 0.284mmol) in DCM (1mL) was added to a solution of **I-3** (177mg, 0.284mmol) and N-hydroxysuccinimide (32.7mg, 0.284mmol) in DCM (2mL) and THF (0.3mL) under N₂. The reaction was stirred at room temperature for 4hr. The solvent was evaporated and the residue purified by flash column (silica 12g, 0-35% EtOAc / HEP) to yield **I-4** as a colorless oil (153mg, 0.213mmol, 75%): ¹H NMR (400 MHz, CHLOROFORM-d) δ ppm 0.86 - 0.93 (m, 3 H) 1.12 - 1.21 (m, 2 H) 1.21 - 1.37 (m, 30 H) 1.66 (quin, *J*=7.40 Hz, 2 H) 1.89 - 2.07 (m, 4 H) 2.37 (t, *J*=7.58 Hz, 2 H) 2.84 (br. s., 4 H) 5.13 (s, 2 H) 5.25 (s, 2 H) 7.30 - 7.47 (m, 10 H).

### Step 1e: Fatty acid-linker construct (Intermediate 5)

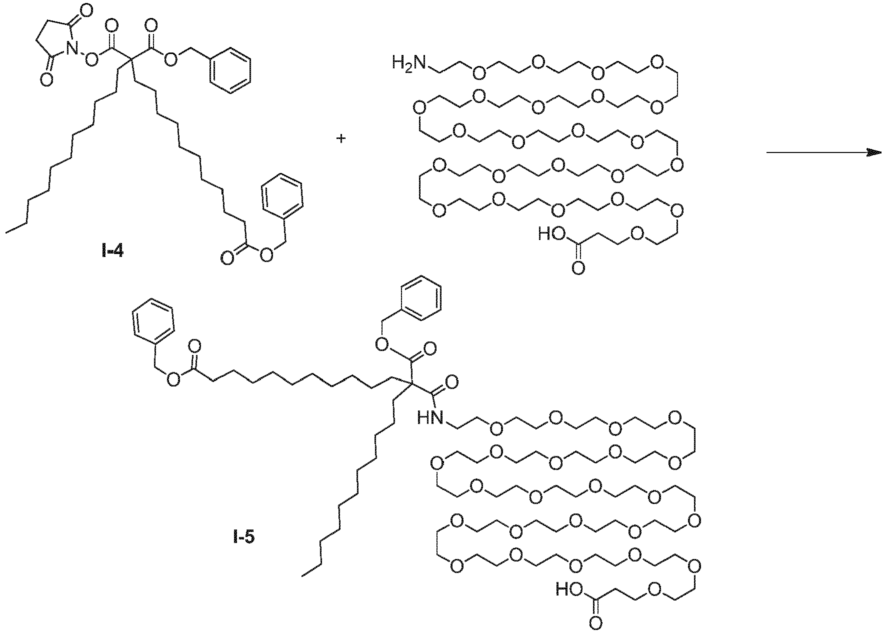

A solution of intermediate **I-4** (145mg, 0.201 mmol) in THF (1.5mL) and DCM (1.5mL) was added to a vial charged with amino-PEG24-acid. DIPEA (88µL, 0.504mmol) was added and the reaction agitated on a shaker plate for 15hrs. The solvent was evaporated and the residue purified by supercritical fluid chromatography (Waters HILIC 20x150mm; 15-25% MeOH / CO2) to yield intermediate **I-5** (151 mg, 0.086mmol, 43%): LCMS Method C Rt = 1.30min, [M+2H]+2 876.4; ¹H NMR (400 MHz, CHLOROFORM-d) δ ppm 0.86 - 0.93 (m, 3 H) 0.93 - 1.04 (m, 2 H) 1.19 (br. s., 15 H) 1.23 - 1.37 (m, 15 H) 1.61 - 1.68 (m, 2 H) 1.78 (td, *J*=12.44, 4.34 Hz, 2 H) 1.92 - 2.05 (m, 2 H) 2.37 (t, *J*=7.58 Hz, 2 H) 2.62 (t, *J*=6.05 Hz, 2 H) 3.49 (dd, *J*=6.72, 2.32 Hz, 2 H) 3.52 - 3.59 (m, 2 H) 3.59 - 3.73 (m, 92 H) 3.80 (t, *J*=6.05 Hz, 2 H) 5.13 (s, 2 H) 5.18 (s, 2 H) 7.31 - 7.42 (m, 10 H) 8.09 (t, *J*=5.26 Hz, 1 H).

### Step 1f: Intermediate 6

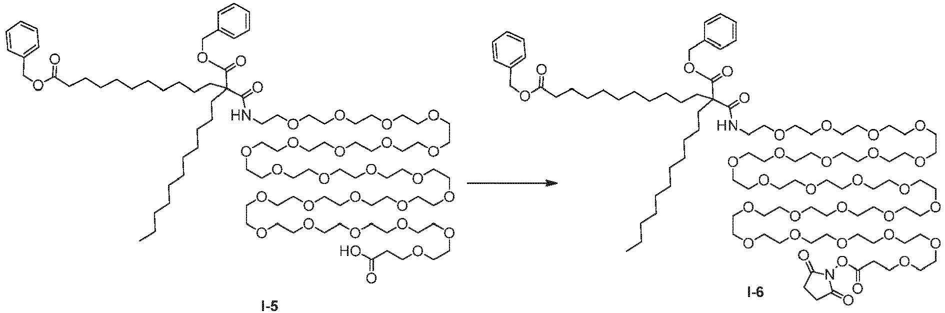

DCC (22mg, 0.103mmol) in DCM (0.265mL) was added to a solution of intermediate **I-5** (150mg, 0.086mmol) and N-hydroxysuccinimide in DCM (1.5mL). The reaction was stirred for 1.5hrs. Additional N-hydroxysuccinimide (10mg) in THF (0.5mL) and DCC (22mg) in DCM (0.265mL) was added and the reaction stirred overnight. The solvent was evaporated and the residue purified by flash column (silica 12, 0-5% MeOH / DCM) to yield intermediate **I-6** (159mg, quantitative) as a white solid: LCMS Method A Rt = 1.55min, [M+H₃O+H]⁺² 933.9 .

### Step 1g: fatty acid-linker acylating agent (Intermediate 7)

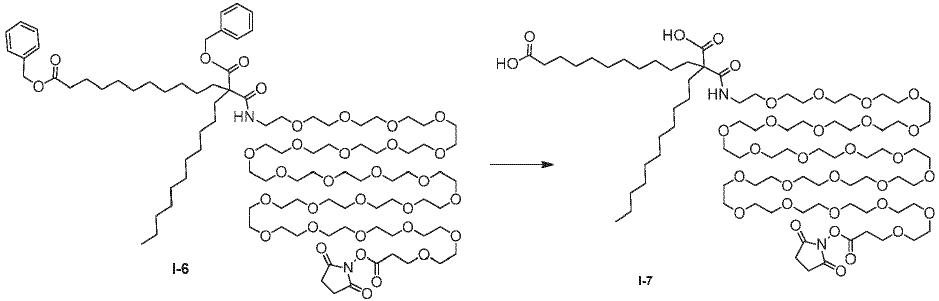

To a solution of intermediate **I-6** (159mg, 0.086mmol) in THF (5mL) was added a suspension of 10% Pd on carbon (4.6mg, 4.3µmol) in THF (1mL). The reaction was placed under hydrogen and stirred for 40min. More Pd on carbon (7mg, 6.5µmol) was added and then stirred another 1hr under hydrogen. The reaction was passed through a membrane filter and the filtrate evaporated. The residue was purified by HPLC (Sunfire C18 30x50mm, 45-70% ACN / water + 0.1% TFA) to yield the title fatty acid-linker acylating agent (83mg, 0.047mmol, 54%): LCMS Method A Rt = 1.03min, [M+2H]+2 835.2; ¹H NMR (400 MHz, CHLOROFORM-*d*) δ ppm 0.84 - 0.94 (m, 3 H) 1.17 (br. s., 2 H) 1.21 - 1.39 (m, 30 H) 1.57- 1.68 (m, 2 H) 1.69- 1.80 (m, 2 H) 1.97 - 2.10 (m, 2 H) 2.34 (t, J=7.21 Hz, 2 H) 2.86 (s, 4 H) 2.92 (t, J=6.48 Hz, 2 H) 3.51 - 3.73 (m, 96 H) 3.87 (t, J=6.48 Hz, 2 H) 7.45 (t, J=4.46 Hz, 1 H)

### Example 5: Step 2: Selective conjugation of MH-(199-308)-hGDF15 with fatty acid-linker acylation agent

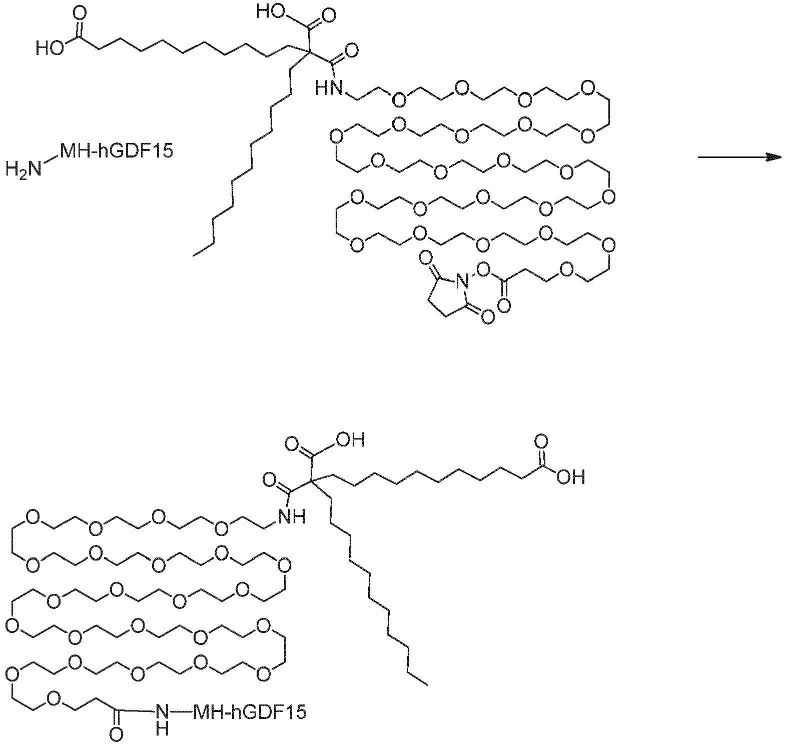

MH-(199-308)-hGDF15 (0.393 mL, 0.028 µmol, 1.78 mg/mL) was added to 1.5 ml of 30mM sodium acetate buffer pH 4 NHS fatty acid (**I-7:** 474 ug, 0.284umol, 10mg/ml) was added to solution. After 5 hours, reaction was complete according to MALDI. Products were purified by washing 5 times using amicon ultrafiltration 10kD to give 575 ug of conjugate in 73% yield. MALDI: sm (18%), expected mass: 24638 observed mass: 24735; +1 fatty acid (38%) expected mass: 26192 observed mass: 26268 ; +2 fatty acid (40%) expected mass: 27746 observed mass: 27798 ; +3 fatty acid (4%) expected mass: 29300 observed mass: 29333.

### Example 6: Step 2: Site specific conjugation of M-(His)6-hGDF15-(199-308) with fatty acid-linker acylation agent

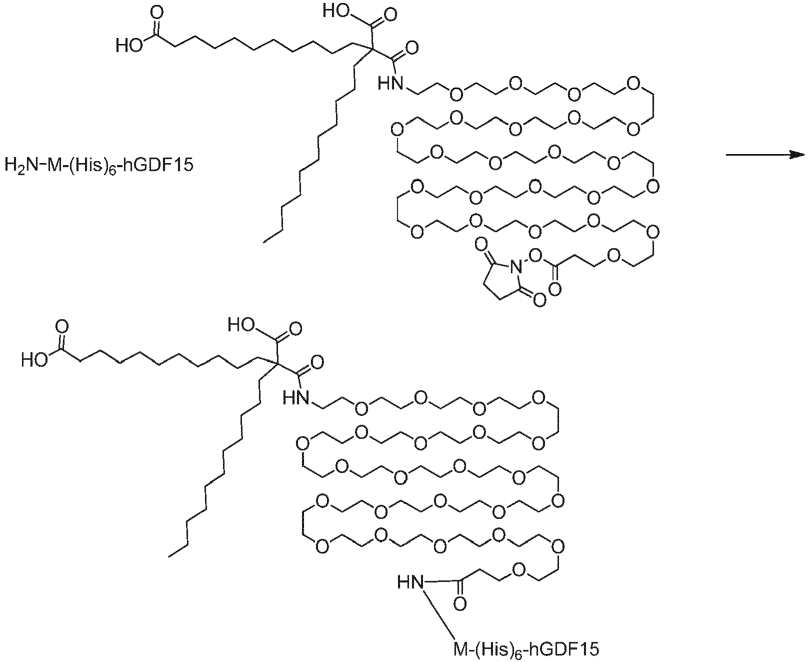

M-(His)₆-hGDF15 (0.493 ml, 0.026 µmol, 1.42mg/ml) was added to 1.5 ml of 30mM sodium acetate buffer NHS fatty acid (**I-7**; 0.221 mg, 0.132 umol, 10mg/mL) was added to the solution. Overnight the reaction was not complete so 2.5 more equivalents of fatty acid NHS (0.110 mg, 0.066 umol, 10mg/mL) were added and after 5 hrs Maldi showed +2 conjugate as major product. Product was purified by washing 5 times using amicon ultrafiltration 10kD to give 565 ug of conjugate in 76% yield. MALDI: sm (18%), expected mass: 26468 observed mass: 26553; +1 fatty acid (38%) expected mass: 28022 observed mass: 28099 ; +2 fatty acid (40%) expected mass: 29576 observed mass: 29649 ; +3 fatty acid (4%) expected mass: 31130 observed mass: 31201.

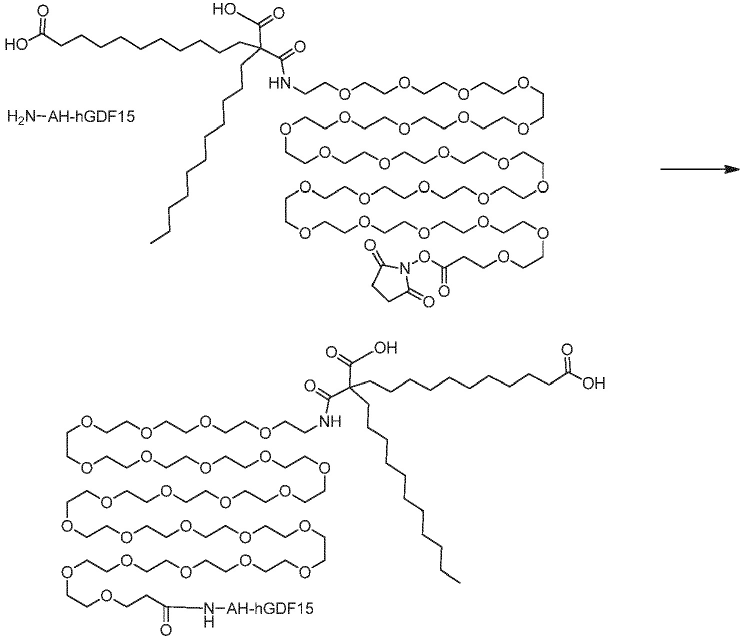

### AH-GDF15 + I-7:

| Degree of Labelling | Calculated | Observed | % |
|---|---|---|---|
| AH-GDF15 (dimer) | 24518 | 24559 | 3 |
| AH-GDF15 +1 FA | 26072 | 26091 | 44 |
| AH-GDF15 +2 FA | 27626 | 27615 | 42 |
| AH-GDF15 +3 FA | 29180 | 29164 | 11 |

| | | | |
|---|---|---|---|
| +1FA and +2FA refer to addition of a fatty acid at the N-terminus of the monomeric unit and dimeric unit respectively. +3FA refer to non-selective acylation. | | | |

Sequence (monomer):
ahngdhcplgpgrccrlhtvrasledlgwadwvlsprevqvtmcigacpsqfraanmhaqiktslhrlkpdtvpapccvpasynpm vliqktdtgvslqtyddllakdchci

A 10 mg/mL solution of fatty acid **I-7** was prepared in H₂O. AH-hGDF15 (1.12 mg/mL, 175 µL, 0.0080 µmol) was combined with 202 µL 30 mM NaOAc buffer pH 4.0 and aliquot of **I-7** (13.33 µL) was added. The reaction was mixed at r.t. for 16 hours at which point MALDI analysis showed 30% conversion of starting material. An additional 6.67 µL aliquot of **I-7** was added and the reaction mixed for 16 hours and 13.33 µL aliquot of **I-7** was added. After 16 hours of mixing at r.t. **I-7** (13.33 µL) was added and the reaction mixed at r.t. for 3 days. A 13.33 µL aliquot of **I-7** was added and the reaction mixed at r.t. for 16 hours at which point MALDI analysis indicated >95% conversion. The solution was exchanged into 30 mM NaOAc buffer pH 4.0 using 10 kDa MWCO Amicon centrifugal filter by diluting and concentrating the reaction 5 times to a volume of 0.2 mL. The concentration was measured by A₂₈₀ (30040 M-1cm-1; 26072 g/mol) to be 0.31 mg/mL , 30%.

### Example 6A: Site specific conjugation of AHA-hGDF15-(200-308) with fatty acid-linker acylation agent

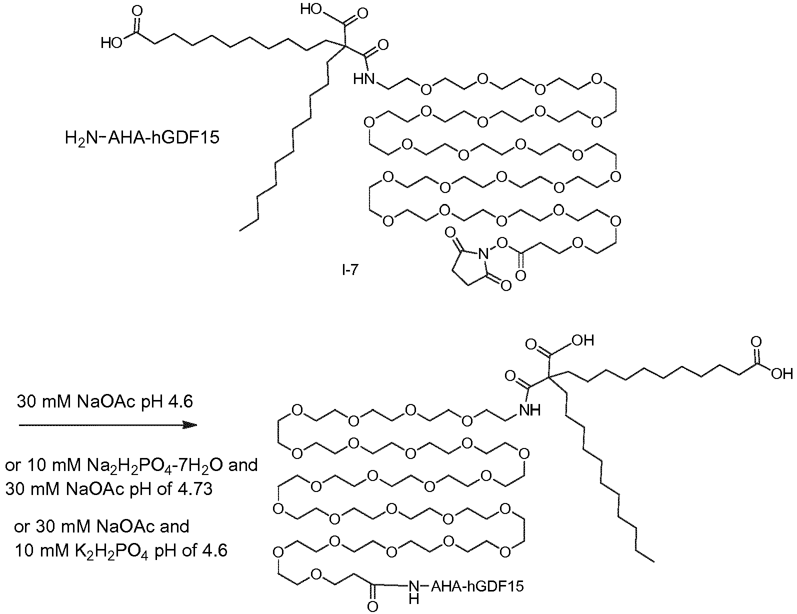

A 10 mg/mL solution of fatty acid (Intermediate 7) in molecular biology grade water was prepared. To AHA-hGDF15 (6.67 mg/mL in 30 mM NaOAc pH 4.0, 5.247 mL, 1.433 µmol) was added 30 mM NaOAc pH 4.6 (acceptable range 4.5-5.0) to give a final protein concentration of 0.88 mg/mL. Intermediate 7 (10eq., 2.39 mL, 0.014 mmol) was added and the reaction was mixed at r.t. for 18 hours. Precipitate had formed in the reaction vial. The reaction mixture was split amongst 4x15 mL 10kDa Amicon centrifugal filters and each was diluted to 15 mL with 30 mM NaOAc pH 4.0. The material was buffer exchanged 4x into 30 mM NaOAc pH 4.0 and samples were combined to a volume of 25.6 mL, agitating the precipitate in the filter with a pipette tip in between washes. Precipitate remained in solution so the mixture was let sit at 4 °C overnight. Concentration was measured by A280 (30040 cm⁻¹M⁻¹, 27538 g/mol) to be 1.62 mg/mL (100%). UPLC analysis showed 60% recovery of +1 and +2 products (Method F).

| **Species** | **% observed** |
|---|---|
| AHA-GDF15 | 29 |
| AHA-GDF15 +1 FA | 27 |
| AHA-GDF15 +2 FA | 33 |
| AHA-GDF15 +3 FA | 11 |

### Purification:

The crude product was purified by reverse phase chromatography (Buffer A 0.1% TFA in water; Buffer B 0.1M TFA in ACN gradient; 99%-80% Buffer A) on a Waters BEH300 130Å, 3.5 µm, 4.6mmX100mm flow rate 2.5ml/min.
Fraction 1: Unreacted AHA-hGDF15: Rt=17.33 min
Fraction 2: AHA-GDF15 +1FA: Rt=20.20 min (approximately 15% yield)
Fraction 3: AHA-GDF15 + 2FA: Rt=21.60 min (approximately 15% yiled)
Fraction 4: AHA-GDF15 + 3 FA: Rt=23.0 min (approximately 5% yield)

Alternatively the reaction may be carried out in 10 mM Na₂HPO₄-7H₂O and 30 mM NaOAc at a pH of 4.73: A 10 mg/mL solution of Intermediate 7 in molecular biology grade water was prepared. To AHA-hGDF15 (12.04 mg/mL in 30 mM NaOAc pH 4.0, 4.15 µL, 0.002 µmol) was added 30 mM NaOAc 10 mM Na₂HPO₄ - 7H₂O pH 4.73 to give a final protein concentration of 0.88 mg/mL. Intermediate 7 (20eq., 6.83 µL, 0.041 µmol) was added and the reaction was mixed at r.t. for 18 hours. The reaction mixture had turned cloudy with precipitate. UPLC analysis showed 58% +1 and +2 products (Method F).

| **Species** | **% observed** |
|---|---|
| AHA-GDF15 | 0 |
| AHA-GDF15 +1 FA | 11 |
| AHA-GDF15 +2 FA | 47 |
| AHA-GDF15 +3 FA | 34 |
| AHA-GDF15 +4 FA | 7 |

The reaction may also be carried out in 30 mM NaOAc and 10 mM K₂HPO₄ at a pH of 4.6: A 10 mg/mL solution of intermediate 7 in molecular biology grade water was prepared. To AHA-hGDF15 (6.21 mg/mL in 30 mM NaOAc pH 4.0, 5.261 mL, 1.337 µmol) was added 30 mM NaOAc 10 mM K₂HPO₄ pH 4.6 (acceptable range 4.5-5.0) to give a final protein concentration of 0.88 mg/mL. Intermediate 7 (10eq., 68.3 µL, 0.409 µmol) was added and the reaction was mixed at r.t. for 7 hours. The reaction mixture had turned cloudy with precipitate. The reaction mixture was split into four 9 mL portions in 15 mL 10kDa Amicon centrifugal filter and diluted to 15 mL with 30 mM NaOAc pH 4.0. The material was buffer exchanged 4x into 30 mM NaOAc pH 4.0, agitating the precipitate between each wash with a pipette tip. The reaction mixture was concentrated to a volume of 75 mL. Precipitate remained so the material was stored at 4 °C for two days. Concentration was measured by A280 (30040 cm⁻¹M⁻¹, 27538 g/mol) to be 0.4 mg/mL (97%). UPLC analysis showed 61% recovery of +1 and +2 products (Method F).

| **Species** | **% observed** |
|---|---|
| AHA-GDF15 | 34 |
| AHA-GDF15 +1 FA | 34 |
| AHA-GDF15 +2 FA | 27 |
| AHA-GDF15 +3 FA | 5 |

### Example 7: Biotinylation of M(His)₆M-GDF15 (reference example) with mapping to confirm the N-terminal labeling

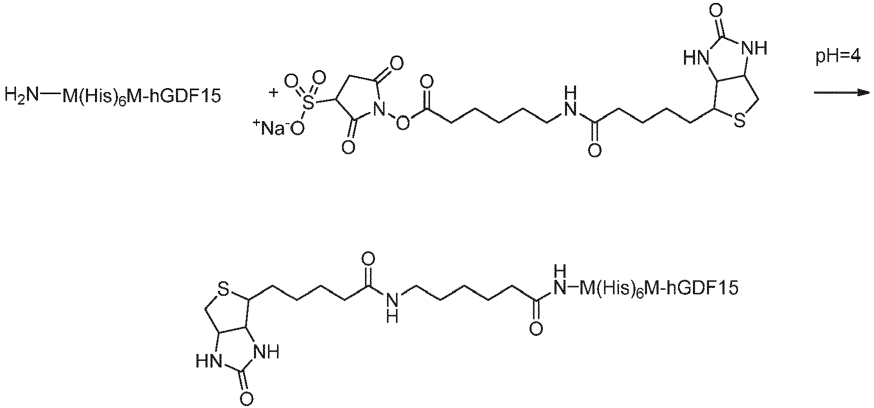

To a solution of MHHHHHHM-hGDF15 (0.53 mg/mL, 8 mL) in 30 mM NaOAc pH 4 sodium acetate buffer was added a solution of NHS ester (1.5 mg/mL in pH 4 30 mM NaOAc buffer) in the following amount sequentially in every two hours, 118 uL,177 uL. The mixture was concentrate/washed using amicon concentrator (3 times) with pH 4 NaOAc buffer, and the cartridge was repeatedly washed to achieve high recovery of protein. 5.8 mL 0.64 mg/mL product was obtained. LCMS: Expected (+1): 27066, Observed (+1): 27066 ; Expected (+2): 27406, Observed (+2):27405.

### Biotinylation confirmed 90% at N-terminus using Glu-C digestion and mapping.

The objective of this protocol is to reduce, alkylate and digest proteins with Glu-C (S. aureus V8) for subsequent analysis by either HPLC-MS/MS (high performance liquid chromatography - tandem mass spectrometry) or MALDI MS (matrix assisted laser desorption ionization mass spectrometry). Briefly, pure proteins are reconstituted in chaotrope, reduced with dithiothreitol, alkylated with iodoacetamide and then digested with Glu-C. Glu-C has specificity for glutamate residues when buffered in ammonium bicarbonate or ammonium acetate buffers. Glu-C will cleave either aspartate or glutamate in the presence of phosphate buffer.

### 1. Experimental Method

### 1.1. Reconstitution/denaturation (4 M GnHCl in 25 uL)

1.1.1. Add 25 uL 4 M guanidine hydrochloride (GnHCl) to a dry protein aliquot.
1.1.2. Gently agitate, shake or tap for approximately 2 minutes to reconstitute and denature the protein.

### 1.2. Reduction (2 M GnHCl, 100 mM ABC, 10 mM DTT in 50 uL)

1.2.1. Add 20 uL freshly prepared 250 mM ammonium bicarbonate buffer (ABC).
1.2.2. Add 5 uL freshly prepared 100 mM dithiothreitol (DTT) in water.
1.2.3. Incubate for 1 hour at 37°C with gentle shaking.

### 1.3. Alkylation (1.6 M GnHCl, 83.3 mM ABC, 8.3 mM DTT, 35 mM IAA in 60 uL)

1.3.1. Add 4.75 uL water.
1.3.2. Add 5.25 uL freshly prepared 400 mM iodoacetamide (IAA) in water.
1.3.3. Briefly and gently agitate or shake to mix the sample.
1.3.4. Incubate for 40 minutes at room temperature in the dark.

### 1.4. Cleanup

1.4.1. Add at least 1000 uL ice-cold ethanol.
1.4.2. Gently agitate or shake to mix.
1.4.3. Incubate at -20°C for 2 hours to precipitate the protein.
1.4.4. Centrifuge for 10 minutes at 10,000 RPM at 4°C.
1.4.5. Remove the majority of the supernatant.
1.4.6. Dry to completion in a Speedvac.

### 1.5. Glu-C digestion (0.25 M GnHCl, 25 mM Am Ac ∼pH 6.5, 20:1 protein:Glu-C in 100 uL)

1.5.1. Reconstitute the dried protein in 10 uL 2.5 M GnHCl.
1.5.2. Gently agitate, shake or tap for approximately 1 minute to reconstitute the protein.
1.5.3. Add 10 uL freshly prepared 250 mM ammonium acetate buffer.
1.5.4. Reconstitute 10 ug Glu-C in 100 uL milliQ water.
1.5.5. Add an appropriate quantity of 0.1 ug/uL Glu-C to achieve a ratio of (1 ug Glu-C : 20 ug protein). For instance, add 10 uL for 20 ug of protein.
1.5.6. Add water to achieve a final reaction volume of 100 uL.
1.5.7. Incubate for 4 hours at 37°C with gentle shaking.
1.5.8. Glu-C has enzymatic activity from pH 4.0 - 9.0 so it is not possible to quench the activity with addition of acid or base. It is recommended to analyze immediately.

The biotinylated GDF15 sample should yield a 339 da mass shifted GluC peptide.

N-terminal GluC digestion product of chemically biotinylated GDF15. Sequence: LC-biotin-M(His)₆MARNGDHCPLGPGRCCRLHTVRASLE. Calculated monoisotopic mass with 3 cysteine alkylations = 4311.9755 Da M 3+ charge +1 calc.=1438.3325 Observed mass=1438.3331.

### Example 8: Site specific Biotinylation of M(His)₆-hGDF15 (reference example)

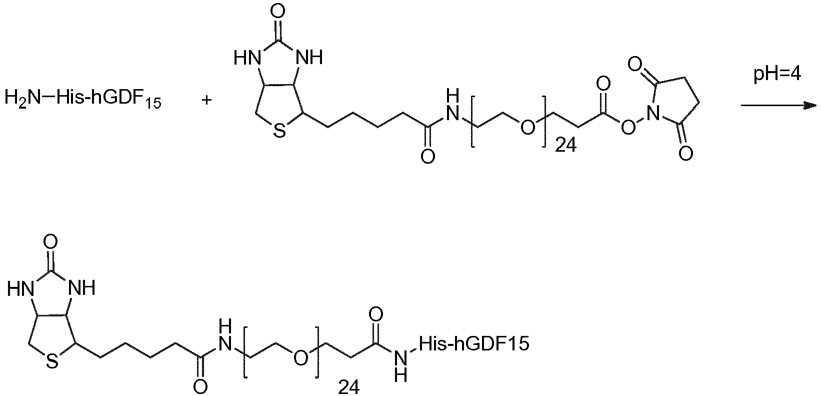

### His-hGDF15 + Biotin-PEG₂₄-NHS ester:

| Degree of Labelling | Calculated | Observed | % | Rₜ (min) |
|---|---|---|---|---|
| His-hGDF15 (dimer) | 26468 | 26464 | 18 | 2.35 |
| His-hGDF15 +1 biotin | 27823 | 27818 | 61 | 2.48 |
| His-hGDF15 +2 biotin | 29179 | 29174 | 21 | 2.75 |

| | | | | |
|---|---|---|---|---|
| +1 biotin and + biotin refer to the acylation of the N-terminus of the monomeric and dimeric moieties respectively. | | | | |

A 10 mg/mL solution of biotin-PEG₂₄-NHS (Biomatrik, Inc.) in 30 mM pH4 NaOAc was prepared. MHHHHHH-hGDF15 (1.007 mL, 0.054 µmol) was combined with 30 mM NaOAc pH 4 (1.5 mL) and biotin-PEG₂₄-NHS (Biomatrik, Inc., 397 µL, 2.70 µmol). The mixture was shaken at r.t. for 16 hours at which point an additional 10 equivalents (79.4 µL) of NHS solution was added and the reaction was mixed at r.t. for 16 hours. An additional 10 equivalents (79.4 µL) of NHS solution was added and the reaction mixed at r.t. for 16 hours. The reaction was heated to 37°C and stirred for 10 hours at which point the mixture was exchanged into 30 mM NaOAc pH 4 using 10 kDa MWCO Amicon centrifugal filter by diluting and concentrating the sample 5 times to a volume of 2 mL. LCMS analysis indicated the presence of starting material, +1 and +2 products. The concentration was measured by A₂₈₀ (29090 M⁻¹cm⁻¹, 27818 g/mol) to be 1.20 mg/mL (100%). LCMS **Method D:** Rₜ = 2.48 min; MS [M+1+1 biotin], [M+1+2 biotin]: observed: 27818.0000, 29174.0000, calculated: 27823.7, 29179.4.

### Example 8A: Site specific biotinylation of M(His)6-hGDF15 (reference example)

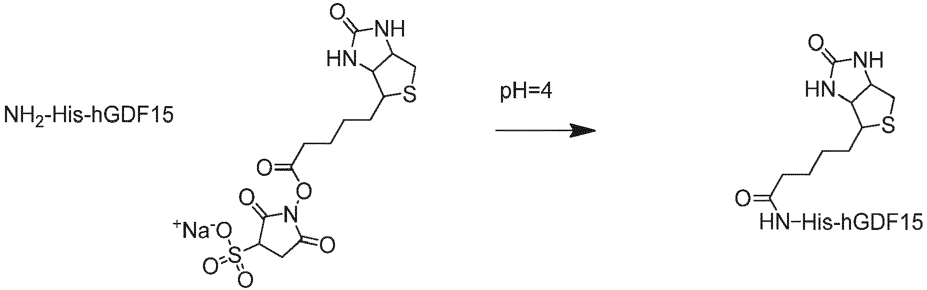

### His-hGDF15 + Sulfo-NHS-Biotin:

| Degree of Labelling | Calculated | Observed | **%** | Rₜ(min) |
|---|---|---|---|---|
| His-hGDF15 B8 | 26468 | n/a | 0 | n/a |
| His-hGDF15 B8 +1 biotin | 26692 | 26690 | 7 | 5.05 |
| His-hGDF15 B8 +2 biotin | 26920 | 26918 | 71 | 5.05 |
| His-hGDF15 B8+3 biotin | 27148 | 27143 | 21 | 5.05 |

A 10 mg/mL solution of sulfo-NHS-biotin (ThermoFisher) in 30 mM pH 4 NaOAc was prepared. His-hGDF15 B 8 (100 µL, 0.0076 µmol) was combined with 30 mM NaOAc pH 4 (293.3 µL) and sulfo-NHS-biotin (ThermoFisher, 6.70 µL, 0.151 µmol). The mixture was shaken at r.t. for 16 hours. The mixture was exchanged into 30 mM NaOAc pH 4 using 10 kDa MWCO Amicon centrifugal filter by diluting and concentrating the sample 5 times to a volume of 150 µL. LCMS analysis indicated the presence of starting material, +1 and +2 products. The concentration was measured by A₂₈₀ (29090 M⁻¹cm⁻¹, 26917 g/mol) to be 1.08 mg/mL (80%). LCMS **Method E:** Rₜ = 5.05 min; MS [M+1+1 biotin] observed: 26690, calculated: 26692, [M+1+2 biotin]: observed: 26918, calculated: 26920, [M+1+3 biotin]: observed: 27143, calculated: 27148.

### Example 9: Site specific conjugation of BCN acylating agent with M(His)6M-hGDF15 (reference example)

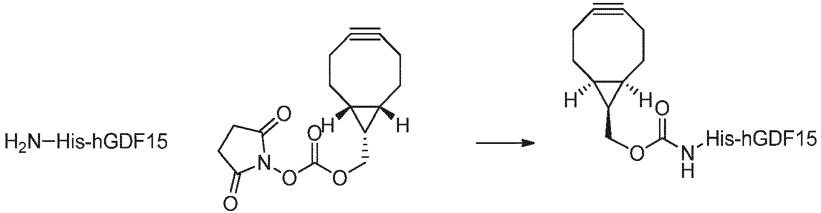

His-hGDF15

Seq:MHHHHHHMARNGDHCPLGPGRCCRLHTVRASLEDLGWADWVLSPREVQVTMCIGACP
SQFRAANMHAQIKTSLHRLKPDTVPAPCCVPASYNPMVLIQKTDTGVSLQTYDDLLAKDCHCI

A stock solution of His-hGDF15 (0.6mL, 4.8mg/mL) was diluted to 0.5mg/mL with 30mM NaOAc pH 4.5 buffer (5.2mL). A 10mg/mL stock solution of (1R,8S,9s)-bicyclo[6.1.0]non-4-yn-9-ylmethyl (2,5-dioxopyrrolidin-1-yl) carbonate (NHS-BCN) in DMSO (251µL) was slowly added and the reaction placed on a shaker plate at 24°C for 21hrs. The reaction was diluted to 30mL with 30mM NaOAc pH 4.5 buffer and concentrated to 2mL using a 10kDa MWCO ultrafiltration cartridge (repeat 4x) to yield 2.5mL of concentrate. Based on A₂₈₀ (ε = 29090M⁻¹ cm⁻¹, 26730g/mol) the concentrate was 0.93mg/mL (2.33mg, 80%): LCMS QT2_15-60kDa_15min_polar

| Degree of Labelling | Calculated | Observed | %TIC (MS+) Intensity |
|---|---|---|---|
| GDF15 | 26726 | 26726 | 26 |
| GDF15 +1BCN | 26903 | 26904 | 43 |
| GDF15 +2BCN | 27080 | 27080 | 23 |
| GDF15 +3BCN | 27257 | 27256 | 9 |

| | | | |
|---|---|---|---|
| BCN: Bicyclononynyl +1BCN and +2 BCN refer to the acylation of the N-terminus amino group of the monomeric and dimeric moieties. +3 BCN refer to non-selective acylation. | | | |

### Example 10: : Site specific conjugation of BCN acylating agent with M(His)₆-hGDF15 (reference example)

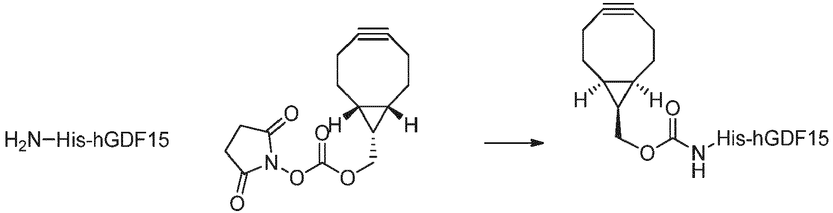

His-hGDF15 Seq:

MHHHH HHAR NGDHC PLGPG RCCRL HTVRA SLEDL GWADW VLSPR EVQVT MCIGA
CPSQF RAANM HAQIK TSLHR LKPDT VPAPC CVPAS YNPMV LIQKT DTGVS LQTYD
DLLAK DCHCI

A stock solution of His-hGDF1 (7.04mL, 1.42mg/mL) was diluted to 0.5mg/mL with 30mM NaOAc pH 4.5 buffer (12.95mL). A 10mg/mL stock solution of (1R,8S,9s)-bicyclo[6.1.0]non-4-yn-9-ylmethyl (2,5-dioxopyrrolidin-1-yl) carbonate (NHS-BCN) in DMSO (0.88mL) was slowly added and the reaction placed on a shaker plate at 24°C for 24hrs. An additional portion of the NHS-BCN stock solution (176µL) was added, and the reaction maintained at 24°C for 24hrs. The reaction was diluted to 60mL with 30mM NaOAc pH 4.5 buffer and concentrated to 4mL using a 10kDa MWCO ultrafiltration cartridge (repeat 4x) to yield 4.1mL of concentrate. Based on A₂₈₀ (ε = 29090M⁻¹ cm⁻¹, 26700g/mol) the concentrate was 2.19mg/mL (8.98mg, 89%): LCMS QT2_15-60kDa_15min_polar

| Degree of Labelling | Calculated | Observed | % TIC (MS+) Intensity |
|---|---|---|---|
| GDF15 | 26468 | 26464 | 33 |
| GDF15 +1BCN | 26645 | 26640 | 34 |
| GDF15 +2BCN | 26822 | 26817 | 21 |
| GDF15 +3BCN | 26999 | 26993 | 3 |

| | | | |
|---|---|---|---|
| BCN: Bicyclononynyl +1BCN and +2 BCN refer to the acylation of the N-terminus amino group of the monomeric and dimeric moieties. +3 BCN refer to non-selective acylation. | | | |

### Example 11: Fluorophore labeling of His-hGDF15 (reference example)

### Step 1: Preparation of the Fluorophore-linker acylating agent (I-8)

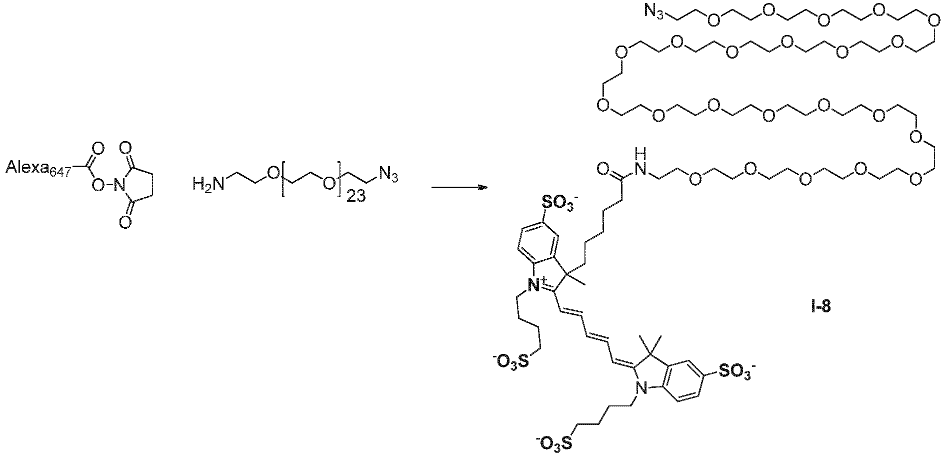

To a solution of Alexafluor647-NHS ester (Life Technologies, 5 mg, 4.00 µmol) in PBS (1 mL) was added azido-dPEG₂₃-amine (Quanta Biodesign cat# 10525, 4.84 mg, 4.4 µmol). The reaction was mixed at r.t. for 6.5 hours at which point an additional 0.25 equivalents of azido-dPEG₂₃-amine (1 mg) was added with two drops of DIPEA. The reaction was mixed at r.t. for 16 hours. Complete conversion was observed by LCMS analysis. The mixture was used in the next step without further purification. LCMS **Method E:** Rₜ = 1.73 min; MS [M+H/3]: observed: 646.1, calculated: 655.3.

### Step 2: Further derivatization of the his-hGDF15-BCN construct (example 10) with a fluorophore agent (I-8)

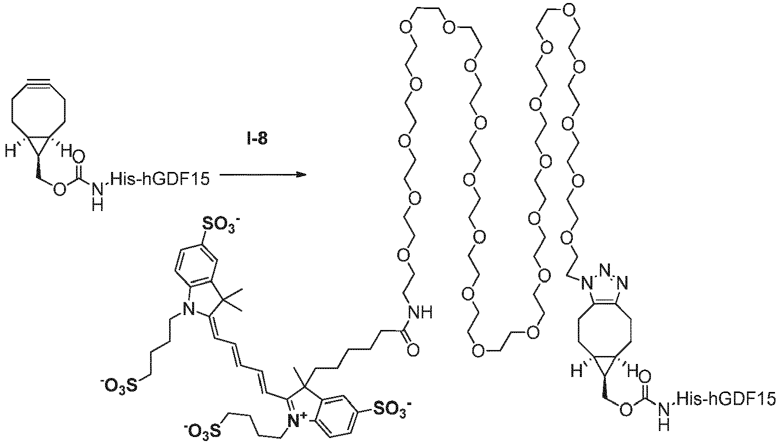

### His-hGDF15 -AF647:

| Degree of Labelling | Calculated | Observed | % | Rₜ (min) |
|---|---|---|---|---|
| His-hGDF15 | 26468 | 26465 | 27 | 6.19 |
| His-hGDF15-BCN | 26644 | N/A | 0 | 6.19 |
| His-hGDF15 +1AF647 | 28609 | 28580 | 42 | 6.19 |
| His-hGDF15+2AF647 | 30574 | 30714 | 21 | 6.19 |
| His-hGDF15+3AF647 | 32539 | 32830 | 10 | 6.19 |

To a solution of His-hGDF15-BCN (500 µL, 2.19 mg/mL, 0.041 µmol) was added a solution of 1 (20.55 µL, 7.86 mg/mL, 0.082 µol). The reaction was mixed at r.t. for 2 days at which point 75% conversion was observed by LCMS analysis. The mixture was exchanged into PBS using 10 kDa MWCO Amicon centrifugal filter by diluting and concentrating the sample 8 times to a volume of 2.25 mL. The concentration was measured by A₂₈₀ (29090 M-1cm-1, 28600 g/mol) to be 0.32 mg/mL (60%). LCMS **Method B:** Rₜ = 6.19 min; MS [M+H+1AF647]: observed 28580.0000, calculated: 28609.336.

### Example 12: site specific conjugation of Cu64 isotope chelator to GDF15 (reference example)

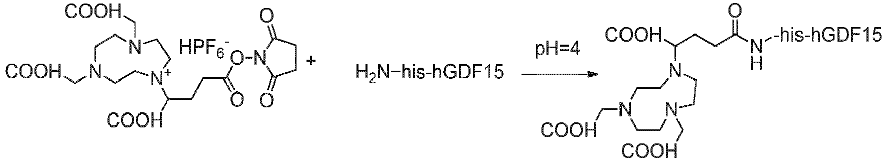

To a solution of His-hGDF15 (1.12 mg/mL, 200 uL) was added a solution of NODA-GA-NHS, HPF6, TFA salt (CheMatech Cat # C098) (20 uL, 10 mg/mL), and the mixture was stirred at r.t. for 2 days. Partial conversion (50% of +1) was observed by LC-MS analysis. 200 uL pH4 NaOAc buffer and 20 uL NODA-GA-NHS solution was added and the mixture was stirred at r.t. for 2 more days. About 70% conversion to +1 and +2 was observed by LC-MS analysis. More NODA-GA-NHS solution (20 uL) was added and the mixture was stirred at r.t. overnight. About 80% conversion to +1 and +2 was observed by LC-MS analysis. The mixture was washed with Amicon 10k 4 times to give 150 uL 0.40 mg/mL solution.

| Degree of Labelling | Calculated | Observed | % |
|---|---|---|---|
| His-hGDF15 | 26726 | 26726 | 20 |
| His-hGDF15 +1AF647 | 27084 | 27086 | 50 |
| His-hGDF15+2AF647 | 27442 | 27442 | 30 |

### Example 13: site specific conjugation of Toxin to GDF15 (reference example)

### Step 1: preparation of the Toxin contruct

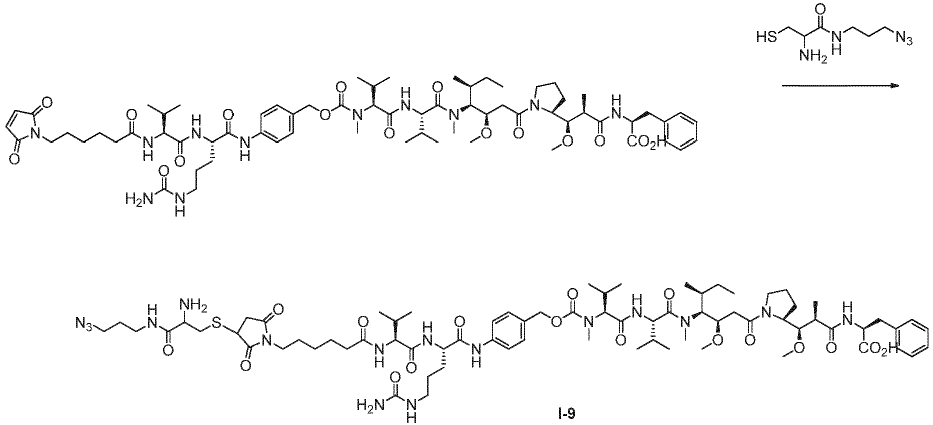

To a solution of maleimide MMAF (Concortis) (1.57 mg, 1.2 eq) in DMSO (130 uL) was added a solution of 2-AMINO-N-(3-AZIDOPROPYL)-3-MERCAPTOPROPIONAMIDE (0.2 mg, 1 eq) in 72.7 uL DMSO, and DIPEA (1 uL, 6 eq), and the mixture is stirred at r.t. for 1h. Partial conversion is observed by LC-MS analysis. More azide cystein (100 uL) was added. Reaction stirred three days.

Scale up: 1.7 mg maleimide MMAF (Concortis) (in 200 uL DMSO) was added to cys-N3 (130 uL+100 uL) and DIPEA (2 uL) and reaction stirred over three days.

Both mixtures were combined and purified by Sunfire C18 eluting with 10-90% ACN/wather (0.1% TFA) (rt=1.15min) to give 2.9 mg clean product in 92% yield.

### Step 2: Site specific conjugation of BCN to His-cvnoGDF15 (corresponding to step b of the instant invention)

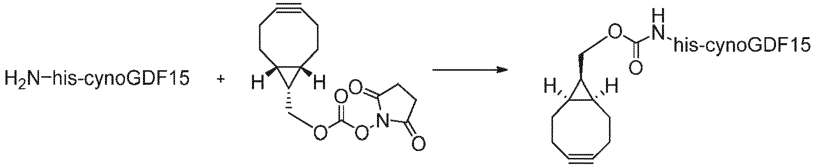

To his-cynoGDF15 (B1, 150 uL, 0.87 mg/mL) was added pH 4 buffer (450 uL) and NHS-BCN (Berry and associates cat # LK4320) in DMSO (10 mg/mL, 4.12 uL) and the mixture was stired at 4C over weekend. 60% conversion was evident after 1 day at 4C, at which time 7 eq NHS-BCN in DMSO (10mg/ml, 4.12ul) was added. After another 24hrs at 4C, 13 eq NHS-BCN (10mg/mL in DMSO, 8ul) were added and reaction incubated at rt 4hs. Partial conversion was observed. The mixture was incubated at r.t. for another 4 hr. Complete conversion was observed and the mixture was diluted to 15 mL and washed/concentrated via Amicon 10K cartridge 4 times to give 7 mL 0.60 mg/mL solution (4.2mg) in 88% yield. LCMS: +1 mass expected: 26844, mass observed: 26842 ; +2 mass expected: 27024 mass observed:27020.

### Step 3: Further modification of the conjugate construct

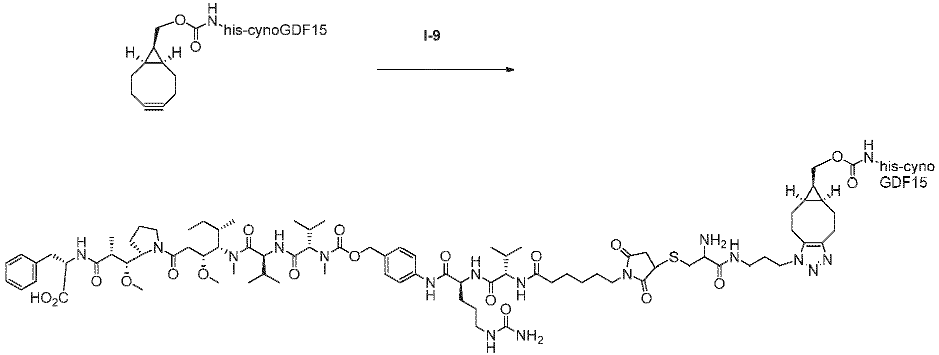

To BCN-GDF15 (7 mL, 0.6 mg/mL) was added Azido-MMAF (200 uL, 7.25 mg/mL in DMSO) and the mixture was stirred at r.t. over three days followed by 30°C for 2 days. About 70% conversion to mono- and bis-modified product was obtained. The product was washed with Amicon 10k to give 4.7 mL of 1 mg/mL product in pH 4 30mM sodium acetate in quantitative yield. Maldi: sm (25%) calculated mass: (+1) 26844 (+2) 27024 observed mass: (+1) 26699; +1 toxin (50%) calculated mass: 28377 observed mass: (+1) 28525; +2 toxin (25%) calculated mass: 30088 observed mass: 29910.

### Example 14: Site specific conjugation of Fc to an APJ peptide agonist

### General scheme:

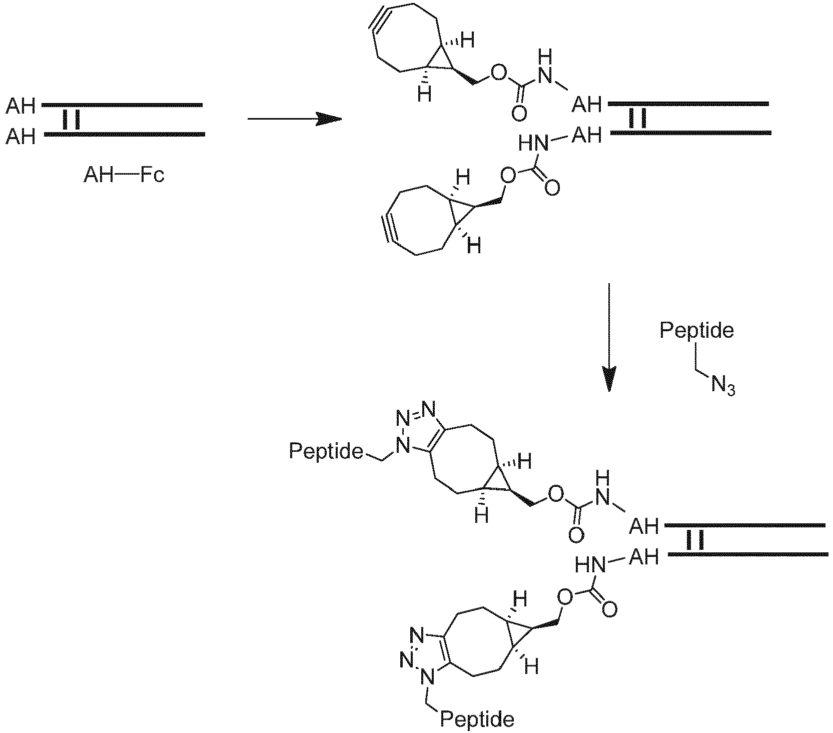

### Step 1: Synthesis of NH2-AzidoLys-GGGGS-Q-R-P-C-L-S-C-K-G-P-DNIe-Phenethylamine

### Intermediate 14

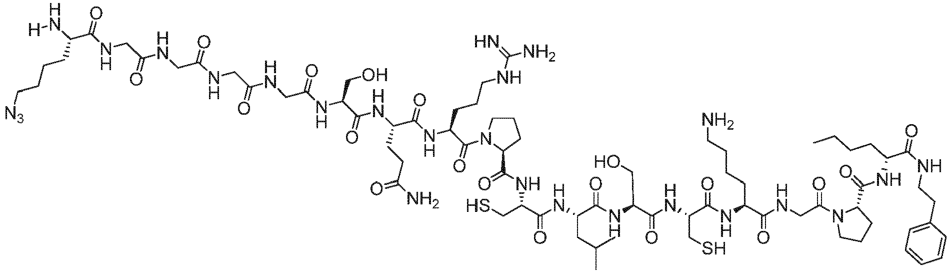

Phenethylamine-AMEBA resin (Sigma Aldrich, 0.25 mmol, 1.0 mmol/g) was subjected to solid phase peptide synthesis on an automatic peptide synthesizer (CEM Liberty Blue Microwave) with standard double Arg for the Arg residues and Dnle and Azidolysine coupled double time. Amino acids were prepared as 0.2 M solution in DMF. A standard coupling cycle was defined as follows:
• Amino acid coupling: AA (5 eq.), HATU (5 eq.), DIEA (25 eq.)
• Washing: DMF (3 X 7 mL)
• Fmoc Deprotection: 20% Piperidine/0.1 M HOBt (2 x 7 mL)
• Washing: DMF (4 x 7 mL then 1 x 5 mL)

| Coupling | AA | Number of couplings x Reaction time (Temp) | Coupling Method |
|---|---|---|---|
| 1 | Fmoc-D-Nle-OH | 1 x 10 min (70°C) | DIEA/HATU |
| 2 | Fmoc-L-Pro-OH | 1 x 5 min (70°C) | DIEA/HATU |
| 3 | Fmoc-L-Gly-OH | 1 x 5 min (70°C) | DIEA/HATU |
| 4 | Fmoc-L-Lys-OH | 1 x 5 min (70°C) | DIEA/HATU |
| 5 | Fmoc-L-Cys-OH | 1 x 5 min (70°C) | DIEA/HATU |
| 6 | Fmoc-L-Ser-OH | 1 x 5 min (70°C) | DIEA/HATU |
| 7 | Fmoc-L-Leu-OH | 1 x 5 min (70°C) | DIEA/HATU |
| 8 | Fmoc-L-Cys-OH | 1 x 5 min (70°C) | DIEA/HATU |
| 9 | Fmoc-L-Pro-OH | 1 x 5 min (70°C) | DIEA/HATU |
| 10 | Fmoc-L-Arg-OH | 2 x 25 min (25°C) | DIEA/HATU |
| 11 | Fmoc-L-Gln-OH | 1 x 5 min (70°C) | DIEA/HATU |
| 12 | Fmoc-L-Ser-OH | 1 x 5 min (70°C) | DIEA/HATU |
| 13 | Fmoc-L-Gly-OH | 1 x 5 min (70°C) | DIEA/HATU |
| 14 | Fmoc-L-Gly-Gly-Gly-OH | 1 x 10 min (70°C) | DIEA/HATU |
| 15 | Fmoc-L-AzidoLys-OH | 1 x 10 min (70°C) | DIEA/HATU |

After the assembly of the peptide, the resin was washed with DMF (2 x 50 mL) and DCM (2 x 50 mL) then dried under vacuum to give **Intermediate 14a** (770 mg, 0.250 mmol).

### Step 2: Preparation of Intermediate 14b (Cleavage of peptide from resin)

**Intermediate 14a** (770 mg, 0.250 mmol) was divided in half and each sample was combined with 6 mL TFA solution (37 mL TFA, 1 mL H2O, 1 mL TIPS, 2.569 g (20eq.) DTT) and shaken at r.t. for 3 hours. The solution was removed from the resin and precipitated into 40 mL cold Et₂O. The solution was vortexed and let stand over ice for 10 minutes before centrifuging at 4000 rpm for 5 minutes. The solvent was removed and the white solid was washed twice more with cold Et₂O (40 mL each time), centrifuged (5 minutes each time) and decanted. The solid was dried under vacuum overnight and purified via M-triggered HPLC yielding **Intermediate 14b** as a white powder (80 mg, 0.045 mmol, 80%). LCMS (SQ2 ProductAnalysis-Acidic-Peptide-Polar, Acquity UPLC BEH C18 column, 130 Å, 1.7 µm, 2.1 mm x 50 mm, 50°C): Rₜ = 2.32 minutes, MS [M+H+2/2] 888.0.

### Step 3: Preparation of Intermediate 14c (Cyclization of cysteine residues)

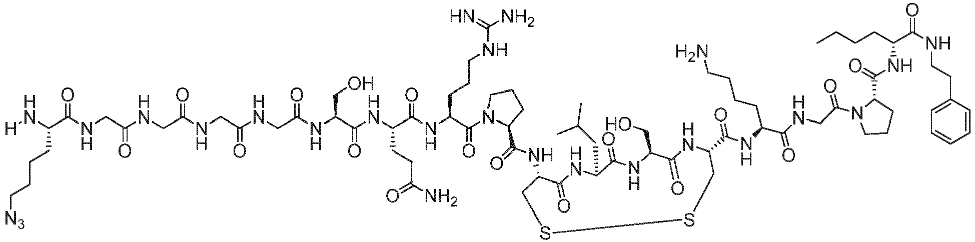

**Intermediate 14b** (80 mg, 0.045 mmol) was dissolved in water (1.25 mL) and iodine (50 mM in HOAc, 1.804 mL, 0.090 mmol) was added slowly dropwise and the reaction was mixed at r.t. for 3 hours. LCMS analysis of the crude reaction showed complete conversion of starting material. 0.5 M ascorbic acid was added dropwise until color dissipated. The material was purified via MS-triggered HPLC. Lyophilization of the pooled fractions gave 20.1 mg of the desired product as a white powder (0.045 mmol, 25%). LCMS (SQ2 ProductAnalysis-Acidic-Peptide-Polar, Acquity UPLC BEH C18 column, 130 Å, 1.7 µm, 2.1 mm x 50 mm, 50°C): Rₜ = 2.17 minutes, MS [M+H+2/2] 886.8.

### Step 4: Preparation of Fc-AH construct

### AH-Fc Construct Cloning:

The DNA fragment shown below was generated by standard PCR techniques using the vector pPL1146 containing the mouse Ig Kappa signal sequence followed by the human Fc downstream of a CMV promoter, as a template. A forward primer (5'-GCT TGC TAG CCA CCA TGG AAA CTG-3') was designed that contains a Nhel site followed by sequences corresponding to the 5' end of the signal sequence contained in vector pPL1146. A reverse primer (5'-GTT GAT TGA ATT CTT AGA AGC ACA TGG GGC CCT TGT GGC ACA GCC GGG GCC GCT GGC TTC CGC CAC CTC CGC TGC CTC CAC CGC CGC TGC CTC CGC CAC CTG CGC CTG GAC TCA GAG ACA GGG-3') was designed to contain a EcoRI site, Apelin sequence, a glycine serine linker and sequence complementary to the 3' end of the human Fc contained in pPL1146. Following amplification, the fragment was restriction digested with both Nhel and EcoRI, isolated and purified from an agarose gel, and ligated into vector pPL1146 digested and purified in the same manner. The ligations were transformed into E coli DH5□ cells and colonies containing the correct plasmids were identified by DNA sequencing. Sequences shown are for the sense strand and run in the 5'- to 3'- direction.

Nucleotide sequence

GCTAGCCACCATGGAAACTGACACCCTGCTGCTGTGGGTCCTGCTGCTGTGGGTGCCTGG
CAGCACTGGCGCTCATGATAAGACACACACATGCCCCCCTTGTCCAGCACCAGAGGCAGC
TGGAGGACCAAGCGTGTTCCTGTTTCCACCCAAGCCTAAAGACACACTGATGATCTCAAGG
ACCCCAGAAGTCACATGCGTGGTCGTGGACGTGTCTCACGAGGACCCCGAAGTCAAGTTC
AACTGGTACGTGGATGGCGTCGAGGTGCATAATGCTAAGACCAAACCCCGAGAGGAACAG
TACAACAGCACCTATCGGGTCGTGTCCGTCCTGACAGTGCTGCACCAGGATTGGCTGAAC
GGCAAAGAGTATAAGTGCAAAGTGAGTAATAAGGCTCTGCCTGCACCAATCGAGAAAACAA
TTTCTAAGGCTAAAGGGCAGCCAAGAGAACCCCAGGTGTACACTCTGCCTCCATCTAGGG
AGGAAATGACAAAGAACCAGGTCAGTCTGACTTGTCTGGTGAAAGGCTTCTACCCCTCCGA
CATCGCAGTGGAGTGGGAATCTAATGGCCAGCCTGAAAACAATTACAAGACCACACCCCC
TGTGCTGGACTCCGATGGGTCTTTCTTTCTGTATTCTAAGCTGACCGTGGATAAAAGTCGG
TGGCAGCAGGGAAACGTCTTCTCATGCAGCGTGATGCACGAGGCCCTGCACAATCATTAC
ACACAGAAGTCCCTGTCTCTGAGTCCAGGCAAATGAGAATTC

### AH-Fc Protein Expression and Purification:

AH-Fc expression plasmid DNA was transfected into HEK293T cells at a density of 1 x 10⁶ cells per ml using standard polyethylenimine methods. 500 ml cultures were then grown in FreeStyle 293 Medium (Life Technologies) in 3 L flasks for 4 days at 37 °C.

AH-Fc protein was purified from clarified conditioned media. Briefly 500 ml of conditioned media was flowed over a 5 ml HiTrap MabSelect SuRe column (GE Life Sciences) at 4 ml/min. The column was washed with 20 column volumes of PBS containing 0.1% Triton X-114 and then the Fc-AH protein was eluted with 0.1M glycine, pH 2.7, neutralized with 1 M Tris-HCl, pH 9 and dialyzed against PBS. Protein yields were 10 to 20 mg per 500 ml conditioned media and endotoxin levels were <1 EU/mg as measured by the Charles River ENDOSAFE PTS test.

The protein was characterized by LC/MS of the reduced, N-deglycosylated AH-Fc proteins. The molecular weight for Fc-AH was as expected.

**LC/MS of native AH-Fc protein:** Peaks were heterogeneous and about 3 kDa larger than expected for dimers. This is characteristic of N-linked glycosylation expected for Fc which has a consensus N-linked glycosylation site.

**Analytical size exclusion on Superdex 200:** AH-Fc protein have between 89 and 100% dimer, 0 to 10% tetramer, and 0 to 1% aggregate.

**Reducing SDS/PAGE:** protein migrated as predominately monomers of the expected size.

### Step 5: Preparation of AH-Fc BCN (Installation of click handle on Fc-HA N-terminus)

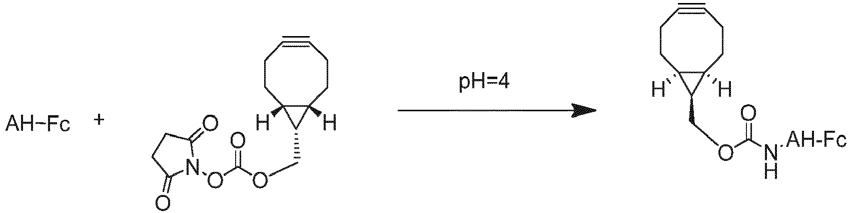

AH-Fc sequence:

AHDKTHTCPPCPAPEAAGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKGQPR

EPQVYTLPPSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK

AH-Fc (from step 4: 1 mL, 3 mg/mL, 0.117 µmol) was dissolved in 30 mM NaOAc pH 4.0 (4.3 mL) and a 10mg/mL stock solution of (1R,8S,9s)-bicyclo[6.1.0]non-4-yn-9-ylmethyl (2,5-dioxopyrrolidin-1-yl) carbonate (BCN) in DMSO (0.70 mL) was slowly added and the reaction placed on a shaker plate at r.t. for 3 days. BCN (0.25 mL) was added and the reaction was mixed at r.t. for 3 days. BCN (0.70 mL) was added and the reaction was mixed at r.t. for 16 hours. The solution was exchanged into 30 mM NaOAc pH 4.0 using 10 kDa MWCO Amicon centrifugal filter by diluting and concentrating the reaction 5 times to a volume of 900 µL. The solution was centrifuged and supernatant removed. The concentration was measured by A280 to be 1.73 mg/mL (1.56 mg, 26%). LCMS (QT2, Protein_20-70 kDa_3min, Proswift Monolith 4.6 x 50 mm, 50°C, Eluent A: Water + 0.1% Formic Acid, Eluent B: CAN + 0.1% Formic Acid, 2-98% over 2 min) Rₜ = 1.58 min.

| Degree of Labelling | Calculated | Observed | % TIC (MS+) Intensity |
|---|---|---|---|
| Fc-AH | 51141 | 51141.5 | 18 |
| Fc-AH +1BCN | 51318 | 51317 | 31 |
| Fc-AH +2BCN | 51495 | 51496 | 31 |
| Fc-AH +3BCN | 51672 | 51671 | 20 |

### Step 6: Fc-HA- BCN + Intermediate 14c (Conjugation of CNM peptide to Fc)

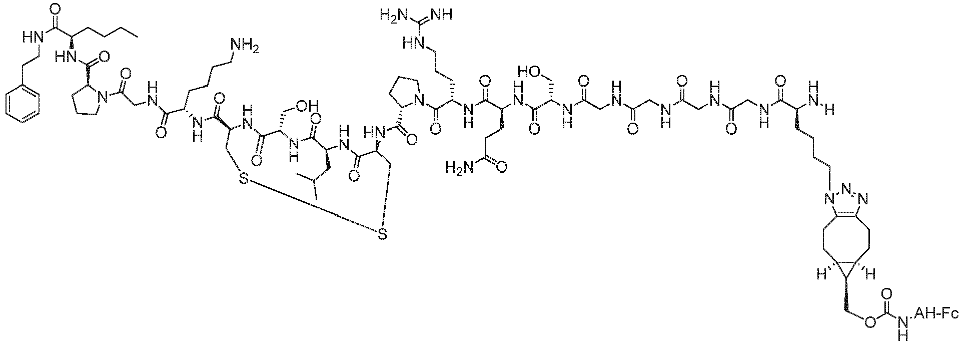

A 50 mg/mL stock solution of **intermediate 14c** in H₂O was prepared. **Intermediate 14c** (53.7 µL, 1.515 µmol) was added to a stock solution of **Fc-HA-BCN** in 30 mM NaOAc pH 4.0 (1.73 mg/mL, 1.56 mg, 0.030 µmol) and the reaction was mixed at r.t. for 16 hours. The solution was exchanged into 30 mM NaOAc pH 4.0 using 50 kDa MWCO Amicon centrifugal filter by diluting and concentrating the reaction 5 times to a volume of 250 µL. The concentration was measured by A280 to be 3.32 mg/mL (830 µg, 50%). LCMS (QT2, Protein_35-70 kDa_3min, Proswift Monolith 4.6 x 50 mm, 50°C, Eluent A: Water + 0.1% Formic Acid, Eluent B: CAN + 0.1% Formic Acid, 10-80%B over 2 min) Rₜ = 1.43 min, MS [M(glycosylated)+H] 54524.5.

## Claims

1. A method for modifying a target protein or a target peptide at the amino functionality of the N-terminal amino acid comprising the steps of:
a. modifying the target protein or target peptide so that the resulting protein or peptide contains a histidine amino acid at the position adjacent to the N-terminus amino acid;
b. contacting the protein or peptide containing a histidine amino acid at the position adjacent to the N-terminus amino acid with an acylating agent at a pH lower than 6, to form a modified protein or peptide;
wherein the acylating agent is of the following Formula: 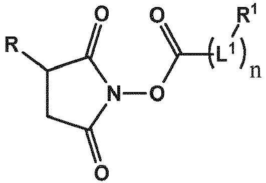 wherein
R is H or SO₃Na;
L¹ is a linear or branched C₁-C₃₀ alkyl or an unbranched oligo ethylene glycol moiety of Formula: 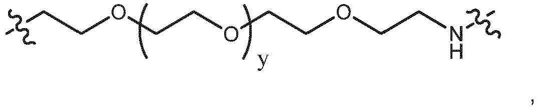 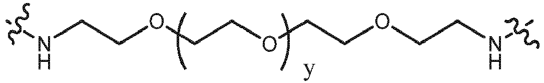 or, 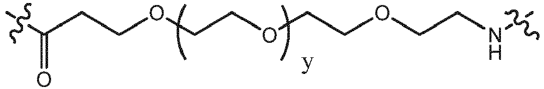
wherein y is 0 to 34;
R¹ is biotin or a fatty acid moiety; and
n is 0 or 1;
or wherein the acylating agent is of the formula: 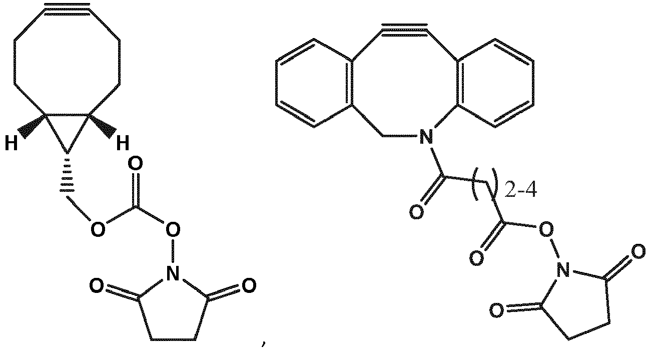 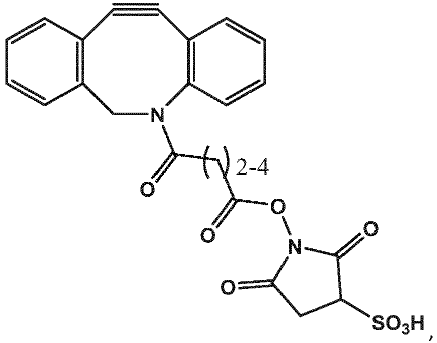 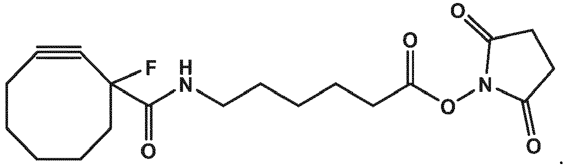 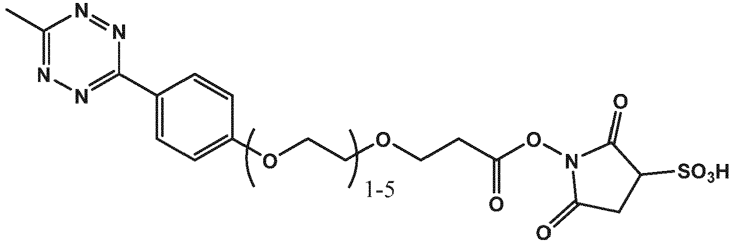
or 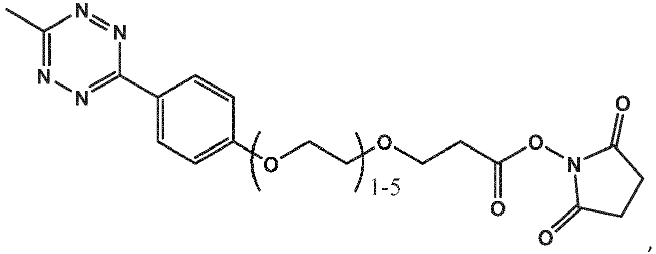 and wherein
the protein or peptide containing a histidine amino acid at the position adjacent to the N-terminus amino acid is prepared by replacing the two amino acids at the N-terminus of said target protein or target peptide with an amino acid sequence XH- wherein H is histidine and X is the amino acid A; or
the protein or peptide containing a histidine amino acid at the position adjacent to the N-terminus amino acid is prepared by replacing the three amino acids at the N-terminus of said target protein or target peptide with an amino acid sequence XHX'- wherein H is histidine and X and X' are the amino acid A; or
the protein or peptide containing a histidine amino acid at the position adjacent to the N-terminus amino acid is prepared by appending a two amino acid sequence of Formula XH or a three amino acid sequence of Formula XHX'- at the N-terminus of said target protein or target peptide, wherein H is histidine and X and X' are A.

2. The method according to claim 1, wherein R¹ is selected from the following acids: 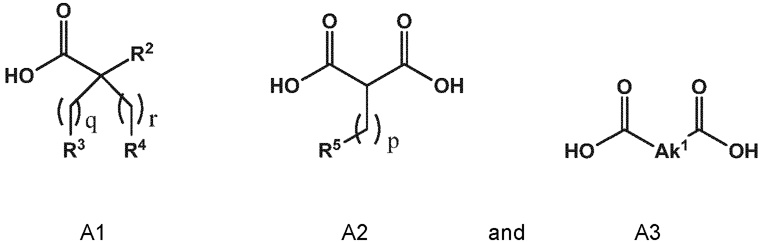
R² is CO₂H, H;
R³, R⁴ and R⁵ are independently of each other H, OH, CO₂H, -CH=CH₂ or -C≡CH;
Ak¹ is a branched C₆-C₃₀alkylene;
q, r and p are independently of each other an integer between 6 and 30; and wherein the point of attachment is any of the CO2H functionality; or an amide, an ester or a pharmaceutically acceptable salt thereof.

3. The method according to claim 2, wherein R1 is selected from the following fatty acids:

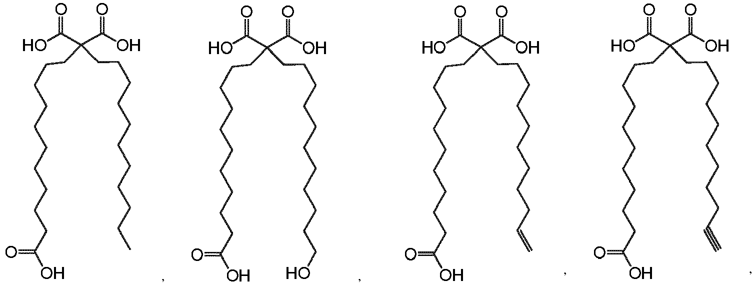

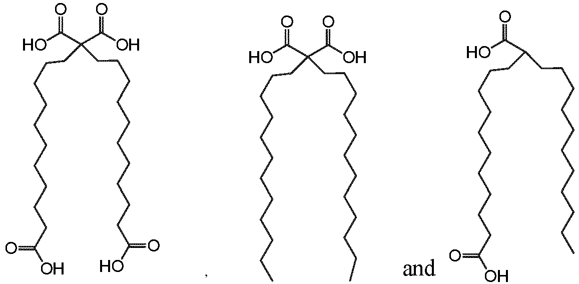

4. The method according to claim 1 wherein the acylating agent is: 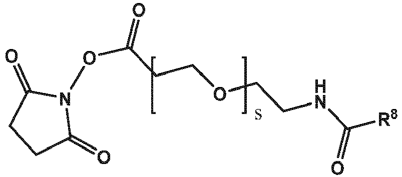 wherein
R8 is a linear or branched C6-70alkyl, a linear or branched C6-70alkenyl or a linear or branched C6-70alkynyl, each of which is optionally substituted with 1 to 3 substituents selected from carboxy and hydroxyl; and
s is 1 to 34.

5. The method according to any one of claims 1 to 4 wherein the acylating agent is:

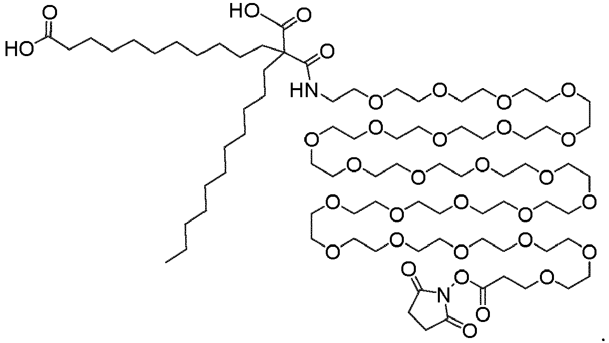

6. The method according to claim 1 wherein the acylating agent is:

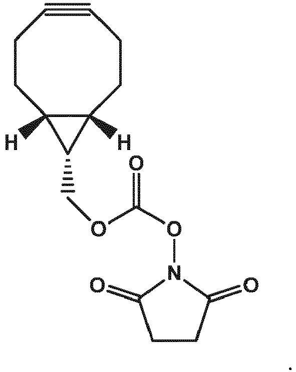

7. The method according to claim 6 further comprising a step of reacting a biointeractive agent or an analytical agent of the following formula: 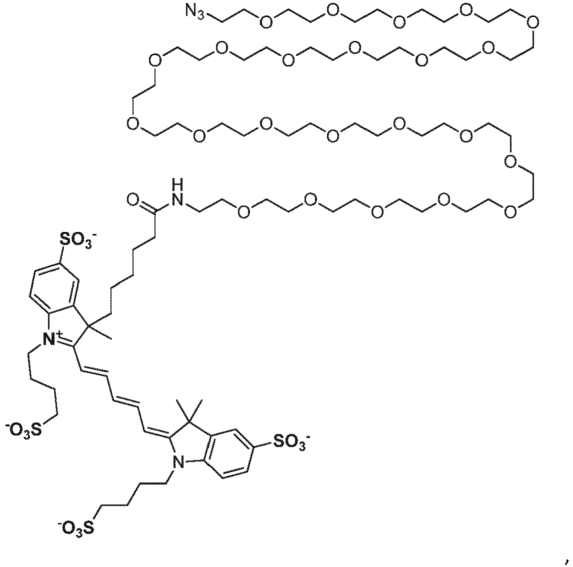 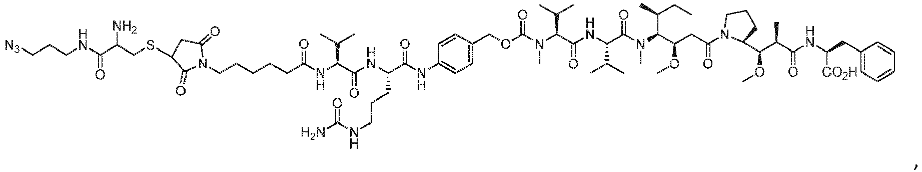 or 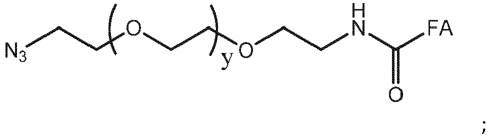 wherein y is 0 to 34 and FA is of the following Formulae: 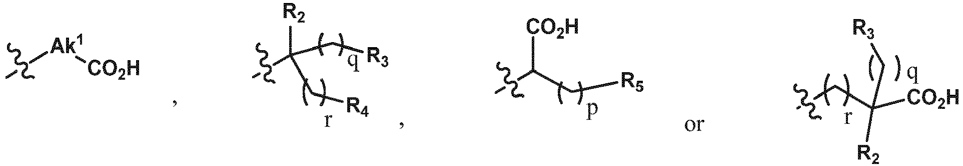
wherein Ak¹ is a branched C₆-C₃₀alkylene;
R² is CO₂H or H;
R³, R⁴ and R⁵ are independently of each other H, OH, CO₂H, -CH=CH₂ or -C≡CH;
q, r and p are independently of each other an integer between 6 and 30;
with the reactive group present in the acylating agent.

8. The method according to any one of the preceding claims wherein the target protein or target peptide is APJ agonist peptide, a native Fc or a GDF15 protein.

9. The method according to any one of the preceding claims wherein the pH is 4.

## Patentansprüche

1. Verfahren zum Modifizieren eines Zielproteins oder eines Zielpeptids an der Aminofunktionalität der N-terminalen Aminosäure, welches die folgenden Schritte umfasst:
a. das Modifizieren des Zielproteins bzw. Zielpeptids, so dass das erhaltene Protein bzw. Peptid eine Histidinaminosäure in der zur N-terminalen Aminosäure benachbarten Position enthält,
b. das Inkontaktbringen des eine Histidinaminosäure in der zur N-terminalen Aminosäure benachbarten Position enthaltenen Proteins bzw. Peptids mit einem Acylierungsmittel bei einem pH-Wert von unter 6 unter Bildung eines modifizierten Proteins bzw. Peptids,
wobei das Acylierungsmittel die folgende Formel aufweist: 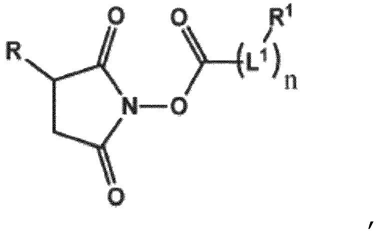
wobei R für H oder SO₃Na steht,
L¹ für geradkettiges oder verzweigtes C₁-C₃₀-Alkyl oder eine unverzweigte Oligoethylenglykolgruppierung der Formel: 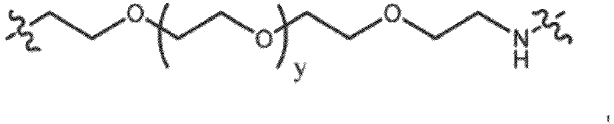 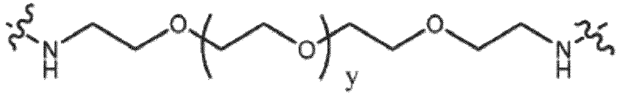 oder 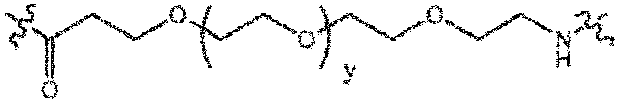
steht,
wobei y für 0 bis 34 steht,
R¹ für Biotin oder eine Fettsäuregruppierung steht und
n für 0 oder 1 steht,
oder wobei das Acylierungsmittel die folgende Formel aufweist: 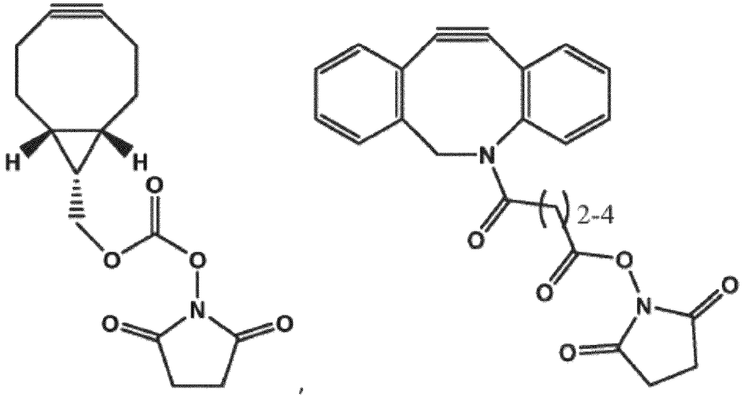 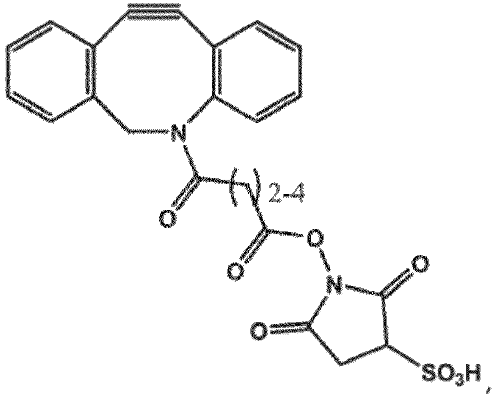 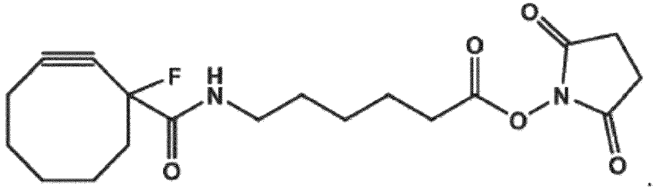 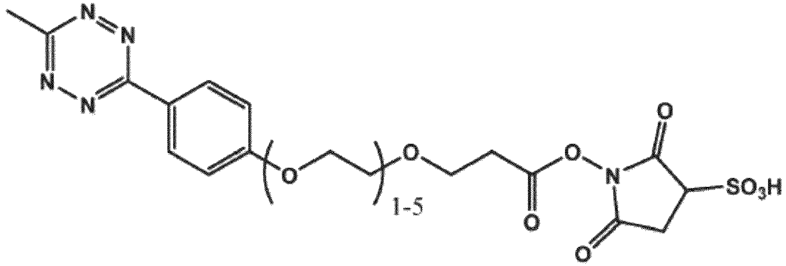
oder 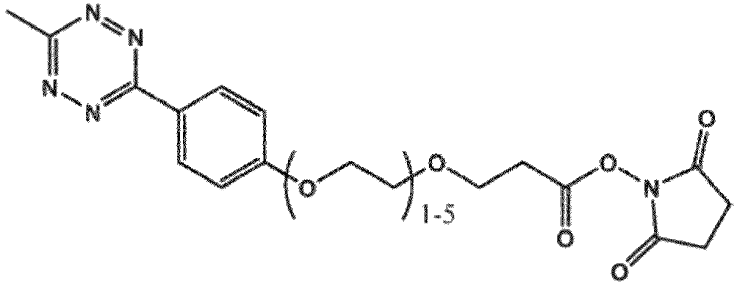 und wobei
das eine Histidinaminosäure in der zur N-terminalen Aminosäure benachbarten Position enthaltene Protein bzw. Peptid hergestellt wird, indem man die beiden Aminosäuren am N-Terminus des Zielproteins bzw. Zielpeptids durch eine Aminosäuresequenz XH-, wobei H für Histidin steht und X für die Aminosäure A steht, ersetzt oder
das eine Histidinaminosäure in der zur N-terminalen Aminosäure benachbarten Position enthaltene Protein bzw. Peptid hergestellt wird, indem man die drei Aminosäuren am N-Terminus des Zielproteins bzw. Zielpeptids durch eine Aminosäuresequenz XHX'-, wobei H für Histidin steht und X und X' für die Aminosäure A stehen, ersetzt oder
das eine Histidinaminosäure in der zur N-terminalen Aminosäure benachbarten Position enthaltene Protein bzw. Peptid hergestellt wird, indem man eine 2-Aminosäuren-Sequenz der Formel XH oder eine 3-Aminosäuren-Sequenz der Formel XHX'- am N-Terminus des Zielproteins bzw. Zielpeptids anfügt, wobei H für Histidin steht und X und X' für A stehen.

2. Verbindung nach Anspruch 1, wobei R¹ aus den folgenden Säuren ausgewählt ist: 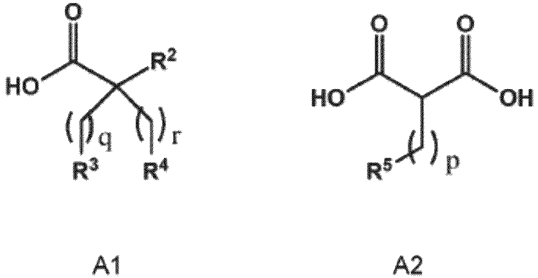 und 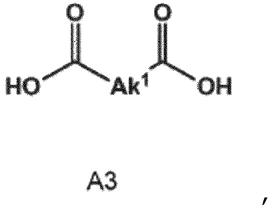
R² für CO₂H, H steht,
R³, R⁴ und R⁵ unabhängig voneinander für H, OH, CO₂H,-CH=CH₂ oder -C=CH stehen,
Ak¹ für verzweigtes C₆-C₃₀-Alkylen steht,
q, r und p unabhängig voneinander für eine ganze Zahl zwischen 6 und 30 stehen, und wobei sich der Anbindungspunkt an einer der CO2H-Funktionalitäten befindet, oder ein Amid, ein Ester oder ein pharmazeutisch unbedenkliches Salz davon.

3. Verfahren nach Anspruch 2, wobei R¹ aus den folgenden Fettsäuren ausgewählt ist:

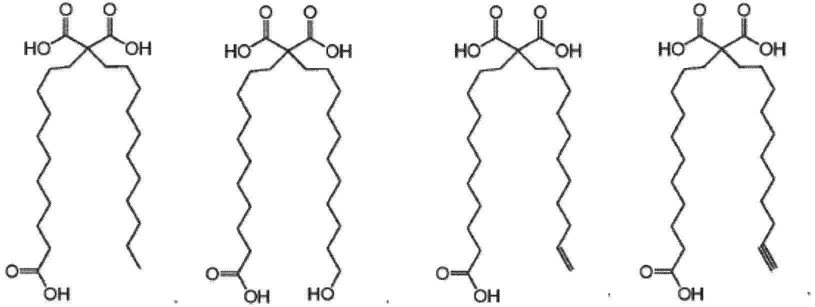

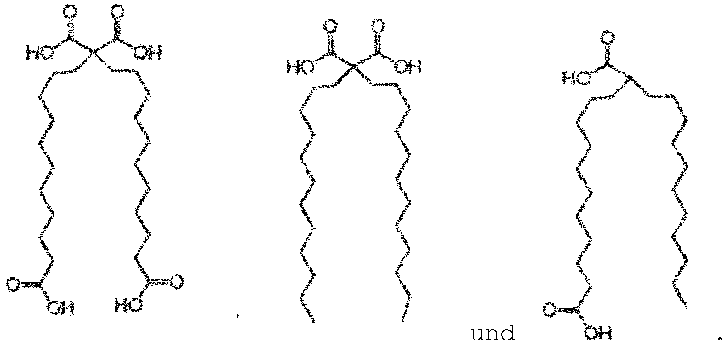

4. Verfahren nach Anspruch 1, wobei es sich bei dem Acylierungsmittel um: 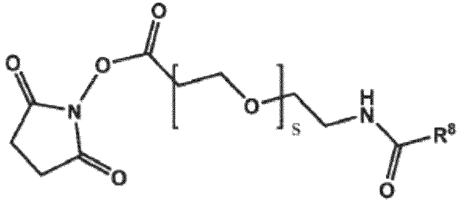 handelt, wobei
R8 für geradkettiges oder verzweigtes C6-70-Alkyl, geradkettiges oder verzweigtes C6-70-Alkenyl oder geradkettiges oder verzweigtes C6-70-Alkinyl steht,
jeweils gegebenenfalls substituiert durch 1 bis 3 aus Carboxy und Hydroxyl ausgewählte Substituenten, und
s für 1 bis 34 steht.

5. Verfahren nach einem der Ansprüche 1 bis 4, wobei es sich bei dem Acylierungsmittel um: 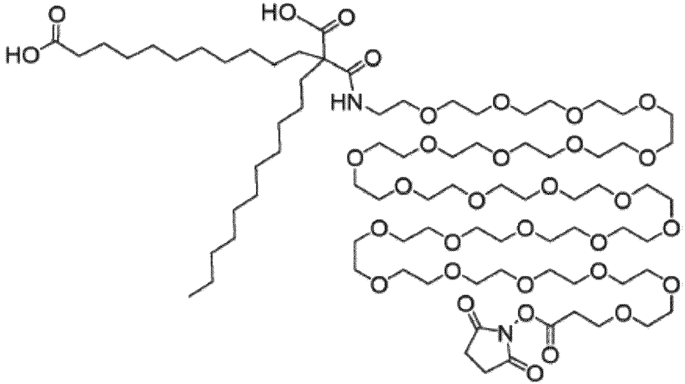 handelt.

6. Verfahren nach Anspruch 1, wobei es sich bei dem Acylierungsmittel um: 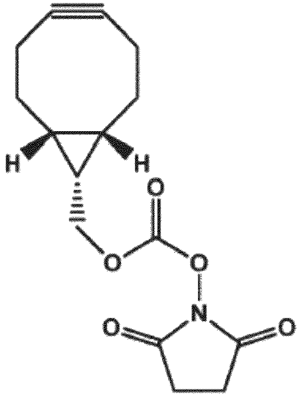 handelt.

7. Verfahren nach Anspruch 6, weiterhin umfassend einen Schritt, bei dem man ein biointeraktives Mittel oder ein analytisches Mittel der folgenden Formel: 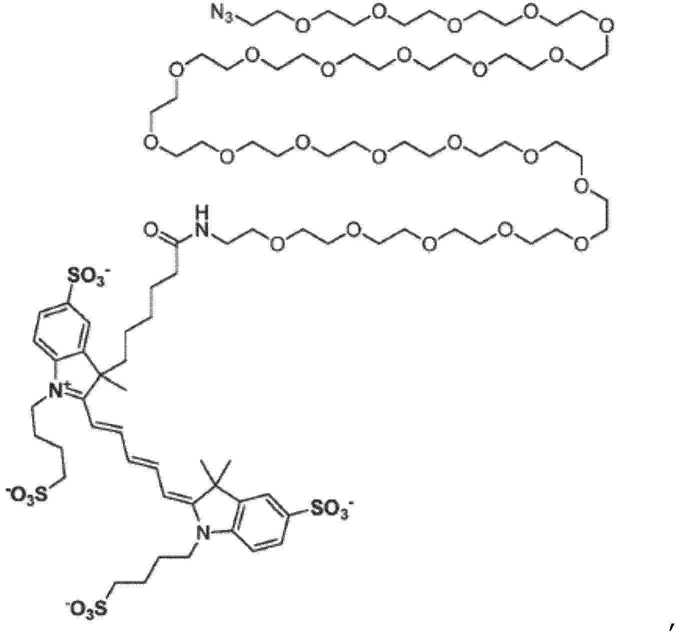 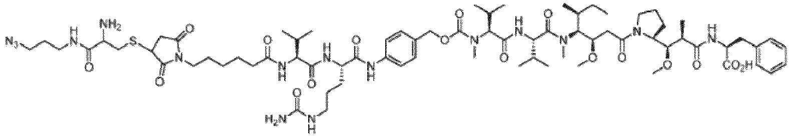 oder 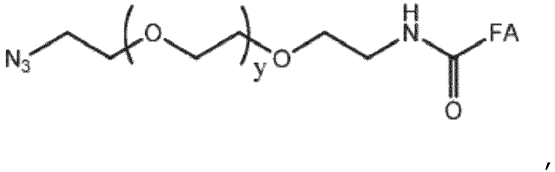 wobei y für 0 bis 34 steht und FA für eine der folgenden Formeln steht: 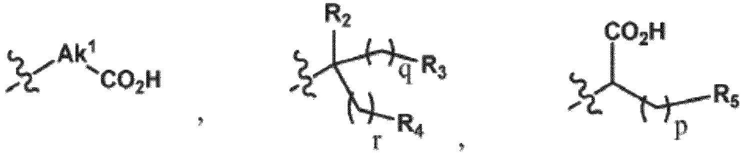 oder 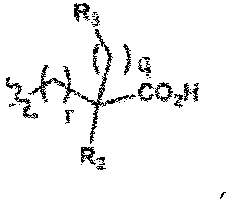
wobei Ak¹ für verzweigtes C₆-C₃₀-Alkylen steht,
R² für CO₂H oder H steht,
R³, R⁴ und R⁵ unabhängig voneinander für H, OH, CO₂H,-CH=CH₂ oder -C=CH stehen,
q, r und p unabhängig voneinander für eine ganze Zahl zwischen 6 und 30 stehen,
mit der in dem Acylierungsmittel vorhandenen reaktiven Gruppe umsetzt.

8. Verfahren nach einem der vorhergehenden Ansprüche, wobei es sich bei dem Zielprotein bzw. Zielpeptid um APJ-Agonist-Peptid, ein natives Fc oder ein GDF15-Protein handelt.

9. Verfahren nach einem der vorhergehenden Ansprüche, wobei der pH-Wert 4 beträgt.

## Revendications

1. Procédé de modification d'une protéine cible ou d'un peptide cible au niveau de la fonctionnalité amino de l'acide aminé N-terminal comprenant les étapes de :
a. modification de la protéine cible ou du peptide cible de sorte que la protéine ou le peptide résultant(e) contient un acide aminé histidine à la position adjacente à l'acide aminé N-terminal ;
b. mise en contact de la protéine ou du peptide contenant un acide aminé histidine à la position adjacente à l'acide aminé N-terminal avec un agent d'acylation à un pH inférieur à 6, pour former une protéine ou un peptide modifié(e) ;
dans lequel l'agent d'acylation est de la formule suivante : 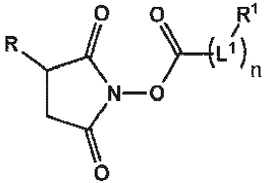 dans laquelle
R est H ou SO₃Na ;
L¹ est un alkyle en C₁-C₃₀ linéaire ou ramifié ou un fragment oligo-éthylène glycol non ramifié de formule : 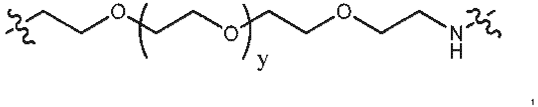 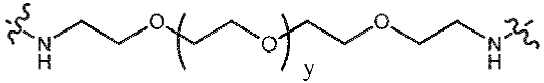 ou 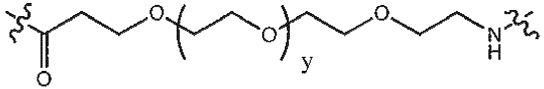
dans lequel y est 0 à 34 ;
R¹ est la biotine ou un fragment d'acide gras ; et n est 0 ou 1 ;
ou dans lequel l'agent d'acylation est de formule : 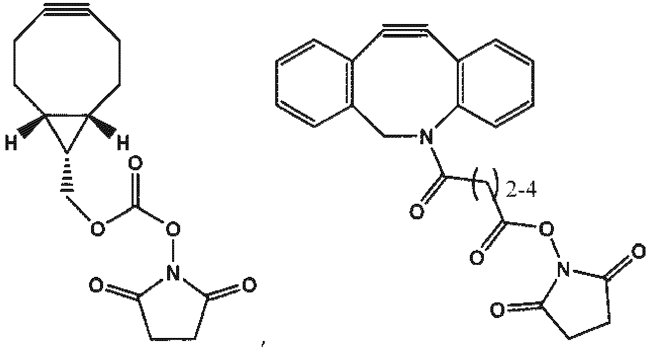 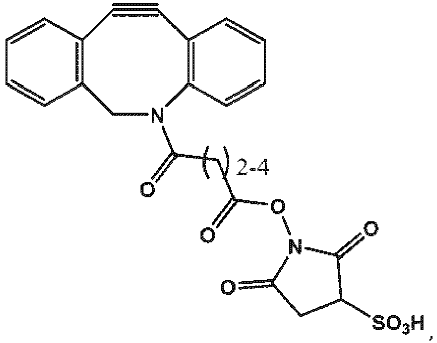 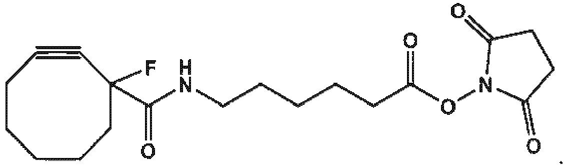 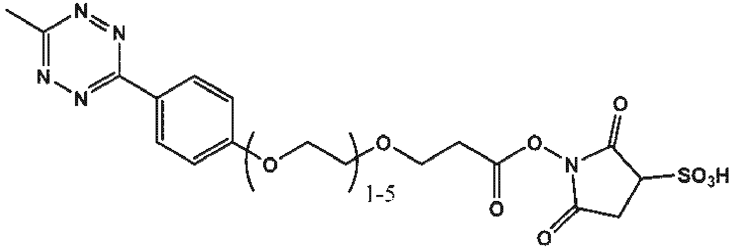
ou 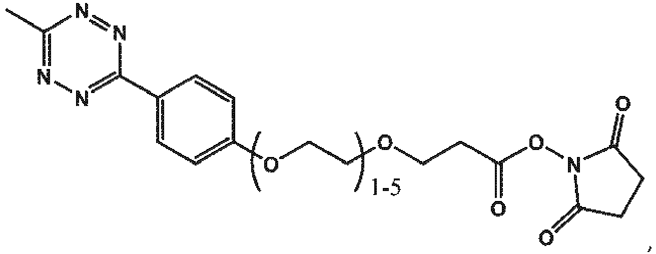 et dans lequel
la protéine ou le peptide contenant un acide aminé histidine à la position adjacente à l'acide aminé N-terminal est préparé(e) par remplacement des deux acides aminés à l'extrémité N-terminale de ladite protéine cible ou dudit peptide cible par une séquence d'acides aminés XH-, H étant histidine et X étant l'acide aminé A ; ou la protéine ou le peptide contenant un acide aminé histidine à la position adjacente à l'acide aminé N-terminal est préparé (e) par remplacement des trois acides aminés à l'extrémité N-terminale de ladite protéine cible ou dudit peptide cible par une séquence d'acides aminés XHX'-,H étant l'histidine et X et X' étant l'acide aminé A ; ou
la protéine ou le peptide contenant un acide aminé histidine à la position adjacente à l'acide aminé N-terminal est préparé(e) par liaison d'une séquence de deux acides aminés de formule XH ou d'une séquence de trois acides aminés de formule XHX'- à l'extrémité N-terminale de ladite protéine cible ou dudit peptide cible, H étant l'histidine et X et X' étant A.

2. Procédé selon la revendication 1, dans lequel R¹ est choisi parmi les acides suivants : 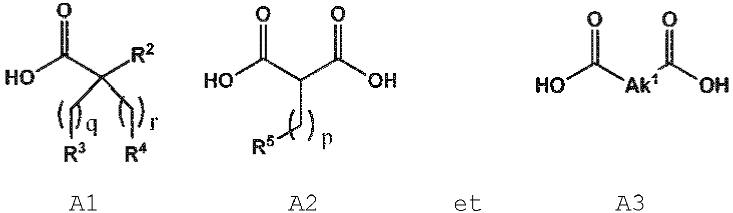
R² est CO₂H, H ;
R³, R⁴ et R⁵ sont, indépendamment les uns des autres, H, OH, CO₂H, -CH=CH₂ ou -C≡CH ;
Ak¹ est un alkylène en C₆-C₃₀ ramifié ;
q, r et p sont, indépendamment les uns des autres, un entier compris entre 6 et 30 ; et dans lequel le point de liaison est l'un quelconque parmi une fonctionnalité CO2H ; ou un amide, un ester ou un sel pharmaceutiquement acceptable de celui-ci.

3. Procédé selon la revendication 2, dans lequel R1 est choisi parmi les acides gras suivants :

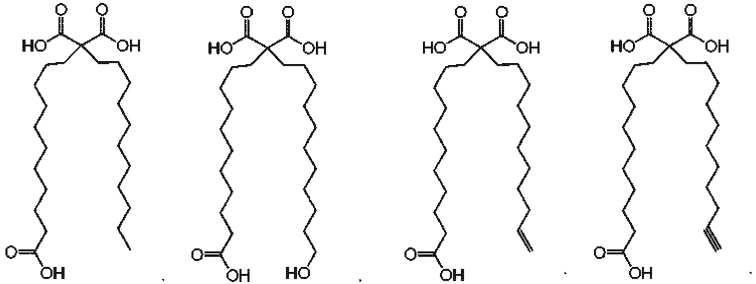

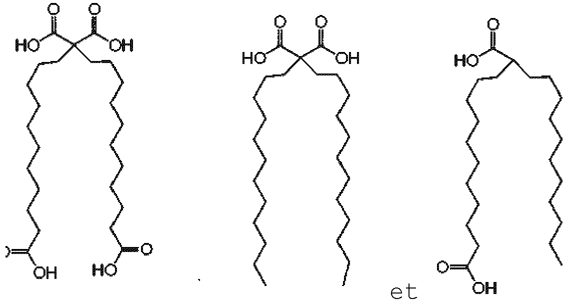

4. Procédé selon la revendication 1, dans lequel l'agent d'acylation est : 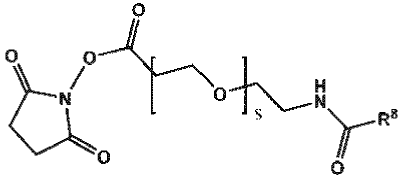 dans lequel
R8 est un alkyle en C6-70 linéaire ou ramifié, un alcényle en C6-70 linéaire ou ramifié ou un alcynyle en C6-70 linéaire ou ramifié, dont chacun est facultativement substitué par 1 à 3 substituants choisis parmi carboxy et hydroxyle ; et
s est 1 à 34.

5. Procédé selon l'une quelconque des revendications 1 à 4, dans lequel l'agent d'acylation est :

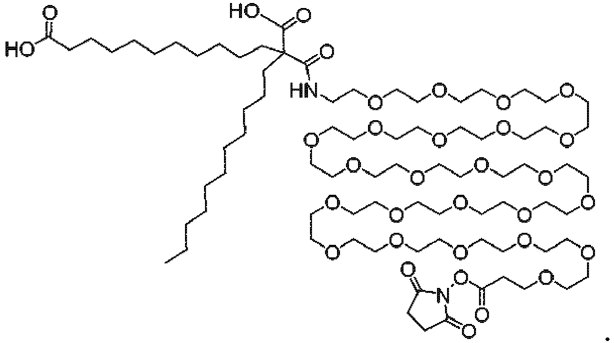

6. Procédé selon la revendication 1, dans lequel l'agent d'acylation est :

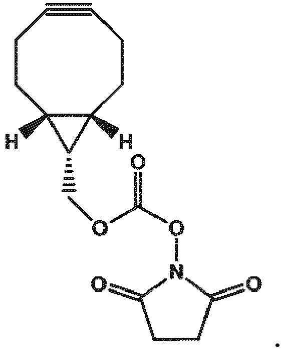

7. Procédé selon la revendication 6 comprenant en outre une étape de réaction d'un agent bio-interactif ou d'un agent analytique de la formule suivante : 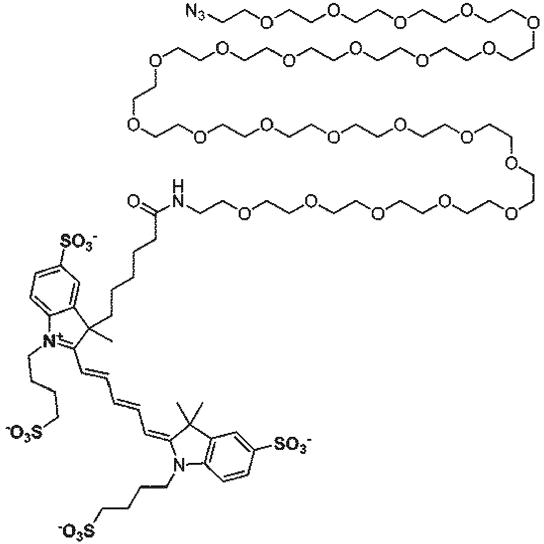 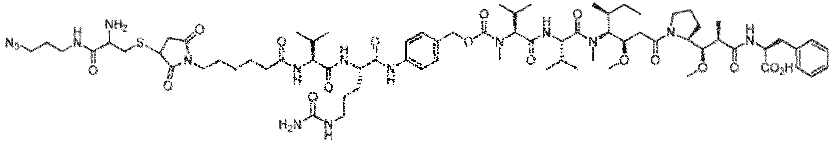 ou 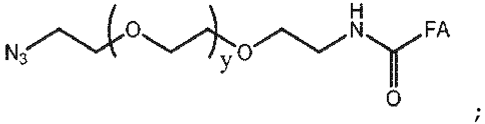 dans lequel y est 0 à 34 et FA est parmi les formules suivantes : 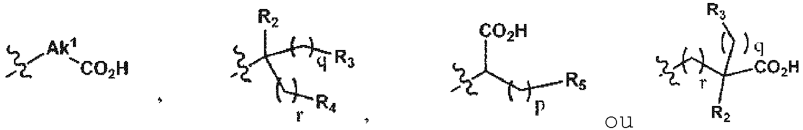
dans lesquelles Ak¹ est un alkylène en C₆-C₃₀ ramifié ;
R² est CO₂H ou H ;
R³, R⁴ et R⁵ sont, indépendamment les uns des autres, H, OH, CO₂H, -CH=CH₂ ou -C≡CH ;
q, r et p sont, indépendamment les uns des autres, un entier compris entre 6 et 30 ;
avec le groupe réactif présent dans l'agent d'acylation.

8. Procédé selon l'une quelconque des revendications précédentes, dans lequel la protéine cible ou le peptide cible est un peptide agoniste d'APJ, un Fc natif ou une protéine GDF15.

9. Procédé selon l'une quelconque des revendications précédentes, dans lequel le pH est 4.
